# EUROPEAN PATENT APPLICATION

(11) **EP 4 268 842 A2**
(43) Date of publication of application: **01.11.2023**
(21) Application number: 23178097.4
(22) Date of filing: 15.06.2020
(51) Int. Cl.: A61K 39/00

(54) **BISPECIFIC ANTIBODY AGAINST a-SYN/IGF1R AND USE THEREOF**

(30) Priority: 14.06.2019 KR 20190071057
(62) Divisional of application: 20822425.3
(71) Applicant: ABL Bio, Inc., Seongnam-si, Gyeonggi-do 13488 (KR)
(72) Inventor: AN, Sungwon, 13488 Seongnam-si (KR); AHN, Jinhyung, 13488 Seongnam-s (KR); SUNG, Byungje, 13488 Seongnam-si (KR); KIM, Dongin, 13488 Seongnam-si (KR); SONG, Daehae, 13488 Seongnam-s (KR); EOM, Jaehyun, 13488 Seongnam-si (KR); SON, Yong-Gyu, 13488 Seongnam-si (KR); PARK, Kyungjin, 13488 Seongnam-si (KR); KIM, Juhee, 13488 Seongnam-si (KR); JUNG, Jinwon, 13488 Seongnam-si (KR); LEE, Bora, 13488 Seongnam-si (KR); YUN, Hyesu, 13488 Seongnam-si (KR)
(74) Representative: Zwicker, Jörk

(57) **Abstract**

The present disclosure relates to a bispecific antibody that specifically binds to alpha-synuclein and IGF1R, and an use of the bispecific antibody for the prevention, treatment and / or diagnosis of synucleinopatheis associated with alpha-synuclein or alpha-synuclein aggregates, and can allow the alpha-synuclein antibody or an antigen-binding fragment thereof to penetrate the blood brain barrier to exert its action in the brain, and extend the half-life to maintain the efficacy for a long time.

## Description

### [TECHNICAL FIELD]

The present invention relates to a bispecific antibody against alpha-synuclein and IGF1R, a pharmaceutical composition for prevention and/or treatment of synucleinopathies (α-synucleinopathies) including the bispecific antibody, and a method of detecting alpha-synuclein aggregates or providing information for diagnosing alpha-synucleinopathies including the bispecific antibody.

### [RELATED ART]

Alpha-synuclein (α-Synuclein, α-syn) is expressed primarily in the presynaptic terminals of neurons, and is a naturally unfolded monomer in normal conditions. Alpha-synuclein helps to regulate the release of dopamine that is a kind of important neurotransmitter controlling initiation and stop of voluntary or involuntary movements. Particularly, the function of alpha-synuclein is important with increased synaptic activity and aging, and is an important factor of neurodegeneration.

However, in the pathological state, alpha-synuclein undergoes structural changes through binding and interaction with droplets, phospholipid bilayers, or lipid membranes to form a folded or folded α-helical secondary structure. Such a secondary structure forms aggregate comprise molecules in the form of dimers, oligomers and/or fibers.

These alpha-synuclein aggregates have been known to induce toxicity to cells, and are the major component of an abnormal protein aggregate of Lewy bodies that are found in neurons of Parkinson's disease (PD), Parkinson's disease dementia (PDD), multiple system atrophy (MSA), Dementia with Lewy bodies (DLB), and various diseases. It is also known that post-translational modifications of alpha-synuclein, such as phosphorylation, or ubiquitination, are also associated with aggregation and neurotoxicity of alpha-synuclein. Alpha-synuclein has been known to kill dopamine neurons and cause inflammatory reactions in animal experiments and cell experiments, and to cause motor symptoms similar to Parkinson's disease in experimental animals . In addition, alpha-synuclein aggregation has been known to be related to an etiology of a group of neurodegenerative diseases called α-synucleinopathies, including Parkinson's disease, Parkinson's disease dementia, Lewy body dementia, multiple system atrophy and many other neuro-axonal diseases.

Antibodies to alpha-synuclein or fragments of alpha-synuclein to induce such antibodies have been proposed as methods of immunotherapy against synuclein disease. However, brain penetration of antibodies may be limited by the blood brain barrier (BBB).

In addition, the deficiency of highly-specific BBB carriers has delayed the development of new therapeutics and diagnostics for diseases originating in the brain, including brain tumors and neurodegenerative diseases. There is clearly a need for a method for delivering therapeutic and diagnostic molecules at a pharmaceutically effective dose to the brain without disrupting the physiology and homeostasis of BBB.

### [DETAILED DESCRIPTION]

### [TECHNICAL PROBLEM]

An embodiment of the present invention provides an antibody comprising an antigen-binding region against alpha-synuclein (α-Syn) and an antigen-binding region against IGF1R, or a method for preparing the antibody.

Another embodiment provides a polynucleotide encoding the antibody, a recombinant vector comprising the same, and a recombinant cell comprising the same.

Further embodiment provides a pharmaceutical composition for prevention and/or treatment of alpha-synucleinopathies, comprising the bispecific antibody against α-Syn and IGF1R and a pharmaceutically acceptable excipient.

Provided is a method for delivering drugs used for diagnosis, treatment or prevention of alpha-synucleinopathies to brain, using the antibody or antigen-binding fragment of the present invention.

An embodiment of the present invention provides an antibody against IGF1R (Insulin-like Growth Factor 1 Receptor) or an antigen-binding fragment thereof, which can specifically recognize IGF1R without affecting the binding of IGF1R ligand, and perform transcytosis without inhibiting signal transduction through IGF1R.

In another embodiment, there is provided an isolated polynucleotide encoding an anti-IGF1R antibody or antigen-binding fragment thereof according to the present invention.

In another embodiment, there is provided a composition for delivery of a physiologically active substance to pass through the blood-brain barrier, comprising an anti-IGF1R antibody or antigen-binding fragment thereof according to the present invention.

Another embodiment of the present invention is to provide a blood brain barrier transporter in which an anti-IGF1R antibody or antigen-binding fragment thereof according to the present invention delivers a physiologically active substance, for example a physiologically active substance acting in a brain to pass through the blood-brain barrier through IGF1R to a brain.

In another embodiment, there is provided a protein complex delivering to pass through the blood-brain barrier through to a brain, where an anti-IGF1R antibody or antigen-binding fragment thereof according to the present invention forms a conjugate with a physiologically active substance, for example a physiologically active substance acting in a brain.

In another embodiment, there is provided a method of detecting IG1R in a biological sample, comprising a step of providing an anti-IGF1R antibody or antigen-binding fragment thereof according to the present invention, and contacting the anti-IGF1R antibody or antigen-binding fragment with a biological sample to be detected for IGF1R expression.

An embodiment provides a method of delivering a physiologically active substance used for diagnosis, treatment or prevention of brain disease to pass through BBB into inner brain, by using an anti-IGF1R antibody or antigen-binding fragment thereof according to the present invention.

### [TECHNICAL SOLUTION]

Hereinafter, the present invention will be described in more detail.

Herein, "antibody" means a complete immunoglobulin in any isotype, or an antigen-binding fragment which can compete with a complete antibody for binding to a target antigen. For example, it includes chimeric, humanized, complete human or bispecific antibodies, or their antigen-binding fragments. The antibody is one kind of antigen binding proteins by itself. Generally, the complete antibody comprises at least two (2) full-length heavy chains and two (2) full-length light chains, but in some cases, the antibody may comprise only heavy chains. The antibodies include monospecific antibodies that specifically bind to one target, and multispecific antibodies (e.g., bispecific antibodies and trispecific antibodies) that specifically bind multiple targets.

The antibody also includes a monoclonal antibody and a polyclonal antibody, and the monoclonal antibody may be an isolated antibody that specifically binds to IGF1R, such as human antibody, a humanized antibody, or a chimeric antibody. The monoclonal antibody is an isolated antibody that specifically binds to IGF1R, in a form of of IgG1, IgG2, IgG3 or IgG4 subtype.

Herein, "light chain" includes a full-length light chain and its fragments having a variable region sequence to sufficiently provide binding specificity to an antigen or epitope. The full-length light chain comprises a variable region domain VL and a constant region domain CL. The variable region domain of light chain is present in an N terminus of a light chain polypeptide. The kinds of light chain include kappa and lambda chains.

As used herein, the term "complementarity-determining regions (CDR)" refers to a region that imparts antigen-binding specificity among variable regions of an antibody.

Herein, "heavy chain" includes a full-length heavy chain and its fragments having a variable region sequence to sufficiently provide binding specificity to an antigen or epitope. The full-length heavy chain comprises a variable region domain VH and three (3) constant region domains of CH1, CH2 and CH3. The VH domain is present in N-terminus of a heavy chain polypeptide and the CH domain is present in a carboxyl-terminus, and CH3 is positioned closest to C-terminus. The heavy chain includes IgG (including IgG1, IgG2a, IgG2b, IgG3 and IgG4 subtypes), IgA (including IgA1 and IgA2 subtypes), and isotypes of IgM and IgE.

The antibody may be selected from all subtypes of immunoglobulins (e.g., IgA, IgD, IgE, IgG (IgG1, IgG2, IgG3, IgG4), IgM, etc.). The IgG form of the antibody may be of the IgG1, IgG2, IgG3, or IgG4 subtype, for example IgG1 or IgG2 subtype. The IgG type antibody comprises two heavy chains and two light chains, and each heavy chain and light chain are combined through disulfide bonds to form two heavy chain-light chain dimers, and the formed two heavy chain-light chains are linked through a disulfide bond at the Fc region of the heavy chain. The IgG form of the antibody comprises a single target antibody targeting one antigen, including antigen binding sites for the same antigen in both heavy chain-light chain constructs, or bispecific antibody targeting two antigens, including antigen-biding sites for different antigens in heavy chain-light chain constructs.

Antibodies according to the present invention include bispecific antibodies, whole antibodies, minibodies, domain antibodies, antibody mimetics (or synthetic antibodies), antibody fusions (or antibody conjugates), fragments thereof and combinations thereof, but not limited thereto. The structures of various antibodies are further disclosed herein below.

In the present invention, for example, "variant" of a polypeptide, such as an antigen-binding fragment, protein, or antibody, is a polypeptide in which insertion, deletion, addition, and/or substitution occurs at one or more amino acid residues compared to other polypeptide sequences, and includes fusion polypeptides. For example, some of the antibodies include conservative amino acid substitutions in one or more residues of the heavy or light chain, variable region or CDR sequence.

The term "derivative" of a polypeptide in the present invention means a polypeptide that is chemically modified through conjugation with other chemical moieties, and is different from insertion, deletion, addition or substitution variants.

The antibodies of the present invention can be generated and selected from transgenic mice, e.g. those described above, where the mice is introduced by the gene encoding the antigen-specific human mAbs having the desired specificity using hybridoma technology. Such antibodies can be cloned and expressed using appropriate vectors and host cells, or the antibodies can be harvested from cultured hybridoma cells. In addition, the antibody may be derived from a phage-display library. Phage display technology is a method that mimics a sort of immune selection by selecting an antibody repertoire on the surface of a filamentous bacteriophage and selecting phage binding to a desired antigen therefrom. Such a technique may refer to an embodiment of the present application or WO1999/010494. In an embodiment, the humanized IGFIR antibody of the present invention is selected through a phage display method.

The antibody or its antigen-binding fragment may be derived from only one source or be chimeric antibody. The chimeric antibody comprises a part derived from two kinds of different antibodies, and is described in more detail below. The antibody or its antigen-binding fragment can be produced by hybridoma, recombinant DNA technique, or enzymatic or chemical cutting of an intact antibody. Unless otherwise stated herein, the term of antibody includes antibodies comprising 2 full-length heavy chains and 2 full-length light chains, and their derivatives, variants, fragments, and mutants, and the examples thereof are as described below.

As used herein, "antigen-binding fragment" includes a part of an antibody which can specifically bind to an antigen, or a polypeptide including the part. For example, the antigen binding fragment includes a part of an antibody containing amino acid residues that gives the specificity and affinity to the antibody by interacting with antigen (e.g., epitope), and a polypeptide including the part. Such fragments comprise at least one CDR present within a full length light or heavy chain, and in some embodiments comprise a single heavy and/or light chain, or a portion thereof. This biologically-active fragment may be produced by a recombinant DNA technique or may be produced for example, by cutting an intact antibody enzymatically or chemically.

An immunologically-functional immunoglobulin fragment includes Fab, Fab', F(ab')₂, Fv, domain antibody and single chain antibodies (e.g., scFv, scFv-Fc etc.) but not limited thereto, and may be derived from any mammal including human, mouse, rat, camelid or rabbit, but not limited thereto. The functional part of the antibody such as one or more CDRs described herein may be linked with a secondary protein or small molecular compound by a covalent bond, and be used as a target therapeutic agent to a specific target.

Herein, "single chain antibody" is a single polypeptide chain of the antigen-binding region formed by connecting heavy chain variable region and light chain variable region via flexible linker. For example, the single chain antibody may be at least one selected from the group consisting of a scFv in which the heavy chain variable region and the light chain variable region are linked in a single chain form, a scFv-Fc in which the heavy chain variable region, a light chain variable region, and Fc are linked in a single chain form, and the like. The single chain antibody may refer to for example, U.S. patent No. 5,260,203.

Herein, "affinity" or "affinity degree is the strength of interaction between an antibody or its antigen-binding fragment and an antigen, and can be determined by properties of the antigen such as size, shape and/or charge of antigen, and CDR sequences and/or physiochemical properties (hydrophilic/hydrophobic properties, electrostatic property and etc.) of the antibody or antigen-binding fragment. The methods for determining the affinity are known in the art, and generally indicated as dissociation constant (KD), but not limited thereto.

In the full length forms of light chain and heavy chain, the variable region and the constant region are joined by a "J" region in about 12 or more amino acid length, and the heavy chain also includes a "D" region in about 10 or more amino acid length. For example, Fundamental Immunology, 2nd ed., Ch. 7 (Paul, W., ed.) 1989, New York: Raven Press can be referred. Typically, the variable regions of the light chain and heavy chain pair in the antibody can form an antigen binding region.

An embodiment of the present invention relates to an antibody comprising an antigen-binding region for alpha-synuclein (α-syn) and an antigen-binding region for IGF1R, specifically a bi-specific antibody against alpha-synuclein (α-syn) and IGF1R (hereinafter, anti-α-syn/anti-IGF1R bispecific antibody). Accordingly, the bispecific antibody according to the present invention can recognize and bind both alpha-synuclein and IGFIR as antigens.

The anti-α-syn/anti-IGF1R bispecific antibody according to the present invention includes anti-α-syn antibody and an antigen-binding fragment thereof, and can recognize and bind to alpha-synuclein, especially C-terminal region of alpha-synuclein, and thus can be used for prevention, treatment and/or diagnosis of synuclienopathies which is associated with alpha-synuclein or aggregates of alpha-synuclein.

Herein, "bivalent antigen-binding protein" or "bivalent antibody" comprises two antigen-binding sites. Two antigen-binding sites comprised in this bivalent antibody may have the same antigen specificity or may be a bispecific antibody binding to different antigens respectively. Herein, "multi-specific antigen-binding protein" or "multi-specific antibody" is targeting two or more of antigens or epitopes.

Herein, "bispecific" or "dual-specific" antigen-binding protein or antibody is a hybrid antigen-binding protein or antibody having 2 different antigen-binding sites. This bispecific antibody is one kind of multi-specific antigen-binding protein or multi-specific antibody, and it can be produced by known various methods, for example, methods such as fusion of hybridoma or linking of Fab' fragment. For example, Songsivilai and Lachmann, Clin. Exp. Immunol. 1990, 79:315-321; Kostelny et al., J. Immunol. 1992, 148:1547-1553 and the like may be referred. The two epitopes being different each other to which twos antigen-binding sites of the bispecific antigen-binding protein or antibody bind may be positioned on the same or different target protein. In one embodiment, the antibody of the present invention may be in the form of a bispecific antibody which additionally comprises the binding to a delivery carrier for delivering the antibody through the blood brain barrier. One method for delivering drugs through the blood brain barrier includes the use of delivery systems such as receptor-mediated transcytosis such as glucose transporter, amino acid transporter, insulin receptor or transferrin receptor in a cell.

According to the present invention, the term "synucleinopathies" include all neurodegenerative disorders characterized by pathological synuclein aggregates. Parkinson's disease, Parkinson's disease dementia (PDD), Dementia with Lewy body (DLB), Lewy body disease, Dementia accompanied with Lewy bodies, Parkinson's syndrome with Dementia, Multiple system atrophy (MSA), multiple nervous system atrophy, and neurodegeneration type I with brain iron accumulation (NBIA Type I), are collectively grouped as synucleinopathies. In addition, the aggregations of alpha-synucleins have been also found secondary in Alzheimer's disease (Kim et al. Alzheimer's Research & Therapy 2014, 6:73).

The synucleinopathies is a diverse group of neurodegenerative disorders that share common pathological properties: In neuropathic experiments, distinct lesions can be detected by detecting abnormal aggregation of alpha-synuclein in the selected populations of neurons and oligodendrocytes. Alpha-synuclein (formerly known as PARKl and PARK4) is a protein composed of 140 amino acids that is extensively expressed in the neocortex, hippocampus, dentate gyrus, posterior neurosphere, striatum, thalamus and cerebellum. Alpha-synuclein is also highly expressed in hematopoietic cells including monocytes such as B cells T cells and NK cells and platelets. The exact role in these cells has been unknown, but associated with differentiation of megakaryocytes (platelet precursors).

Herein "a disease associated with alpha-synuclein aggregates" is a group of neurodegenerative diseases called as synucleinopathies, in which alpha-synuclein aggregates are found in lesions including neurons and glia, and has characteristics. These diseases include Parkinson's disease, Parkinson's disease dementia, Lewy body dementia, Lewy body variant of Alzheimer's disease, combined Alzheimer's and Parkinson's disease, multiple system atrophy, and many other neuroaxonal diseases, but are not limited to. In one embodiment, the antibody according to the present invention is effectively used for treating Parkinson's disease.

In addition, the anti-α-Syn/anti-IGF1R bispecific antibody of the present invention includes an anti-IGF1R antibody or antigen-binding fragment thereof, so that the anti-α-Syn antibody or antigen-binding fragment thereof can penetrate the blood brain barrier to exert its action, and extend the half-life to maintain an efficacy for a long time.

Moreover, the anti-α-Syn/anti-IGF1R bispecific antibody of the present invention binds to IGF1R on the cell surface without affecting the binding of the ligand, and has properties of no effect on the signaling pathway through IGF1R. Because it does not inhibit the binding of IGF1R to its ligand and signaling through IGF1R, it can be used as a shuttle mean to penetrate the blood brain barrier.

Particularly, the anti-IGF1R antibody or antigen-binding fragment thereof of the present invention specifically recognizes IGF1R (Insulin-like Growth Factor 1 Receptor) and binds to IGF1R, particularly human IGF1R, mouse IGF1R, rat IGF1R, and monkey IGF1R. However, it does not interfere with the binding of IGF1R ligand such as IGF-1, IGF-2 and/or insulin to IGF1R and does not inhibit signal transduction through IGF1R, and can be used for transcytosis to pass through BBB. It does not have antibody-dependent cell-mediated cytotoxicity (ADCC), and thus does not decrease IGF1R levels in the brain even when administered to animals repeatedly, thereby having no toxicity.

In particular, the anti-IGF1R antibody of the present invention binds to IGF1R located on the surface of brain endothelial cells constituting BBB and internalizes into the inner part of the cell.

For example, the anti-IGF1R antibody may have a form of scFv, and may be combined to therapeutic antibody in various ways. For example, the scFv of an anti-IGF1R antibody can be prepared in a bispecific antibody, for example a bivalent form of a bispecific antibody in which two scFvs bind to C-terminus of the therapeutic antibody, for example, α-syn antibody, or a monovalent form of a bispecific antibody in which one scFv binds to C-terminus of the therapeutic antibody. Both of these bispecific antibodies can internalize into the cells expressing IGF1R. The IGF1R antibody has a high binding affinity to the antigen on the cell surface, which enhances the internalization effect and lead to BBB-passing ability. If an antibody has the ability to pass BBB and interfering with the signaling of IGF1R, it can cause side effects. However, the antibody of present invention is characterized in both the binding capacity suitable for BBB shuttle and non-blocking property for the IGF1R signaling.

The anti-IGF1R antibody or antigen-binding fragment thereof has excellent property to be developed easily. In this aspect, the post-translational modification, such as deamidation, that occurs in the CDR region of the anti-IGF1R antibody and reduces the stability and efficacy of the antibody is to be removed.

Alternatively, at least one of the amino acids located on both sides of the deamidation site of the antibody may be substituted, and preferably, the amino acid immediately adjacent to the C-terminal side of the deamidation site of the antibody may be substituted. For example, by substituting with A for G located next to Asn located at the deamidation site of the antibody or substituting with V for S located next to Asn, it is possible to produce deamidated antibodies having a similar binding affinity to that of parental anti-IGF1R antibody, and at the same time has excellent stability and BBB penetrating ability.

Additionally, when linked to a bioactive substance acting in the brain, the anti-IGF1R antibody of the present invention can induce improved BBB-penetrating ability and efficacy compared to the bioactive substance alone.

The anti-IGF1R antibody according to one aspect of the present invention may be used as a bispecific antibody including various second therapeutic antibodies. It showed that the BBB penetrating property is about 15-fold higher than the single antibody composed of only a therapeutic antibody, in the penetrating experiment using in vitro BBB system derived from human. The anti-IGF1R antibody may be bound to the second antibody in the bispecific antibody in a monovalent or bivalent form. For example, when analyzing the amount of antibody in blood and CSF after single administration of bispecific antibody with monovalent form or bivalent form anti-IGF1R antibody to a normal rat, the monovalent form and the bivalent form of anti-IGFIR antibodies showed up to 5-fold increased amount in blood and up to 5-fold increased amount in CSF, compared to parental anti-IGF1R antibody (clone 1564). They showed about 3-fold increased amount in CSF and about 4.5-fold increased amount of brain penetrating properties, compared to the parental anti-IGF1R antibody (clone 1564). Therefore, the bispecific antibody including the anti-IGF1R antibody improved by the above method is expected to show up to about 15 times amount in CSF and about 23 times capacity of antibody to penetrate brain compared to the single antibody composed of the therapeutic second antibody alone.

The anti-IGF1R antibody of the present invention has been identified to bind to IGF1R, particularly IGFIR of mammals including humans, monkeys, rats, and mice, and thus can be useful for screening for drug development, clinical trials, and the like.

The anti-IGF1R antibody or antigen-binding fragment of the present invention is an antibody or antigen-binding fragment thereof that specifically recognizes IGF1R (Insulin-like Growth Factor 1 Receptor).

The anti-IGF1R antibody or antigen-binding fragment of the present invention means "specifically bind" to its target, such as an antigen, when it binds to at the dissociation constant (KD) of ≦ 10-6 M. The antibody specifically binds to the target with high affinity, when KD is ≦ 1 × 10⁻⁸ M or when the effective concentration 50 (EC50) is 2 nM or less. In one embodiment, the antibody or antigen-binding fragment thereof is capable of binding to IGF1R or human IGFIR with a KD ≦ 1 × 10⁻⁸.

As used herein, the term "epitope" is an antigenic determinant, which is interpreted to mean a portion of the antigen recognized by the antibody. According to one embodiment, the binding site of the anti-IGF1R antibody of the present invention may be an extracellular domain of IGF1R protein, for example, a human IGF1R protein (SEQ ID NO: 99). More specifically, the binding sites of the anti-IGF1R antibody of the present invention, for example, 1564 clone antibody for the human IGFIR protein are binding site 1 including Y775, P776, F778, R650, S791, L798 and Glu779, binding site 2 including L641, H808, E809 and L813, and binding site 3 including V397, D435, W434, Y460 and C488, in human IGF1R protein. Thus, the epitope of the IGF1R antibody of the present invention may be the conformational epitope that include all or part of the three binding sites.

The anti-IGF1R antibody or antigen-binding fragment of the present invention does not prevent the binding of IGF1R ligands such as IGF-1, IGF-2, and/or insulin to IGF1R. Specifically, the anti-IGF1R antibody or antigen-binding fragment does not interfere with binding of the IGF1R ligand to the IGF1R located on the membrane of the cells expressing IGF1R, or neither inhibit signal transduction through IGF1R nor affect the expression of IGF1R on the cell surface advantageously. Thus, the anti-IGF1R antibody or antigen-binding fragment of the present invention can be effectively used to penetrate the blood brain barrier through transcytosis. IGF1R has been shown to have a relatively high expression level in the brain compared to other transcytosis targets which have been used currently and have known to be expressed in endothelial cells of the brain for improving the ability to penetrate BBB. In one embodiment of the present invention, when IGF1R is compared to other targets currently being developed for the purpose of improving the BBB-penetrating capacity of therapeutic antibodies, for example, transferrin receptor, or insulin receptor, it has been shown to have a relatively low expression level in peripheral tissue such as the liver, lungs, or large intestine.

IGFIR is a target of Receptor Mediated Transcytosis (RTM), which can deliver useful substances through the blood brain barrier (BBB) into brain. However, in order to be used as a drug delivery target for penetrating the blood-brain barrier, it is desirable to have a property binding to IGF1R on the cell surface without affecting the binding of the ligand and the signaling pathway through IGF1R. Thus, the anti-IGF1R antibody and antigen-binding fragment thereof of the present invention does not inhibit the binding of IGF1R to its ligand and signaling through IGF1R, and thus can be used as a shuttle mean to penetrate the blood brain barrier.

The anti-IGF1R antibody or antigen-binding fragment of the present invention is capable of transcytosis and can pass through endothelial cells of brain. In addition, the antibody of the present invention is located in the same place as the brain blood vessels of the mouse, when it is injected into a blood vessel of mouse. These results indicate that the antibody or antigen-binding fragment of the present invention can be effectively used as a drug carrier that crosses the blood brain barrier.

Therefore, the anti-IGF1R antibody or antigen-binding fragment of the present invention allows the bioactive substance acting in brain to pass through the blood brain barrier. In the present invention, the biological barrier refers to cells, tissues, membranes, or a cell, membrane, or structure that prevents effective passage, diffusion, or transfer of biological molecule. These biological barriers include nerve cells/tissues, connective tissue, muscle, membrane or epithelial (e.g. mucosal or vascular) cells. A typical example is the blood brain barrier.

In the present invention, the term "blood brain barrier" or BBB is a barrier formed by tight junctions in the capillary endothelial membrane of the brain existing between the brain and spine and its surrounding circulatory system. This barrier is so sturdy that it also limits the passage of molecules having low molecular weight of about 60 Da to the brain. The blood brain barrier of the brain, the vascular spinal cord barrier of the spine and the vascular retinal barrier of the retina are continuous capillary barriers in the central nervous system, commonly referred to as BBB.

In the present invention, the term "blood brain barrier transporter" may pass through the blood brain barrier and deliver an antibody or an antigen-binding fragment thereof according to the present invention, and for example, include a protein including g peptide and polypeptide, a nucleic acid, an antibody, or a small molecular compound.

The present invention relates to an isolated antibody or antigen-binding fragment thereof that specifically binds to IGF1R, wherein the antibody or antigen-binding fragment may be a polypeptide, protein or antibody or antigen-binding fragment thereof by including a complementarity determining region of a heavy chain and a complementarity determining region of a light chain, and specifically binds to IGF1R.

In specific examples, the anti-IGF1R antibody or antigen-binding fragment thereof can include:
(i) one or more heavy chain complementarity determining regions selected from the group consisting of H-CDR1, H-CDR2 and H-CDR3 described in Table 1, or a heavy chain variable region comprising the one or more heavy chain complementarity determining regions;
(ii) one or more light chain complementarity determining regions selected from the group consisting of L-CDR1, L-CDR2 and L-CDR3 described in Table 1, or a light chain variable region comprising the one or more light chain complementarity determining regions;
   a combination of one or more heavy chain complementarity determining regions(CDRs) and one or more light chain complementarity determining regions(CDRs); or
   a combination of the heavy chain variable region and the light chain variable region.

Additionally, in the heavy chain variable region, the light chain variable region, or a combination of the heavy chain variable region and the light chain variable region, the heavy chain variable region may include one or more heavy chain framework selected from the group consisting of H-FR1, H-FR2, H-FR3 and H-FR4, and the light chain variable region may include one or more light chain framework selected from the group consisting of L-FR1, L-FR2, L-FR3, and L-FR4.

In one embodiment of the present invention, the elimination of amino acids associated with the occurrence of deamidation in an anti-IGF1R antibody, can reduce the risk of antibody degradation, which is unfavorable to process development, storage, and etc., without changing the binding affinity to ECD of IGFIR as an antigen. In the anti-IGF1R antibody, the positions deleted amino acids may be, for examples, N51D, N51Q or S52V in a light chain L-CDR2 and N95aK, N95aH, N95aR, N95aD or G95bA in a light chain L-CDR3, or N54D, N54Q or G55A in a heavy chain H--CDR2 of 1564 (IgG), 1564 (scFv) or 1564-3. The elimination of deamidation is not limited to the clones described above, but may be applied to other clones according to the method of Table 14.

**[Table 1]**

| CDR of a light chain variable region and a heavy chain variable region in anti-IGF1R antibody | | | | | | |
|---|---|---|---|---|---|---|
| clone ID/ SEQ ID No | H-CDR1 | H-CDR2 | H-CDR3 | L-CDR1 | L-CDR2 | L-CDR3 |
| 1564 (IgG) | 1 | 2 | 8 | 20 | 21 | 24 |
| 1564 (scFv) | 1 | 2 | 8 | 20 | 21 | 24 |
| 1564-3 | 1 | 2 | 8 | 20 | 21 | 24 |
| 1564-DM | 1 | 3 | 8 | 20 | 22 | 25 |
| 1564-DMP | 1 | 3 | 8 | 20 | 22 | 25 |
| VH5-DM | 1 | 4 | 8 | 20 | 22 | 25 |
| VH5-DMP | 1 | 4 | 8 | 20 | 22 | 25 |
| F06-DM | 1 | 3 | 8 | 20 | 22 | 26 |
| F06-DMP | 1 | 3 | 8 | 20 | 22 | 26 |
| F06 (de2)(StoP) | 1 | 5 | 8 | 20 | 23 | 27 |
| VH5(de2)(StoP) | 1 | 6 | 9 | 20 | 23 | 28 |
| VH16(de2)(StoP) | 1 | 7 | 8 | 20 | 23 | 28 |
| 1564(de2)(StoP) | 1 | 5 | 8 | 20 | 23 | 28 |
| VH2 (scFv) | 10 | 11 | 19 | 20 | 21 | 24 |
| VH2-3 | 10 | 11 | 19 | 20 | 21 | 24 |
| VH2-DM | 10 | 12 | 19 | 20 | 22 | 25 |
| VH2-DMP | 10 | 12 | 19 | 20 | 22 | 25 |
| VH5 (scFv) | 1 | 13 | 9 | 20 | 21 | 24 |
| VH5-3 | 1 | 13 | 9 | 20 | 21 | 24 |
| VH7 (scFv) | 10 | 14 | 8 | 20 | 21 | 24 |
| VH7-3 | 10 | 14 | 8 | 20 | 21 | 24 |
| VH7-DM | 10 | 15 | 8 | 20 | 22 | 25 |
| VH7-DMP | 10 | 15 | 8 | 20 | 22 | 25 |
| VH9 (scFv) | 1 | 16 | 8 | 20 | 21 | 24 |
| VH9-3 | 1 | 16 | 8 | 20 | 21 | 24 |
| VH9-DM | 1 | 17 | 8 | 20 | 22 | 25 |
| VH9-DMP | 1 | 17 | 8 | 20 | 22 | 25 |
| VH16 (scFv) | 1 | 11 | 8 | 20 | 21 | 24 |
| VH16-3 | 1 | 11 | 8 | 20 | 21 | 24 |
| VH16-DM | 1 | 12 | 8 | 20 | 22 | 25 |
| VH16-DMP | 1 | 12 | 8 | 20 | 22 | 25 |
| VH32 (scFv) | 10 | 18 | 19 | 20 | 21 | 24 |
| VH32-3 | 10 | 18 | 19 | 20 | 21 | 24 |
| VH32-DM | 10 | 3 | 19 | 20 | 22 | 25 |
| VH32-DMP | 10 | 3 | 19 | 20 | 22 | 25 |
| VH35 (scFv) | 1 | 14 | 9 | 20 | 21 | 24 |
| VH35-3 | 1 | 14 | 9 | 20 | 21 | 24 |
| VH35-DM | 1 | 15 | 9 | 20 | 22 | 25 |
| VH35-DMP | 1 | 15 | 9 | 20 | 22 | 25 |
| C04 (scFv) | 1 | 18 | 8 | 20 | 21 | 29 |
| C04-3 | 1 | 18 | 8 | 20 | 21 | 29 |
| C04-DM | 1 | 3 | 8 | 20 | 22 | 30 |
| C04-DMP | 1 | 3 | 8 | 20 | 22 | 30 |
| F06 (scFv) | 1 | 18 | 8 | 20 | 21 | 31 |
| F06-3 | 1 | 18 | 8 | 20 | 21 | 31 |

**[Table 2]**

| CDR of a light chain variable region and a heavy chain variable region in main anti-IGF1R antibody | | | | | | |
|---|---|---|---|---|---|---|
| clone ID | H-CDR1 | H-CDR2 | H-CDR3 | L-CDR1 | L-CDR2 | L-CDR3 |
| F06-DMP | GFTFSSY DMS | AISYDQ GNTYYA DSVKG | GVLTTL MNWFD Y | TGSSSNI GSNDVS | AQSNRP S | GTWAGS LHGYV |
| SEQ ID NO | 1 | 3 | 8 | 20 | 22 | 26 |
| F06 (de2)(StoP ) | GFTFSSY DMS | AISYDN ANTYYA DSVKG | GVLTTL MNWFD Y | TGSSSNI GSNDVS | ANVNRP S | GTWAGS LNAYV |
| SEQ ID NO | 1 | 5 | 8 | 20 | 23 | 27 |
| VH16(de2 )(StoP) | GFTFSSY DMS | AISGSNA NTYYAD SVKG | GVLTTL MNWFD Y | TGSSSNI GSNDVS | ANVNRP S | GAWDD SLNAYV |
| SEQ ID NO | 1 | 7 | 8 | 20 | 23 | 28 |
| 1564(de2) (StoP) | GFTFSSY DMS | AISYDN ANTYYA DSVKG | GVLTTL MNWFD Y | TGSSSNI GSNDVS | ANVNRP S | GAWDD SLNAYV |
| SEQ ID NO | 1 | 5 | 8 | 20 | 23 | 28 |

The anti-IGF1R antibody comprises a heavy chain variable region and a light chain variable region, wherein the heavy chain variable region comprises a heavy chain CDR1 (H-CDR1) comprising an amino acid sequence elected from the amino acid sequences of SEQ ID NO: 1 or SEQ ID NO: 10, a heavy chain CDR2 (H-CDR2) comprising an amino acid sequence selected from the amino acid sequences of SEQ ID NOs: 2 to 7 and SEQ ID NOs: 11 to 18, and a heavy chain CDR3 (H-CDR3) comprising an amino acid sequence selected from the amino acid sequences of SEQ ID NOs: 8 to 9 and SEQ ID NO: 19, and
the light chain variable region comprises a light chain CDR1 (L-CDR1) comprising an amino acid sequence selected from the amino acid sequences of SEQ ID NO: 20, a light chain CDR2 (L-CDR2) comprising an amino acid sequence selected from the amino acid sequences of SEQ ID NOs: 21 to 23, and a light chain CDR3 (L-CDR3) comprising an amino acid sequence selected from the amino acid sequences of SEQ ID NOs: 24 to 28 and SEQ ID NOs: 29 to 31.

In an embodiment of the present invention, the anti-IGF1R antibody or antigen binding fragment thereof comprises a heavy chain variable region and a light chain variable region,
the heavy chain variable region comprises a heavy chain CDR1 (H-CDR1) comprising an amino acid sequence elected from the amino acid sequences of SEQ ID NO: 1, a heavy chain CDR2 (H-CDR2) comprising an amino acid sequence selected from the amino acid sequences of SEQ ID NO: 3, and SEQ ID NOs: 5 to 7, and a heavy chain CDR3 (H-CDR3) comprising an amino acid sequence selected from the amino acid sequences of SEQ ID NOs: 8 to 9, and
the light chain variable region comprises a light chain CDR1 (L-CDR1) comprising an amino acid sequence selected from the amino acid sequences of SEQ ID NO: 20, a light chain CDR2 (L-CDR2) comprising an amino acid sequence selected from the amino acid sequences of SEQ ID NOs: 22 and 23, and a light chain CDR3 (L-CDR3) comprising an amino acid sequence selected from the amino acid sequences of SEQ ID NOs: 26 to 28.

The heavy chain variable region of the anti-IGF1R antibody according to the present invention can include H-CDR1, H-CDR2 and H-CDR3 described in Table 1, or further include H-FR1 comprising an amino acid sequence of SEQ ID NO: 32, H-FR2 comprising an amino acid sequence of SEQ ID NO: 33 or SEQ ID NO: 34, H-FR3 comprising an amino acid sequence of SEQ ID NO: 35, and H-FR4 comprising an amino acid sequence of SEQ ID NO: 36.

The light chain variable region of the anti-IGF1R antibody according to the present invention can include L-CDR1, L-CDR2 and L-CDR3 described in Table 1, or further include L-FR1 comprising an amino acid sequence selected from SEQ ID NO: 37, L-FR2 comprising an amino acid sequence of SEQ ID NO: 38, L-FR3 comprising an amino acid sequence of SEQ ID NO: 39 or SEQ ID NO: 40, and L-FR4 comprising an amino acid sequence of SEQ ID NO: 41 or SEQ ID NO: 42.

In the frameworks of the heavy chain and the light chain, the framework 1 (FR1) is located at N-terminus of CDR1, framework 2 (FR2) is located between CDR1 and CDR2, framework 3 (FR3) is located between CDR2 and CDR3, and framework 4 (FW4) is located at C-terminus of CDR3.

Specifically, the framework sequences in a heavy chain variable region of 1564 (IgG) include amino acid sequences of SEQ ID Nos: 32, 33, 35 and 36, and those of other clones than 1564 (IgG) in Table 1 include amino acid sequences of SEQ ID Nos: 32, 34, 35 and 36.

Specifically, the framework sequences in a light chain variable region of the anti-IGF1R antibodies according to the present invention are shown in Table 3.

**[Table 3]**

| The framework sequences in a light chain variable region of the anti-IGF1R antibodies | | | | |
|---|---|---|---|---|
| clone ID | L-FR1 SEQ ID NO | L-FR2 SEQ ID NO | L-FR3 SEQ ID NO | LFR4 SEQ ID NO |
| 1564 (IgG) | 37 | 38 | 39 | 41 |
| 1564 (scFv) | 37 | 38 | 40 | 42 |
| 1564-3 | 37 | 38 | 39 | 42 |
| 1564-DM | 37 | 38 | 40 | 42 |
| 1564-DMP | 37 | 38 | 39 | 42 |
| VH05-DM | 37 | 38 | 40 | 42 |
| VH05-DMP | 37 | 38 | 39 | 42 |
| F06-DM | 37 | 38 | 40 | 42 |
| F06-DMP | 37 | 38 | 39 | 42 |
| F06(de2)(StoP) | 37 | 38 | 39 | 42 |
| VH5(de2)(StoP) | 37 | 38 | 39 | 42 |
| VH16(de2Stop) | 37 | 38 | 39 | 42 |
| 1564(de2)(StoP) | 37 | 38 | 39 | 42 |
| VH2 (scFv) | 37 | 38 | 40 | 42 |
| VH2-3 | 37 | 38 | 39 | 42 |
| VH2-DM | 37 | 38 | 40 | 42 |
| VH2-DMP | 37 | 38 | 39 | 42 |
| VH5 (scFv) | 37 | 38 | 40 | 42 |
| VH5-3 | 37 | 38 | 39 | 42 |
| VH7 (scFv) | 37 | 38 | 40 | 42 |
| VH7-3 | 37 | 38 | 39 | 42 |
| VH7-DM | 37 | 38 | 40 | 42 |
| VH7-DMP | 37 | 38 | 39 | 42 |
| VH9 (scFv) | 37 | 38 | 40 | 42 |
| VH9-3 | 37 | 38 | 39 | 42 |
| VH9-DM | 37 | 38 | 40 | 42 |
| VH9-DMP | 37 | 38 | 39 | 42 |
| VH16 (scFv) | 37 | 38 | 40 | 42 |
| VH16-3 | 37 | 38 | 39 | 42 |
| VH16-DM | 37 | 38 | 40 | 42 |
| VH16-DMP | 37 | 38 | 39 | 42 |
| VH32 (scFv) | 37 | 38 | 40 | 42 |
| VH32-3 | 37 | 38 | 39 | 42 |
| VH32-DM | 37 | 38 | 40 | 42 |
| VH32-DMP | 37 | 38 | 39 | 42 |
| VH35 (scFv) | 37 | 38 | 40 | 42 |
| VH35-3 | 37 | 38 | 39 | 42 |
| VH35-DM | 37 | 38 | 40 | 42 |
| VH3 5-DMP | 37 | 38 | 39 | 42 |
| C04 (scFv) | 37 | 38 | 40 | 42 |
| C04-3 | 37 | 38 | 39 | 42 |
| C04-DM | 37 | 38 | 40 | 42 |
| C04-DMP | 37 | 38 | 39 | 42 |
| F06 (scFv) | 37 | 38 | 40 | 42 |
| F06-3 | 37 | 38 | 39 | 42 |

The anti-IGF1R antibody according to the present invention may be an antibody comprising a heavy chain variable region and a light chain variable region, and various heavy and light chain variable regions disclosed herein are exemplarily described in Tables 4 and 5. The heavy chain variable regions and light chain variable regions described in Tables 4 and 5 below can be freely combined for the production of various types of antibodies. Each of these variable regions may be linked to the heavy and light chain constant regions to form each heavy chain and each light chain of an intact antibody.

**[Table 4]**

| Heavy chain variable regions and light chain variable regions of the anti-IGF1R antibodies | | | | | |
|---|---|---|---|---|---|
| clone ID /SEQ ID No | VH | VL | clone ID /SEQ ID No | VH | VL |
| 1564 (IgG) | 43 | 88 | VH9 (scFv) | 66 | 111 |
| 1564 (scFv) | 44 | 89 | VH9-3 | 67 | 112 |
| 1564-3 | 45 | 90 | VH9-DM | 68 | 113 |
| 1564-DM | 46 | 91 | VH9-DMP | 69 | 114 |
| 1564-DMP | 47 | 92 | VH16 (scFv) | 70 | 115 |
| VH05-DM | 48 | 93 | VH16-3 | 71 | 116 |
| VH05-DMP | 49 | 94 | VH16-DM | 72 | 117 |
| F06-DM | 50 | 95 | VH16-DMP | 73 | 118 |
| F06-DMP | 51 | 96 | VH32 (scFv) | 74 | 119 |
| F06(de2)(StoP) | 52 | 97 | VH32-3 | 75 | 120 |
| VH5(de2)(StoP) | 53 | 98 | VH32-DM | 76 | 121 |
| VH16(de2Stop) | 54 | 99 | VH32-DMP | 77 | 122 |
| 1564(de2)(StoP) | 55 | 100 | VH35 (scFv) | 78 | 123 |
| VH2 (scFv) | 56 | 101 | VH35-3 | 79 | 124 |
| VH2-3 | 57 | 102 | VH35-DM | 80 | 125 |
| VH2-DM | 58 | 103 | VH35-DMP | 81 | 126 |
| VH2-DMP | 59 | 104 | C04 (scFv) | 82 | 127 |
| VH5 (scFv) | 60 | 105 | C04-3 | 83 | 128 |
| VH5-3 | 61 | 106 | C04-DM | 84 | 129 |
| VH7 (scFv) | 62 | 107 | C04-DMP | 85 | 130 |
| VH7-3 | 63 | 108 | F06 (scFv) | 86 | 131 |
| VH7-DM | 64 | 109 | F06-3 | 87 | 132 |
| VH7-DMP | 65 | 110 | ** | ** | ** |

**[Table 5]**

| Heavy chain variable regions and light chain variable regions in main anti-IGF1R antibodies | | |
|---|---|---|
| clone ID | VH | VL |
| F06-DMP | EVQLLESGGGLVQPGGSLRLSCA ASGFTFSSYDMSWVRQAPGKCLE WVSAISYDQGNTYYADSVKGRFT ISRDNSKNTLYLQMNSLRAEDTA VYYCAKGVLTTLMNWFDYWGQ GTLVTVSS (SEQ ID NO: 51) | QSVLTQPPSASGTPGQRVTISCTGS SSNIGSNDVSWYQQLPGTAPKLLI YAQSNRPSGVPDRFSGSKSGTSAS LAISGLRSEDEADYYCGTWAGSL HGYVFGCGTKLTVL (SEQ ID NO: 96) |
| F06(de2)(St oP) | EVQLLESGGGLVQPGGSLRLSCA ASGFTFSSYDMSWVRQAPGKCLE WVSAISYDNANTYYADSVKGRFT ISRDNSKNTLYLQMNSLRAEDTA VYYCAKGVLTTLMNWFDYWGQ GTLVTVSS (SEQ ID NO: 52) | QSVLTQPPSASGTPGQRVTISCTGS SSNIGSNDVSWYQQLPGTAPKLLI YANVNRPSGVPDRFSGSKSGTSAS LAISGLRSEDEADYYCGTWAGSL NAYVFGCGTKLTVL (SEQ ID NO: 97) |
| VH16(de2S top) | EVQLLESGGGLVQPGGSLRLSCA ASGFTFSSYDMSWVRQAPGKCLE WVSAISGSNANTYYADSVKGRFTI SRDNSKNTLYLQMNSLRAEDTAV YYCAKGVLTTLMNWFDYWGQG TLVTVSS (SEQ ID NO: 54) | QSVLTQPPSASGTPGQRVTISCTGS SSNIGSNDVSWYQQLPGTAPKLLI YANVNRPSGVPDRFSGSKSGTSAS LAISGLRSEDEADYYCGAWDDSL NAYVFGCGTKLTVL (SEQ ID NO: 99) |
| 1564(de2)( StoP) | EVQLLESGGGLVQPGGSLRLSCA ASGFTFSSYDMSWVRQAPGKCLE WVSAISYDNANTYYADSVKGRFT ISRDNSKNTLYLQMNSLRAEDTA VYYCAKGVLTTLMNWFDYWGQ GTLVTVSS (SEQ ID NO: 55) | QSVLTQPPSASGTPGQRVTISCTGS SSNIGSNDVSWYQQLPGTAPKLLI YANVNRPSGVPDRFSGSKSGTSAS LAISGLRSEDEADYYCGAWDDSL NAYVFGCGTKLTVL (SEQ ID NO: 100) |

The heavy chain variable region of the anti-IGF1R antibody or antigen-binding fragment thereof according to the present invention may include one selected from the group consisting of amino acid sequences of SEQ ID NO: 43 to SEQ ID NO: 87. The light chain variable region of the anti-IGF1R antibody or antigen-binding fragment thereof according to the present invention may include one selected from the group consisting of the amino acid sequences of SEQ ID NOs: 88 to 132. Examples of the heavy chain variable region and the light chain variable region are described in Tables 4 and 5 above.

The anti-IGF1R antibody or an antigen binding fragment thereof that specifically recognizes and binds to at least one amino acids selected from the group consisting of Y775, P776, F778, R650, S791, L798, Glu779, L641, H808, E809, L813, V397, D435, W434, Y460 and C488 in human IGF1R having an amino acid sequence of SEQ ID NO: 174. Specifically, anti-IGF1R antibody or an antigen binding fragment thereof of the present invention can bind to at least one selected from Binding site 1 to Binding site 3 of a human IGF1R protein including an amino acid sequence of SEQ ID NO: 174. The binding site 1 comprise at least one amino acid selected from the group consisting of Y775, P776, F778, R650, S791, L798 and Glu779, the binding site 2 comprise at least one amino acid selected from the group consisting of L641, H808, E809 and L813, and the binding site 3 comprises at least one amino acid selected from the group consisting of V397, D435, W434, Y460 and C488.

Each of the heavy chain variable regions and the light chain variable regions disclosed in Tables 4 and 5 can be used as separate domain antibodies, can be freely combined with each other to form various antibodies, and are linked in a single chain form to obtain single chain antibodies such as scFv.

Herein, "domain antibody" is an immunologically functional immunoglobulin fragment comprising a variable region of heavy chain and/or a variable region of light chain only. In one embodiment, two or more of VH regions are linked by a covalent bond by a peptide linker, to form a bivalent domain antibody. Two VH regions of this bivalent domain antibody may target the same or different antigen.

Antigen-binding fragments of anti-IGF1R antibodies of the present invention may be one selected from the group consisting of scFv, (scFv)2, scFv-Fc, Fab, Fab ', F (ab')2, minibody and diabody include antibody fragments comprising one or more complementarity determining regions.

In the antigen-binding fragments, Fab includes a light chain variable region, a heavy chain variable region, a light chain constant region, and a first constant region (CH1) of the heavy chain, and has one antigen binding site. Fab' has a hinge region in the Fab that contains one or more cysteine residues at the C-terminus of the heavy chain CH1 domain. The F (ab')2 antibody is produced by linking two Fab's are with forming disulfide bond between cysteine residues of the Fab' hinge region.

Fv is a minimal antibody fragment having only a heavy chain variable region and a light chain variable region, and includes single-chain variable fragments (scFv) and double-chain variable fragments (Fv). In the double chain Fv, a heavy chain variable region and a light chain variable region may be linked by non-covalent bonds. In the single-chain Fv, the heavy-chain variable region and the light-chain variable region are covalently linked directly or via a peptide linker, or linked directly at the C-terminus to form a scFv dimer-like structure (di-scFv), such as a double-chain Fv. In the present invention, the single-chain Fv is a single polypeptide chain of an antigen-binding region in which heavy and light chain variable regions are directly or linked by a linker, and can be at least one selected from the group consisting of scFv having single chain linked with the heavy chain variable region and the light chain variable region, a form of scFv dimer-like structure (di-scFv), skFv-Fc in which the heavy chain variable region, the light chain variable region and Fc is linked as a single chain form, and the like.

The peptide linker may be as described above, and may be, for example, 1 to 100, such as 2 to 50 amino acids length or 5 to 25 amino acids length, and the peptide linker can be in a various length within a limit that does not affect the function of the antibody. The kinds of amino acids included in the peptide linker may be composed of one or more amino acids selected from the group consisting of, for example, Gly, Ser and Leu, and specific examples include Gly and Ser residues, or Leu and Ser residues. In a specific example, the peptide linker may be (G4S)n in which n is a repetition number of (G4S) represented by an integer of 1 to 10, such as 2 to 5, especially 3 or 4. An example of the peptide linker may be a peptide consisting of amino acids of SEQ ID NOs: 133 or 134.
SEQ ID NO 133: GGGGSGGGGSGGGGS
SEQ ID NO 134: GGGGSGGGGSGGGGSGGGGS

The single chain Fv (scFv) can be produced by fusing DNA encoding a peptide linker between DNAs encoding two variable domain polypeptides (VL and VH). The prepared polypeptide can form antigen-binding monomer or multimers (e.g., dimers, trimers or tetramers) depending on the length of the flexible linker between the two variable domains, with folding. By combining polypeptides containing different VLs and VHs, multimeric scFv that bind to different epitopes can be formed.

The antigen-binding fragments can be obtained using proteolytic enzymes (e.g., a restriction digestion of the entire antibody with papain to obtain Fab, and digestion with pepsin to obtain F(ab')2 fragment), or using the genetic recombination technology.

The single-chain antibodies disclosed herein include, but are not limited to, scFvs comprising domain combinations of heavy and light chain variable regions, or combinations of light and heavy chain variable domains comprising CDRs.

The antigen-binding fragment of the anti-IGF 1R antibody may be linked with or without a linker, such as a peptide linker. In addition, the heavy and light chain portions in the antigen-binding fragment, such as the heavy chain variable region and the light chain variable region in the scFv fragment, can also be linked with or without a peptide linker. The peptide linker may be as described above.

In the bispecific antibody, the anti-IGF1R antibody and antigen-binding fragments thereof may perform a function of delivering a second antibody or antigen-binding fragment targeting different antigens or epitopes that are bound to it, through the blood brain barrier to brain. The second antibody may be an antibody that exerts efficacy in brain, but is not particularly limited, it may be an anti-alpha-synuclein antibody or a binding fragment thereof according to the present invention.

The anti-IGF1R antibody or antigen-binding fragment thereof of the present invention may share a specific region or sequence with a different second antibody. For example, the anti-IGFIR antibody may share the constant region or the Fc region of the antibody or antigen-binding fragment of the second antibody.

In addition, the structure of the bispecific antibody in the present invention includes a bivalent form of bispecific antibody, in which the scFv of anti-IFG1R antibody is linked to each Fc of two heavy chains of a complete immunoglobulin, for example at the ends of the heavy chain directly or via a linker and a monovalent form of bispecific antibody, in which a scFv of an anti-IGF1R antibody linked to only one end of the two heavy chains of a complete immunoglobulin directly or via a linker, but a monovalent double antibody is preferred.

Specifically, in one embodiment of the present invention, there is a case in which the monovalent form clone has an improved half-life than the bivalent form clone, and the structure of the monovalent form clone is form that domain antibody (scFv) against IGF1R is bound to only an end of one heavy chain in the intact immunoglobulin via a linker. Specifically, the antibody is a heterodimer applied by Knob-In-Hole method that includes two different heavy chains of the intact immunoglobulin in which one heavy chain has domain antibody (scFv) against IGF1R bound to C-terminus thereof, and the other heavy chain has not any at the C-terminus thereof.

In the bispecific antibody, the second antibody that binds to an anti-IGF1R antibody or antigen-binding fragment thereof may be a human antibody, a humanized antibody, a chimeric antibody, or an isolated antibody specifically binding to IGF1R. The second antibody includes, but is not limited to, complete antibodies, bispecific antibodies, minibodies, domain antibodies, antibody mimetics (or synthetic antibodies), antibody fusions (or antibody conjugates), and fragments thereof. In the bispecific antibody, an example of the second antibody that binds to the anti-IGF1R antibody or antigen-binding fragment thereof may be the anti-syn antibody or antigen-binding fragment thereof according to the present invention.

Hereinafter, the present invention relates to an anti-syn antibody and an antigen-binding fragment thereof.

The alpha-synuclein which can be recognized by the antibody provided herein can be selected from the mammal alpha-synucleins of human alpha-synuclein, monkey alpha-synuclein (e.g. Rhesus alpha-synuclein), mouse alpha-synuclein, rat alpha-synuclein, and the like. For example, the human alpha-synuclein can be alpha-synuclein (NCBI ID: NP_000336), but not limited thereto. Unless otherwise stated herein, the alpha-synuclein may refer to human alpha-synuclein, and the antibodies or antigen-binding fragments provided herein have a specific binding property to not only human alpha-synuclein, but also monkey (e.g., Rhesus) alpha-synuclein, rat alpha-synuclein, and/or mouse alpha-synuclein.

The antibody or antigen binding fragment thereof can bind to C-terminal region of alpha-synuclein, specifically C-terminal region including the peptide comprised of at least 11 or 12 consecutive amino acids including 110 to 120 residues or 111 to 122 residues in SEQ ID NO: 173 of human alpha-synuclein. It has been confirmed that the antibody or antigen binding fragment of present invention can recognize the antigen recognition region and bind to the alpha-synuclein aggregate with a high binding affinity.

Herein, "specifically binding to alpha-synuclein protein or alpha-synuclein aggregate" means that the binding affinity to alpha-synuclein protein or alpha-synuclein aggregate is relatively high compared to other antigens, and for example, may be the affinity of 0.1 × 10⁻¹⁰ M to 2 × 10⁻¹⁰ M, or 0.05 × 10⁻¹⁰ M to 0.3×10⁻⁹ M to alpha-synuclein aggregates, specifically amyloid fibrils, protofibrils and oligomers, particularly amyloid fibrils, as measured by the Octet analysis or the SPR analysis, but not limited thereto.

The humanized alpha-synuclein antibodies including light chain and heavy chain according to an embodiment of the present invention, for examples, Hu11F11 (ver.2), and Hu11F11_ABL2-4, exhibit a high activity to promote phagocytic uptake compared to the chimeric alpha-synuclein antibodies. Compared to the chimeric alpha-synuclein antibody, Hu11F11 (ver.1), Hu11F11 (ver.2), Hu11F11 (ver.3), Hu11F11 (ver.4), and ABL2-4 shows high activity of inhibiting the binding of fibril to nerve cell membrane compared to the chimeric alpha-synuclein antibody. Hu11F11 (ver.2), Hu11F11 (ver.4) and ABL2-4 have high activity of inhibiting the propagation of alpha-synuclein secreted from the cells overexpressing alpha-synuclein to other nerve cells compared to the chimeric alpha-synuclein antibody, and show the binding affinity to the alpha-synuclein aggregate, for example, which is has a similar or superior activity to the chimeric alpha-synuclein antibody in a cell-based assay.

The alpha-synuclein antibody according to the present invention can inhibit the function of alpha-synuclein aggregates secreted out of nerve cell in the nervous system of a subject to transfers to other normal cells in an extracellular space and to infect the nerve cells (inhibit cell-to-cell transmission of aggregates), and have an ability of promoting the phagocytic action of microglia to alpha-synuclein aggregates in the extracellular space. The alpha-synuclein aggregates propagates from one cell to other cell like prions, and the alpha-synuclein, especially alpha-synuclein aggregates spread throughout the brain, resulting in synucleinopathies in normal cells. Therefore, alpha-synuclein aggregates are toxic to brain neurons and are well known to cause brain neuron death (neurodegeneration) and neuro-inflammation. Accordingly, as alpha-synuclein aggregates spread to various parts of the brain, the brain cell death and the neuro-inflammation reactions increase and results in occurrence of the brain cell death and the resulting behavior and cognitive impairments which are found with the progression of synucleinopathies such as Parkinson's disease.

Accordingly, the alpha-synuclein antibody of the present invention can prevent the spreading phenomenon of alpha-synuclein aggregates to various regions of the brain by inhibiting the transmission of the alpha-synuclein or alpha-synuclein aggregate between the nerve cells, and reduce the level of the alpha-synuclein aggregates which is an important cause of synucleinopathies by reducing or eliminating the alpha-synuclein aggregates themselves in extracellular region of the nerve cells of subject nerve system with the promotion of the microglia phagocytosis of, resulting in reduction of brain nerve cell death and the neuro-inflammatory reaction and further being expected to improve, alleviate or prevent the symptoms and the progress of synucleinopathies such as Parkinson's disease.

In addition, the alpha-synuclein antibody according to the present invention has excellent activities of performing both of two functions of (i) inhibition of the transmission of the alpha-synuclein or alpha-synuclein aggregate between the nerve cells (see the result of cell-based assay disclosed herein), and (ii) reduction of alpha-synuclein aggregates level in the brain nervous system through the promoted phagocytosis of microglial cells. In particular, since the alpha-synuclein antibodies which have been currently in clinical trials or published in the scientific paper have one of the two activities (i) and (ii), it suggests that the alpha-synuclein antibody of the present invention have an advantage in superior prevention or treatment of synucleinopathies to the the known alpha-synuclein antibodies. Therefore, the alpha-synuclein antibody according to the present invention have more excellent efficacies of reduction and elimination of alpha-synuclein aggregates and inhibition of the action of alpha-synuclein aggregates as etiology, and thus is more effective for synucleinopathies or a symptomatic disease related thereto (e.g., cognitive impairment disorder).

The antibodies or antigen-binding fragments according to the present invention having a high affinity for alpha-synuclein aggregates can reduce the formation of alpha-synuclein aggregate, thereby lowering the concentration of aggregates in the brain. In addition, the antibody or antigen-binding fragment according to the present invention with a high affinity for alpha-synuclein aggregates can reduce the formation of alpha-synuclein aggregates outside the central nervous system and finally, change the equilibrium state between the alpha-synuclein forms bounded by of BBB, thereby bringing the effect of lowering the concentration of alpha-synuclein aggregates inside the central nervous system.

The antibody or antigen-binding fragment according to the present invention can inhibit formation of aggregates by removing monomers, or eliminates both monomers and aggregates.

The antibodies or antigen-binding fragments thereof of the present invention that specifically binds to alpha-synuclein proteins or alpha-synuclein aggregates may not be naturally-occurring product (it can be non-naturally occurring product, for example, by chemical synthesis or recombinant method). The recombination techniques are well known in the art.

The anti-alpha-synuclein antibody or the bispecific antibody including the anti-alpha-synuclein antibody may be used for prevention or treatment of α-synucleinopathy, and the α-synucleinopathy can include Parkinson's disease (PD), Parkinson's disease dementia (PDD), dementia with Lewy bodies, (DLB), Lewy body variant of Alzheimer's disease (LBV)), Combined Alzheimer's and Parkinson disease, or multiple system atrophy( MSA), but not limited thereto.

The antibody or antigen-binding fragment thereof that specifically binds to alpha-synuclein or an aggregate thereof according to the present invention includes a heavy chain variable region comprising a complementarity determining region of CDRH1, CDRH2 and CDRH3; and a light chain variable region comprising a complementarity determining region of CDRL1, CDRL2 and CDRL3.

In one embodiment, the anti-alpha-synuclein antibody or antigen-binding fragment thereof may comprise the following CDR sequences:
a heavy chain CDR1 (H-CDR1) including an amino acid sequence of SEQ ID NO: 135,
a heavy chain CDR2 (H-CDR2) including an amino acid sequence of SEQ ID NO: 136 or SEQ ID NO; 137,
a heavy chain CDR3 (H-CDR3) including an amino acid sequence of SEQ ID NO: 138,
a light chain CDR1 (L-CDR1) including an amino acid sequence of SEQ ID NO: 139,
a light chain CDR2 (L-CDR2) including an amino acid sequence of SEQ ID NO: 140, and
a light chain CDR3 (L-CDR3) including an amino acid sequence of SEQ ID NO: 141.

The amino acid sequences of the heavy chain CDR1 to CDR3 and the amino acid sequences of the light chain CDR1 to CDR3 are summarized in Tables 6 and 7. In the light chains of Hu11F11-VLv3 4c and Hu11F11-VL4 shown in Table 7, the amino acid sequences of light chain CDR1 to CDR3 are the same, but the framework sequences are different.

**[Table 6]**

| Amino acid sequences of heavy chain CDR1 to CDR3 of anti α-syn antibodies | | | | | | |
|---|---|---|---|---|---|---|
| Clone | SE Q ID | VH_CDR1 | SE Q ID | VH_CDR2 | SE Q ID | VH_CDR3 |
| Hu11F11-VH-v1 | 135 | GFTFSDFY ME | 136 | ASRNKANDYTTEYSA SVKG | 138 | DAHGKPF AY |
| Hu11F11-VH-v2 | 135 | GFTFSDFY ME | 136 | ASRNKANDYTTEYSA SVKG | 138 | DAHGKPF AY |
| Hu11F11-VH-v3 | 135 | GFTFSDFY ME | 136 | ASRNKANDYTTEYSA SVKG | 138 | DAHGKPF AY |
| Hu11F11-VH-v4 | 135 | GFTFSDFY ME | 136 | ASRNKANDYTTEYSA SVKG | 138 | DAHGKPF AY |
| Hu11F11-VH2 | 135 | GFTFSDFY ME | 137 | AIRNKANDYTTEYAA SVKG | 138 | DAHGKPF AY |

**[Table 7]**

| Amino acid sequences of light chain CDR1 to CDR3 of anti α-syn antibodies | | | | | | |
|---|---|---|---|---|---|---|
| Clone | SEQ ID NO | VL CDR1 | SEQ ID NO | VL CDR2 | SEQ ID NO | VL CDR3 |
| Hu11F11-VLv3 4c | 139 | KSSQSLLYSSNQKNY LA | 140 | WASTRE S | 141 | QQYYSYPW T |
| Hu11F11-VL4 | 139 | KSSQSLLYSSNQKNY LA | 140 | WASTRE S | 141 | QQYYSYPW T |

The various heavy and light chain variable regions disclosed herein can be linked to the heavy and light chain constant regions to form each heavy chain and light chain of an intact antibody. In addition, each of the heavy and light chain sequences generated in this way can also be combined to form a complete antibody structure.

For example, the anti-alpha-synuclein antibody or antigen-binding fragment thereof according to the present invention comprises a heavy chain variable region comprising an amino acid sequence selected from the group consisting of amino acid sequences of SEQ ID NO: 142 to SEQ ID NO: 146, and a light chain variable region comprising an amino acid sequence selected from the group consisting of an amino acid sequence of SEQ ID NO: 147 to SEQ ID NO: 148, and exemplary sequences of the heavy chain variable regions and the light chain variable regions are shown in Table 8 below.

**[Table 8]**

| Heavy chain variable region and light chain variable region of anti-α-syn antibody | | | |
|---|---|---|---|
| clone | Chain | SEQ ID NO | Amino acid sequence |
| Hu11F11-VH-v1 | VH | 142 | EVQLVESGGGLVQPGGSLRLSCATSGFTFSDFY MEWVRQPPGKRLEWIAASRNKANDYTTEYSAS VKGRFTISRDDSKSSLYLQMNSLRAEDTAIYYCA RDAHGKPFAYWGQGTTVTVSS |
| Hu11F11-VH-v2 | VH | 143 | EVQLVESGGGLVQPGGSLRLSCATSGFTFSDFY MEWVRQPPGKRLEWIAASRNKANDYTTEYSAS VKGRFTVSRDDSKSSLYLQMNSLRAEDTAIYYC ARDAHGKPFAYWGQGTTVTVSS |
| Hu11F11-VH-v3 | VH | 144 | EVQLVESGGGLVQPGGSLRLSCATSGFTFSDFY MEWVRQPPGKRLEWIAASRNKANDYTTEYSAS VKGRFTISRDTSKSSLYLQMNSLRAEDTAIYYCA RDAHGKPFAYWGQGTTVTVSS |
| Hu11F11-VH-v4 | VH | 145 | EVQLVESGGGLVQPGGSLRLSCATSGFTFSDFY MEWVRQPPGKRLEWIAASRNKANDYTTEYSAS VKGRFTVSRDTSKSSLYLQMNSLRAEDTAIYYC ARDAHGKPFAYWGQGTTVTVSS |
| Hu11F11-VH2 | VH | 146 | EVQLVESGGGLVQPGGSLRLSCAASGFTFSDFY MEWVRQAPGKGLEWIAAIRNKANDYTTEYAAS VKGRFTISRDTSKNSLYLQMNSLKTEDTAVYYC ARDAHGKPFAYWGQGTLVTVSS |
| Hu11F11-VLv3 4c | VL | 147 | DIVMTQSPSSLAVSLGERVTMSCKSSQSLLYSSN QKNYLAWYQQKPGQSPKLLIYWASTRESGVPD RFTGSGSGTDFTLTISSVKAEDVAVYYCQQYYS YPWTFGGGTKLEIK |
| Hu11F11-VL4 | VL | 148 | DIQMTQSPSSLSASVGDRVTITCKSSQSLLYSSNQ KNYLAWYQQKPGKAPKLLIYWASTRESGVPSRF SGSGSGTDFTLTISSLQPEDFATYYCQQYYSYPW TFGQGTKVEIK |

In addition, exemplary antibodies through a combination of the heavy chain variable region and the light chain variable region of the antibody or antigen-binding fragment according to one embodiment are described in Table 9.

**[Table 9]**

| Exemplary anti-α-syn antibodies of the present invention | | |
|---|---|---|
| Clone ID | Heavy chain variable region and light chain variable region of each clone | SEQ ID NO |
| hu11F11(ver.1) | Hu11F11-VH-v1 | 142 |
| | Hu11F11-VLv3 4c | 147 |
| hu11F11(ver.2) | Hu11F11-VH-v2 | 143 |
| | Hu11F11-VLv3 4c | 147 |
| hu11F11(ver.3) | Hu11F11-VH-v3 | 144 |
| | Hu11F11-VLv3 4c | 147 |
| hu11F11(ver.4) | Hu11F11-VH-v4 | 145 |
| | Hu11F11-VLv3 4c | 147 |
| hu11F11(H2L4) | Hu11F11-VH2 | 146 |
| | Hu11F11-VL4 | 148 |

In another embodiment, the anti-alpha-synuclein antibody may consist of only the light chain or heavy chain described above. In another embodiment, the anti-alpha-synuclein antibody may consist of only the light chain variable region or the heavy chain variable region.

In further embodiment, the heavy chain variable region and the light chain variable region described in Table 8 above may be combined to form various antibodies, or may be linked in a single chain form to form a single chain antibody such as scFv.

Antibodies disclosed herein may share certain regions or sequences with other antibodies disclosed herein. In one embodiment, the constant region of the antibody or antigen-binding fragment may be shared. In other embodiments, they may share an Fc region.

It may include a heavy chain including the heavy chain variable region and a light chain including the light chain variable region. Specifically, the heavy chain variable region and the light chain variable region may be linked to the heavy chain constant region and the light chain constant region, and the heavy chain and light chain sequences may also be combined to form an intact antibody structure.

The constant region sequences that can be combined with the variable region according to the present invention are exemplary, and the constant region may be appropriately selected from the heavy chain constant region and the light chain constant region of an immunoglobulin (eg, human immunoglobulin). For example, the heavy chain constant region may be an IgG1 heavy chain constant region, an IgG3 heavy chain constant region, or an IgG4 heavy chain constant region, and the light chain constant region may be a kappa constant region or a lambda light chain constant region, but is not limited thereto.

As an exemplary antibody comprising a variable region and a constant region of an anti-α-syn antibody or antigen-binding fragment according to one embodiment, the anti-alpha-synuclein antibody of hu11F11 (ver.2) clone may be an antibody including a heavy chain including an amino acid sequence of SEQ ID NO: 149 and a light chain including an amino acid sequence of SEQ ID NO: 150.

The anti-alpha-synuclein antibody according to the present invention may be used alone as a therapeutic antibody, but may be used as a bispecific antibody in combination with other antibodies capable of delivering it to the brain though passing the blood-brain barrier. An example of an antibody capable of delivering it to the brain though passing the blood-brain barrier may be an anti-IGF1R antibody and an antigen-binding fragment thereof. The anti-IGF1R antibody and antigen-binding fragment thereof, which can form a bispecific antibody, may include all of the above-described anti-IGF1R antibody and antigen-binding fragment thereof. For example, the anti-IGF1R antibody may be a complete antibody, or the antigen-binding fragment may be one selected from the group consisting of domain antibody, scFv, (scFv)2, scFvFc, Fab, Fab' and F(ab')2.

The antigen-binding fragment of the anti-IGF1R antibody may be linked through or without a linker, for example, a peptide linker. Also, the heavy chain region and the light chain region in the antigen-binding fragment, such as the heavy chain variable region and the light chain variable region in the scFv fragment, may be linked via or without a peptide linker. The peptide linker may be as described above.

The anti-alpha-synuclein antibody or antigen-binding fragment thereof according to the present invention can be used in the preparation of heavy chain combinations for the production of bispecific antibodies, and examples of heavy chains of the anti-alpha-synuclein antibodies used for producing a heavy chain combination for the bispecific antibody are associated with an anti-alpha-synuclein antibody of hu11F11 (ver.2), and include hu11F11 (ver.2) (IGG) having an amino acid sequence of SEQ ID NO: 149, and hu11F11 (ver.2)(IGG) WITH HOLE MUTATION AT FC having an amino acid sequence of SEQ ID NO: 151.

Examples of heavy chains of anti-alpha-synuclein antibodies used in the preparation of heavy chain combinations for the production of bispecific antibodies, including the above-described anti-alpha-synuclein antibody hu11F11 (ver.2) are shown in Table 10 below, and the heavy chain sequences are shown in SEQ ID NO: 151 to SEQ ID NO: 172, but not limited thereto. In addition, Table 10 below shows the specific components of the bispecific antibody clone in which the heavy chain combination using the heavy chain of the anti-alpha-synuclein antibody and the light chain are combined. The bispecific antibodies presented below are described as examples, and the construction is clear from the description of the heavy chain combination of bispecific antibody, even if it is not indicated by SEQ ID No. Exemplary bispecific antibodies are specifically shown in Table 10 below.

**[Table 10]**

| Clone ID of bispecific antibody | Light chain of bispecific antibody | Heavy chain combination of bispecific antibody | SEQ ID | Description of the componets in the heavy chain combination of bispecific antibody |
|---|---|---|---|---|
| hu11F11(ver.2)-1564 (scFv) monovalent | Hu11F11-VLv3 4c | hu11F11(ver.2)-1564-2 monovalent (HC 1, -hole) | 158 | hu11F11(ver.2 (IGG) WITH HOLE MUTATION AT FC, |
| | | | | (G4S)3, |
| | | | | 1564 (scFv) VL, |
| | | | | (G4S)4, |
| | | | | 1564 (scFv) VH |
| | | hu11F11(ver.2)-1564 monovalent (HC 2, - knob) | 152 | hu11F11(ver.2 (IGG) WITH KNOB MUTATION |
| hu11F11(ver.2)-VH5 bivalent | Hu11F11-VLv3 4c | hu11F11(ver2)-VHS bivalent (HC) | * | hu11F11(ver.2 (IGG), |
| | | | | (G4S)3, |
| | | | | VH5 VL, |
| | | | | (G4S)4, |
| | | | | VH5 VH |
| hu11F11(ver.2)-VH16 bivalent | Hu11F11-VLv3 4c | hu11F11(ver.2)-VH16 bivalent (HC) | * | hu11F11(ver.2 (IGG), |
| | | | | (G4S)3, |
| | | | | VH16 VL, |
| | | | | (G4S)4, |
| | | | | VH16 VH |
| hu11F11(ver.2)-F06-DM monovalent | Hu11F11-VLv3 4c | hu11F11(ver.2)(M42 8L)-F06-DM monovalent (HC 1, - hole) | 165 | hu11F11(ver.2 (IGG), M428L, HOLE MUTATION, |
| | | | | (G4S)3, |
| | | | | F06-DMP VL, |
| | | | | (G4S)4, |
| | | | | F06-DMP VH |
| | | hu11F11(ver.2)(M42 8L)-F06 monovalent (HC 2, -knob) | 155 | hu11F11(ver.2 (IGG),M428L MUTATION, KNOB MUTATION |
| hu11F11(ver.2)-F06-DMP monovalent | Hu11F11-VLv3 4c | hu11F11(ver.2) -F06-DMP monovalent (HC 1, -hole) | * | hu11F11(ver.2 (IGG), HOLE MUTATION, |
| | | | | (G4S)3, |
| | | | | F06-DMP VL, |
| | | | | (G4S)4, |
| | | | | F06-DMP VH |
| | | hu11F11(ver.2) -F06 monovalent (HC 2, - knob) | * | hu11F11(ver.2 (IGG), KNOB MUTATION |
| hu11F11(ver.2)(M4 28L)-F06-DMP monovalent | Hu11F11-VLv3 4c | hu11F11(ver.2)(M42 8L)-F06-DMP monovalent (HC 1, - hole) | 164 | hu11F11(ver.2 (IGG), M428L, HOLE MUTATION, |
| | | | | (G4S)3, |
| | | | | F06-DMP VL, |
| | | | | (G4S)4, |
| | | | | F06-DMP VH |
| | | hu11F11(ver.2)(M42 8L)-F06 monovalent (HC 2, -knob) | 155 | hu11F11(ver.2 (IGG),M428L MUTATION, KNOB MUTATION |
| hu11F11(ver.2)(M4 28L)-F06-DM monovalent | Hu11F11-VLv3 4c | hu11F11(ver.2)(M42 8L)-F06-DM monovalent (HC 1, - hole) | 165 | hu11F11(ver.2 (IGG), M428L, HOLE MUTATION, |
| | | | | (G4S)3, |
| | | | | F06-DM VL, |
| | | | | (G4S)4, |
| | | | | F06-DM VH |
| | | hu11F11(ver.2)(M42 8L)-F06 monovalent, deamidated, S->P (HC 2, -knob) | 155 | hu11F11(ver.2 (IGG),M428L MUTATION, KNOB MUTATION |
| hu11F11(ver.2)-F06(de2)(StoP) monovalent | Hu11F11-VLv3 4c | hu11F11(ver.2)-F06(de2)(StoP)-(monovalent, deamidated, S->P (HC 1, -hole) | 166 | hu11F11(ver.2) (IGG) HOLE MUTATION |
| | | | | (G4S)3 |
| | | | | F06(de2)(StoP) VL |
| | | | | (G4S)4 |
| | | | | F06(de2)(StoP) VH |
| | | hu11F11(ver.2)-F06(de2)(StoP)-(de2) monovalent, deamidated, S->P (HC 2, -knob) | 152 | hu11F11(ver.2 (IGG), KNOB MUTATION |
| hu11F11(ver.2)-VH5(de2)(StoP) bivalent | Hu11F11-VLv3 4c | hu11F11(ver.2)-VH5(de2)(StoP) deamidated, S->P bivalent(HC) | 167 | hu11F11(ver.2 (IGG) |
| | | | | (G4S)3, |
| | | | | VH5(de2)(StoP)VL |
| | | | | (G4S)4 |
| | | | | VH5(de2)(StoP)VH |
| hu11F11(ver.2)-VH16(de2)(StoP) bivalent | Hu11F11-VLv3 4c | hu11F11(ver.2)-VH16(de2)(StoP) deamidated, S->P bivalent(HC) | 168 | hu11F11(ver.2 (IGG) |
| | | | | (G4S)3, |
| | | | | VH16(de2)(StoP)VL |
| | | | | (G4S)4 |
| | | | | VH16(de2)(StoP)VH |
| hu11F11(ver.2)(M4 28L)-F06(de2)(StoP) monovalent | Hu11F11-VLv3 4c | hu11F11(ver.2)(M42 8L)-F06(de2)(StoP)-(monovalent, deamidated, S->P (HC 1, -hole) | 169 | hu11F11(ver.2) (IGG) M428L, HOLE MUTATION |
| | | | | (G4S)3 |
| | | | | F06(de2)(StoP) VL |
| | | | | (G4S)4 |
| | | | | F06(de2)(StoP) VH |
| | | hu11F11(ver.2)(M42 8L)- F06(de2)(StoP)-(de2) monovalent, deamidated, S->P (HC 2, -knob) | 155 | hu11F11(ver.2 (IGG),M428L MUTATION, KNOB MUTATION |
| hu11F11(ver.2)-F06(de2)(StoP) bivalent | Hu11F11-VLv3 4c | hu11F11(ver.2)-F06(de2)(StoP) deamidated, S->Pbivalent(HC) | * | hu11F11(ver.2) (IGG) |
| | | | | (G4S)3, |
| | | | | F06(de2)(StoP)VL |
| | | | | (G4S)4 |
| | | | | F06(de2)(StoP)VH |
| hu11F11(ver.2)(M4 28L)-F06(de2)(StoP) bivalent | Hu11F11-VLv3 4c | hu11F11(ver.2)(M42 8L)-F06(de2)(StoP) deamidated, S->Pbivalent(HC) | * | hu11F11(ver.2 (IGG) M428L mutated |
| | | | | (G4S)3, |
| | | | | F06(de2)(StoP)VL |
| | | | | (G4S)4 |
| | | | | F06(de2)(StoP)VH |
| hu11F11(ver.2)-1564 (de2)(StoP) monovalent | Hu11F11-VLv3 4c | hu11F11(ver.2)-1564(de2)(StoP)-monovalent, deamidated, S->P (HC1,-hole) | * | hu11F11(ver.2) (IGG) HOLEMUTATION |
| | | | | (G4S)3 |
| | | | | 1564(de2)(StoP) VL |
| | | | | (G4S)4 |
| | | | | 1564(de2)(StoP) VH |
| | | hu11F11(ver.2)-1564(de2)(StoP)-(de2) monovalent, deamidated, S->P (HC2,-knob) | * | hu11F11(ver.2) (IGG) WITH KNOB MUTATION |
| hu11F11(ver.2)(M4 28L)-VH5(de2)(StoP) bivalent | Hu11F11-VLv3 4c | hu11F11(ver.2)(M42 8L)-VH5(de2)(StoP) deamidated, S->P bivalent(HC) | 170 | hu11F11(ver.2 (IGG) M428L mutation |
| | | | | (G4S)3, |
| | | | | VH5(de2)(StoP)VL |
| | | | | (G4S)4 |
| | | | | VH5(de2)(StoP)VH |
| hu11F11(ver.2)(M4 28L)-VH16(de2)(StoP) bivalent | Hu11F11-VLv3 4c | hu11F11(ver.2)(M42 8L)-VH16(de2)(StoP) deamidated, S->P bivalent(HC) | 171 | hu11F11(ver.2 (IGG) M428L mutation |
| | | | | (G4S)3, |
| | | | | VH16(de2)(StoP)VL |
| | | | | (G4S)4 |
| | | | | VH16(de2)(StoP)VH |
| hu11F11(ver.2)(M4 28L)-1564(de2)(StoP) monovalent | Hu11F11-VLv3 4c | hu11F11(ver.2)(M42 8L)-1564(de2)(StoP)-monovalent, deamidated, S->P (HC 1, -hole) | 172 | hu11F11(ver.2) (IGG) M428L, HOLE MUTATION |
| | | | | (G4S)3 |
| | | | | 1564(de2)(StoP) VL |
| | | | | (G4S)4 |
| | | | | 1564(de2)(StoP) VH |
| | | hu11F11(ver.2)(M42 8L)-1564(de2)(StoP)-(de2) monovalent, deamidated, S->P (HC 2, -knob) | 155 | Hu11F11(ver2)(M428L) -knob |

The pharmaceutical composition for preventing or treating alpha-synuclein disease, comprising the alpha-synuclein antibody or antigen-binding fragment thereof, or a bispecific antibody comprising the same according to the present invention, comprises an alpha-synuclein antibody or bispecific antibodies at a pharmaceutically effective amount.

As used herein, "treatment" can refer to any action related to the alleviation or elimination of a disease or symptom, or pathological condition, including reducing, alleviating, relieving or eliminating a disease or disease symptom, making the disease symptom or pathological condition more tolerable, slowing down the rate of exacerbating the disease symptom or pathological state or etc. The term "subject" or "patient" includes human or human patient.

There is provided a pharmaceutical composition comprising a therapeutically effective amount of an antibody and a pharmaceutically acceptable diluent, carrier, solubilizer, emulsifier, preservative and/or adjuvant. For examples, the present invention includes a method of treating a patient associated with alpha-synuclein by administering the pharmaceutical composition. Pharmaceutical compositions used for in vivo administration are typically provided as sterile preparations. Once the pharmaceutical composition is formulated, the pharmaceutical composition may be stored in sterile vials as a solution, suspension, gel, emulsion, solid, crystal, or dehydrated or lyophilized powder. Such formulations may be stored in ready-to-use form, or in a form that is reconstituted immediately prior to administration (e.g., lyophilized).

The route of administration of the pharmaceutical composition may be known method, for example, orally; injection via the intravenous, intraperitoneal, intracerebral (intraparenchymal), intraventricular, intramuscular, intraocular, intraarterial, intraportal, or intralesional routes, and sustained release systems; or implanted devices may be used. In certain embodiments, the composition may be administered continuously by bolus injection, or by infusion or implantation device.

The alpha-synuclein antibody or antigen-binding fragment thereof or a bispecific antibody comprising the same according to the present invention described herein, may be used for diagnostic purposes for detecting, diagnosing, or monitoring diseases and/or symptoms associated with alpha-synuclein. For diagnostic use, antibodies may typically be labeled with a detectable label.

### [Effects of the Invention]

The antibody prepared in one embodiment of the present invention specifically binds to IGF1R with a binding affinity optimized for brain endothelial transcytosis, and is useful for the delivery of antibodies for treatment of degenerative brain diseases and brain cancer, which have limited therapeutic efficacy due to low blood-brain barrier-penetrating ability. In particular, the antibody disclosed in the present invention does not affect the binding of ligands such as IGF-1, IGF-2 and insulin as its homolog to IGF1R, and does not inhibit signal transduction through the IGF1R, and thus it has utility in relation to the penetration of the brain blood barrier. The antibody disclosed herein can effectively remove or promote the degradation of alpha-synuclein aggregates, and can inhibit the intercellular transfer of alpha-synuclein, so that it can be usefully used in the treatment of diseases related to the accumulation of alpha-synuclein aggregates. The alpha-synuclein antibody or the bispecific antibody comprising the same according to the present invention can be used as a pharmaceutical composition for preventing or treating α-synucleinopathy.

### [Brief Description of Drawings]

Fig. 1 is a result of ELISA to measure the affinity of anti-α-syn chimeric antibody and humanized 11F11 antibody prepared in an example of the present invention.
Figs. 2a to 2c are results of BIAcore analyses on the preferred binding specificity and affinity of anti-α-syn chimeric antibody and humanized 11F11 antibody prepared in an example of the present invention for alpha-synuclein aggregates.

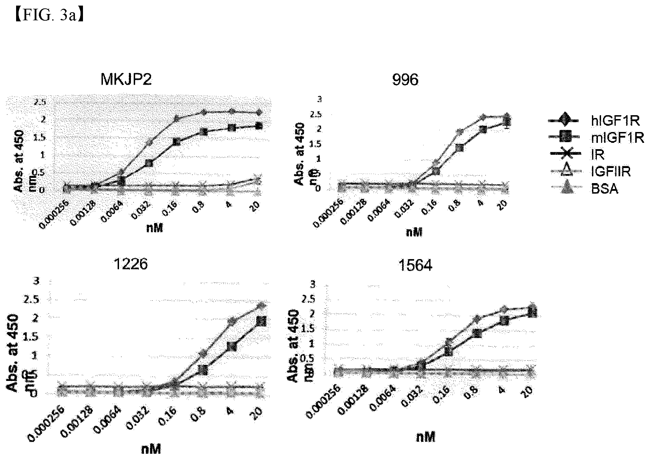

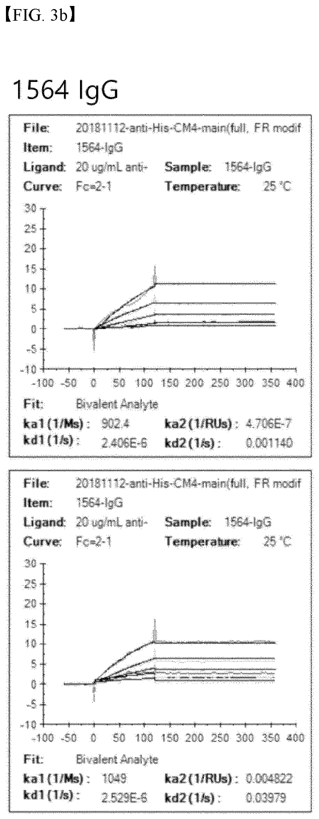

Figs. 3a and 3b are binding data of the anti-IGF1R antibody prepared in an example of the present invention to the IGF1R protein.
Figs. 4a, 4b and 4c are binding data of the anti-IGF1R antibody prepared in an example of the present invention to IGF1R-expressing cell lines.
Fig. 5a, 5b, and 5c show data of the internalization of the anti-IGF1R antibody prepared in an example of the present invention into IGF1R-expressing cell lines and the fate in the cell.
Figs. 6a, 6b, and 6c are data showing that the anti-IGF1R antibody prepared in an example of the present invention does not affect IGFIR signaling induced by IGF1 or insulin.
Figs. 7a, 7b, 7c, 7d, 7e, and 7f show that the anti-IGF1R antibody and the bispecific antibody including the anti-IGF1R antibody and a therapeutic antibody penetrate the BBB in vivo better than the therapeutic antibody alone.
Fig. 8 is a result showing the deamidation position of the anti-IGF1R antibody.
Fig. 9 is an epitope mapping result of an anti-IGF1R antibody.
Figs. 10a and 10b are results of ELISA to measure the binding ability of the bispecific antibody prepared in the example of the present invention each antigen.
Fig. 10c and 10d are results of ELISA to compare the binding affinities of the chimeric antibody and the humanized antibody to each antigen.
Fig. 10e is a result of evaluating the activity of microglial phagocytosis on the bispecific antibody prepared in example of the present invention.
Figs. 11a to 11e are results of evaluating the efficacy of the bispecific antibody prepared in an example of the present invention in comparison with a monospecific antibody in a mouse animal model.
Fig. 12 is a result showing the increased half-life and improvement in BBB penetration by performing Fc engineering on the bispecific antibody prepared in an example of the present invention.
Fig. 13 is a result of evaluating the ability to reduce α-syn in a mouse animal model with respect to the bispecific antibody prepared in an example of the present invention.
Figs. 14a, 14b, and 14c are results of ELISA to perform comparative analysis of the antigen-binding ability of the deamidated anti-IGF1R antibodies prepared in examples of the present invention and control antibodies, respectively.
Figs. 15a to 15c are results of FACS analysis of the IGF1R-specific binding capacity of the deamidated bispecific antibody prepared in an example of the present invention.
Fig. 16 is a result of comparative analysis of in vivo BBB penetration ability of the deamidated bispecific antibody prepared in examples of the present invention and the control antibodies.
Fig. 17 is a result of comparative analysis of in vivo BBB penetration ability of the deamidated bispecific antibody prepared in examples of the present invention and the control antibodies.

### [Detailed Description]

The present invention will be described in more detail with reference to the following examples, but the scope of the present invention is not intended to be limited to the following examples.

### Example 1: Preparation of mouse alpha-synuclein antibody

### 1-1: Immunization and Hybridoma production

Alpha-synuclein monomer with a full length (140 residues) or cleaved with C-terminal 21 residues (119 residues) were placed in a thermomixer at 37 °C, aggregated with shaking at 1050 rpm for 14 days, and sonicated. Each of 140 residues and 119 residues of the α-syn fibril at 1 mg / ml was mixed with the adjuvant at a ratio of 1: 1 (vol: vol). The amino acid sequence of Homo sapiens alpha-synuclein is shown in SEQ ID NO: 173.

Then, 200 µL of the prepared mixture was injected subcutaneously into 5 to 7 week old BALB/c female mice. After 2 weeks, 200 µL of the prepared mixture was further injected subcutaneously for antibody boosting. After one week of boosting, blood was collected and immunization titration was performed by the ELISA method using the administered antigen. Subsequently, third boosting was performed by subcutaneous injection of antigen alone.

The spleen of the immunized mouse was removed, and the spleen cells were obtained from the spleen. The spleen cells were suspended in Hybridoma-SFM medium (Thermo Fisher Scientific, USA) supplemented with 10% FBS. To prepare the hybridoma, the spleen cells and SP2/0-Ag14 of a murine myeloma cell were mixed in a Hybridoma-SFM medium without serum, and followed by centrifugation to remove the medium. Then, PEG was added to the obtained cell pellet and incubated at 37 °C for 1 minute to induce cell fusion.

### 1-2: Single cell cloning and Purification of antibodies

After 2 weeks in the fusion, the fusion with mouse B cells producing antibodies was confirmed with an ELISA method using the antigen administered to the mouse and a cell culture medium. Then, single-cell cloning was carried out using a hybridoma to select 16 hybridomas producing monoclonal antibodies. 9B11 clones (IgG1 kappa) were obtained using the aggregate of full length (140 residues) α-Syn as an antigen, and Clones of 3A9 and 11F11 (IgG2b kappa, and IgG2b kappa, respectively) were obtained using α-Syn aggregates with cleaved C-terminal 21 residues as antigens.

In order to purifying the antibody, each hybridoma was cultured in RPMI1640 medium containing 10% FBS. For antibody production, the culture medium was replaced with serum-free SFM medium and cultured for about 4 days. The cell culture supernatant was separated, centrifuged, filtered with a 0.22 µm filter, and purified with a protein G column for IgG1 type and the protein A column for the remaining antibodies.

### 1-3: Determination of variable region sequence

The variable region and CDR sequences were determined by referring to the disclosure Ahn et of al, Mol. Cells 2004, 18 (2): 237-241. Hybridomas were cultured and centrifuged to isolate only the cells. The RNA was isolated from the isolated hybridoma by the addition of a triazole and was used for synthesize cDNA as a template.

### Example 2. Preparation of anti-alpha-synuclein (chimeric) antibodies

### 2-1: antibody cloning and expression

By using the nucleotide sequences of the heavy chain variable region and the light chain variable region antibody obtained after humanization, gblock (m.biotech) of a short nucleotide fragment was synthesized, and cloned into the animal cell culture vector (pcDNA3.4). The gblock was synthesized by including about 20 bp overlapped sequence before and after the variable region, and the part of the pcDNA3.4 vector excluding the variable region was amplified by PCR and cloned by Gibson assembly method.

In order to transfect and express the cloned antibody, the prepared vector was used for maxi-prep (Qiagen) to obtain a large amount of plasmid DNA, and then introduced into cells as follows. The day before transfection, the concentration of ExpiCHO ^{™} (Gibco, Cat: A29127) cells was adjusted to concentration of 3 × 10E6 to 4 × 10E6 viable cells / mL in in ExpiCHO ^{™} expression medium (Gibco, Cat: A29100-01) and cultured at 8% CO₂, at 37 °C and 120 rpm for 1 day. On the day of DNA transfection, the cells that were grown to 7 × 10E6 to 10 × 10E6 viable cells / mL and had survival rates of 95% or more were prepared by diluting using fresh medium.to 6 × 10⁶ viable cells / mL.

In order to transfect the parent cells, ExpiFectamine^{™} CHO & plasmid DNA complex was prepared by using the ExpiFectamine ^{™} CHO transfection kit (Gibco, Cat: A29129). DNA and ExpiFectamine ^{™} CHO reagents were prepared at appropriate concentrations by dispensing with cold OptiPRO ^{™} SFM^{®} (Gibco, Cat: 12309019) medium, were respectively inoculated, and mixed to stand at room temperature for 5 minutes. The product was inoculated into parent cells, and cultured after transfection. The day after transfection, the enhancer and feed included in the ExpiFectamine ^{™} CHO transfection kit were inoculated into transfected cells, and after 5 days, the feed was additionally inoculated, followed by incubation for 10 days at 8% CO2, 37 °C, and 120 rpm to produce the transfected cells.

In order to obtain the culture solution, the culture medium was transferred to a centrifuge bottle for centrifugation and centrifuged at 4 °C and 6500 rpm for 30 minutes, followed by filtering with a filter having a size of 0.2 µm to obtain a culture medium with removing * suspended solids, and then the culture medium was used for subsequent purification.

### 2-2: Purification and sequencing of antibody

The culture was purified using HiTrap MabSelectSure (GE Healthcare, 11-0034-94). After equilibrating with an equilibration buffer (50 mM Tris-HCl pH7.2, 100 mM NaCl), the recovered culture was loaded onto a column. When the loading was completed, the medium was washed with 50 mM Sodium Citrate (pH 5.0), and then eluted using 50 mM Sodium Citrate (pH 3.4). 1M Tris-HCl pH 9.0 was added to the eluate to neutralize to pH 6.0. Then, the eluate was buffer exchanged and concentrated with PBS (phosphate buffered saline, pH 7.4) and stored at 4 °C until subsequent use.

When additional purification was required, a second purification was performed based on the size of the eluted sample by passing the first purified product through 1X PBS buffer on the HiLoad 26/600 superdex 200 column. The amino acid sequence of the purified antibody was analyzed by mass spectrometry, and confirmed to be consistent with the variable region of the mouse-derived monoclonal antibody.

The backbone variable region portion of the human IgG1 isotype was replaced with the variable regions of the 3A9, 9B11, and 11F11 antibodies identified by the above method to prepare a chimeric human IgG1 antibody. Among the obtained chimeric antibodies, especially Ch11F11 antibody is an antibody in the form of IgG and comprises a combination of the heavy chain variable region sequence of SEQ ID NO: 175 (ch11F11-VH). The bold part in table 11 corresponds to CDR regions.

**[Table 11]**

| Name | Amino acid sequence | SEQ ID NO |
|---|---|---|
| ch11F11-VH | EVQLQESGGGLVQPGGSLRLSCATSGFTFSDFYMEWV RQPPGKRLEWIAASRNKANDYTTEYSASVKGRFIVSR DTSQSILYLQMNALRAEDTAIYYCARDAHGKPFAYWG QGTLVTVSA | 175 |
| ch11F11-VI, | DIVMTQSPSSLAVSVGEKVTMSCKSSQSLLYSSNQKN YLAWYQQKPGQSPKLLIYWASTRESGVPDRFTGSGSG TDFTLTISSVKAEDLAVYYCQQYYSYPWTFGGGTKLEI K | 176 |

### Example 3: Production of humanized antibody

### 3-1: Library phage preparation

A mini-library in which a mouse or human-derived sequence was introduced into each CDR residue was constructed, while binding the human framework to the CDR1, CDR2, and CDR3 residues of the chimeric antibody.

The competent cells of the produced min library were inoculated in 2X YT medium [ 17 g of Tripton (CONDA, 1612.00), 10 g of yeast extract (CONDA, 1702.00) and 5 g of NaCl (Sigma, S7653)] containing 34 µg/ml of chloramphenicol (Sigma, C0857), 2% glucose (Sigma, G5400) and 5 mM MgCl₂ (Sigma, C0857) at 30 °C for 3 hours to be OD600 of 0.5 to 0.7. Then, the cells were infected with a helper phage, and cultured in 2X YT medium containing 34 µg/ml of chloramphenicol, 5 mM MgCl₂, 70 µg/ml of kanamycin (Sigma, K1876) and 1 mM IPTG (ELPISBIO, IPTG025) at 30 °C for 6 hours to induce the phage packing. The culture solution was centrifuged at 4500 rpm at 4 °C for 15 minutes. The supernatant was added with 4% PEG 6000 (Fluka, 81253) and 3% NaCl (Sigma, S7653) and incubated for 1 hour on ice. The product was centrifuged at 8000 rpm for 20 minutes at 4 °C, and then, the pellet was suspended in PBS and centrifuged again at 4 °C and 12,000 rpm for 10 minutes to obtain a supernatant containing the phage library. The obtained supernatant was stored at 4 °C until subsequent use.

### 3-2: Phage display panning

In order to select antibodies that preferentially bind to alpha-synuclein aggregates to the monomers, the panning was performed using the full-length alpha-synuclein aggregates prepared in Example 1, and total three panning were performed as follows.

Bovine serum albumin (BSA) was added to the cells at a concentration of 3 % in a test tube at 4 °C overnight, adding 10 µg/ml of recombinant alpha-synuclein aggregates and monomers to the PBS in an immunotube (maxisorp 444202) solution was added to the test tube and the surface of which alpha-synuclein aggregates and monomers were not adsorbed was protected. After emptying the test tube, the antibody phage library of 10¹² CFU dispersed in BSA 3% solution was put into the immunotube in which the alpha-synuclein aggregates and monomers were absorbed and reacted for 1 hour (negative selection). Then, the phages were not bound to alpha-synuclein aggregates and monomers were recovered and reacted for 2 hours at room temperature in the alpha-synuclein aggregates and monomers were adsorbed. Phosphate buffered saline (0.05% Tween 20) solution was used to recover 100 µM triethylamine solution, which was recovered by using a PBS-T solution. E. coli at 37 °C for 1 hour, and the infected E. coli was painted out on a 2X YT agar medium and cultured at 37 °C overnight (pH 7.4), they were infected by ER2537. On next day, the cultured E. coli was suspended in 4 ml of 2X YT culture solution containing carbenicillin and 15% glycerol was added, and a part was stored at - 80 °C and the rest was used for preparing phages for next experiments. By repeating this process at 3 rounds in total, alpha-synuclein antigen-specific phage pool was amplified and concentrated. As the panning round progressed, the number of washing using PBS-T was increased to amplify and concentrate the antigen-specific phage.

### 3-3: Single clone screening

To sort monoclonal antibodies specifically binding to alpha-synuclein aggregate from the phage pool obtained through the panning, the experiment as follows was performed.

To isolate monoclones from the concentrated pool, after painting out the phage pool on a LB-tetracycline/carbenicillin agar medium and culturing, a single colony was secured. Then, after inoculating monoclones on a 96-deep well plate in which 400µℓ of 2X YT-tetracycline/carbenicillin medium was put per well and growing overnight, 10µℓ culture solution was put on a new 96-deep well plate in which 390µℓ of 2X YT-tetracycline/carbenicillin medium was put and it was cultured at 37°C for 4 hours. 1mM IPTG was put into the culture solution and it was cultured at 30°C overnight. The culture solution cultured overnight was centrifuged to take a supernatant.

Then, the clones expressing a monoclone-soluble scFv which binds to alpha-synuclein aggregate were selected by using the ELISA method. Specifically, the selected 7B7 antibody in Example 1-1 was put on a 96-well plate (*Nunc-Immuno Plates*, *NUNC*, *USA*) and it was coated at 4°C overnight. 3% BSA was added to each well in an amount of 200 µL, followed by blocking at 37 °C for 2 hours. Then, the alpha-synuclein aggregates and the monomer were loaded at a concentration of 100 ng/well reacted at 37 °C for 2 hours and washed five times with 300 µL of PBS-T. The prepared single clone supernatant was mixed with 3% BSA in a volume ratio of 1: 1 (vol: vol), and 100 µL of the solution was loaded on the plate bound to the aggregate and the monomer, followed by reaction at 37 C for 2 hours. The cells were washed five times with 300 µL of PBS-T, and incubated at 37 °C for 1 hour with an anti-HA HRP-conjugated antibody, followed by washing with PBS-T five times. After adding 100 µL of TMB (Tetramethylbenzidine, Sigma, T0440), the reaction was stopped by adding 50 µL of 1 N H 2 SO 4 to measure the absorbance at 450 nm. Clones with an absorbance of 0.5 or greater were regarded as positive reaction by binding and clones bind to BSA nonspecifically were excluded.

The CDR residues of clones found in the library are analyzed in silico in parallel and the clones to cause serious problems with binding to the framework or clones that do not have T-cell epitope, B cell epitope, and MHCII epitope in the framework parts other than CDR were selected.

Thereafter, the variable regions of the selected clones substituted for the backbone variable region of the human IgG1 isotype to prepare an IgG1 backbone humanized antibody. Specifically, hu11F11 (H2L4) is an IgG-type antibody of a combination of Hu11F11-VH2 of SEQ ID NO: 146 and Hu11F11-VL4 of SEQ ID NO: 148; Hu11F11_(ver. 1) is an IgG-type antibody of a combination of Hu11F11-VH-v1 of SEQ ID NO: 142 and Hu11F11-VLv3 4c of SEQ ID NO: 147; Hu11F11_(ver.3) is an IgG-type antibody of a combination of Hu11F11-VH-v3 of SEQ ID NO: 144 and Hu11F11-VLv3 4c of SEQ ID NO: 147; and Hu11F11 (ver.4) is an IgG-type antibody of a combination of Hu11F11-VH-v4 of SEQ ID NO: 145 and Hu11F11-VLv3 4c of SEQ ID NO: 147.

### Example 4. ELISA assay of anti-alpha-synuclein antibody

Sandwich ELISA was performed to quantitatively analyze the binding affinity of the chimeric antibody (Ch11F11) obtained in Example 2 and the humanized antibodies (Hu11F11) obtained in Example 3.

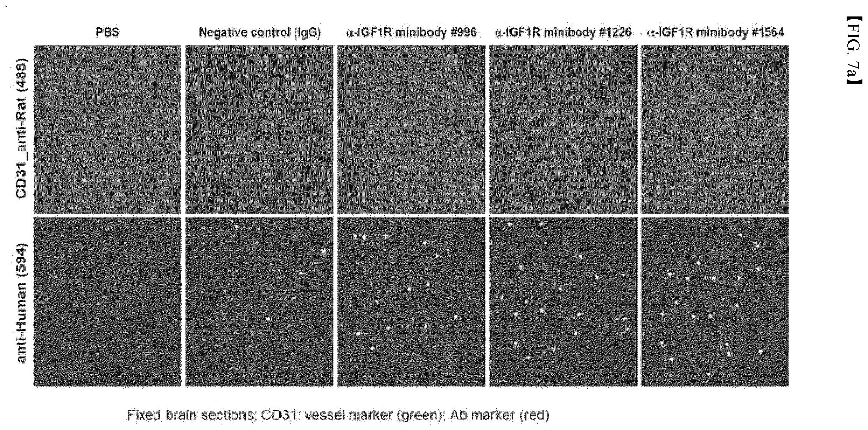

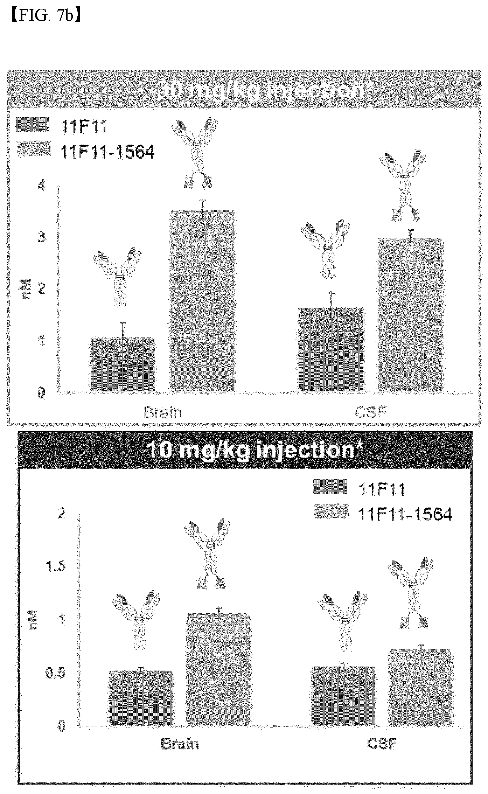

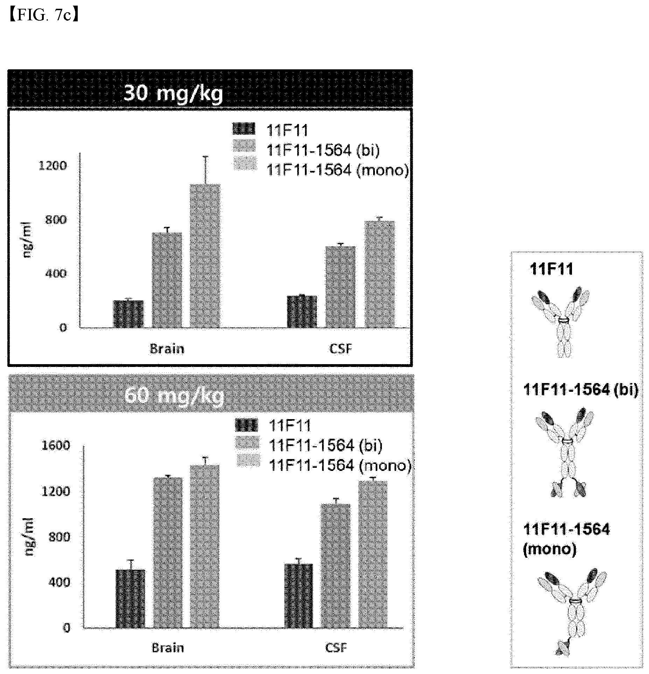

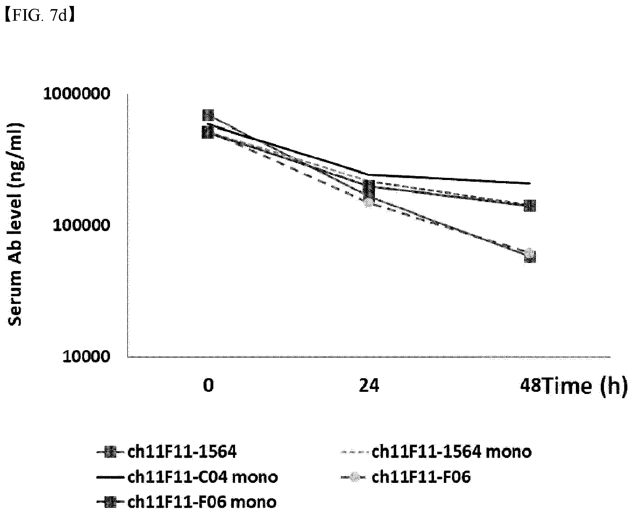

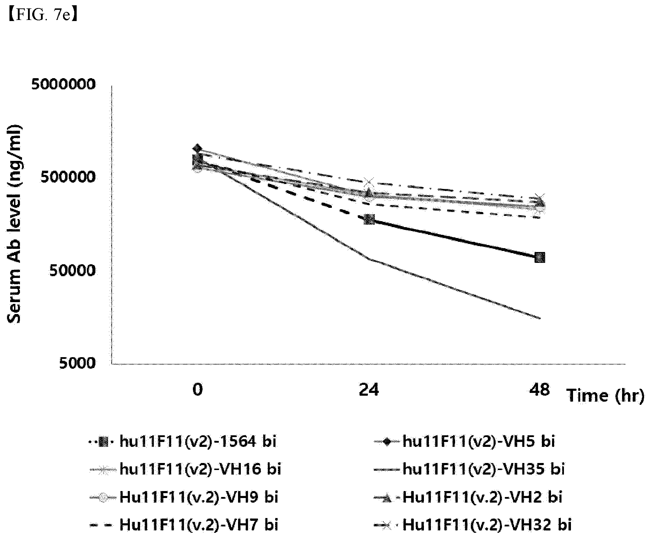

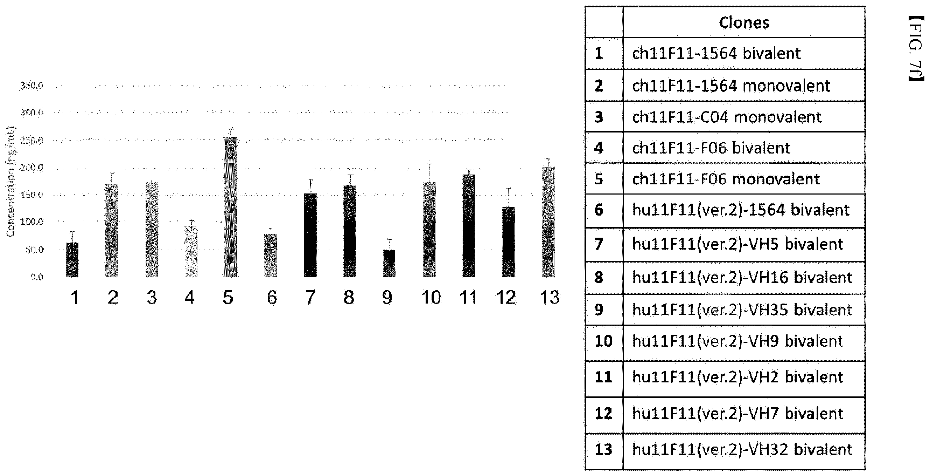

Specifically, each antibody was diluted by 1/10 to a concentration of 0.04 to 400 nM and coated on a 96-well plate, and 2000 ng/ml aggregates were treated in each well. After washing with 1XPBS, biotin-conjugated capture antibody and HRP-conjugated streptavidin were treated, followed by reaction with TMB as a substrate, and absorbance thereof was measured. The results are shown in FIG. 7.

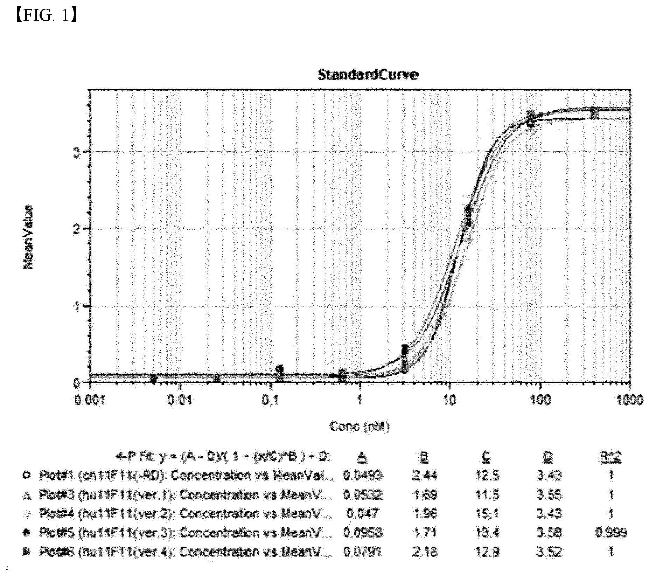

As shown in FIG. 1, it was confirmed that the humanized antibodies according to the present invention, particularly the humanized antibody derived from chimeric 11F11 (humanized 11F11 antibody), exhibited the binding affinity equivalent to that of the chimeric 11F11 clone. The humanized antibodies, especially variants derived from 11F11 such as hu11F11(ver.1) having a combination of Hu11F11-VH-v1 and Hu11F11-VLv3 4c, hu11F11(ver.2) having a combination of Hu11F11-VH-v2 and Hu11F11-VLv3 4c, hu11F11(ver.3) having a combination of Hu11F11-VH-v3 and Hu11F11-VLv3 4c, and hu11F11(ver.4) having a combination of Hu11F11-VH-v4 and Hu11F11-VLv3 4c, showed equivalent binding affinity to the chimeric 11F11 clone. Their EC₅₀ values were 11.5 to 15.1 nM, which was similar to 12.5 nM of EC₅₀ in the chimeric 11F11.

### Example 5. BIAcore analysis using anti-alpha-synuclein antibody

Many chimeric antibodies produced in Example 2 and the humanized antibodies in Example 3 were quantitatively analyzed using the binding affinities of many humanized antibodies.

The used instrument was T200 (GE Healthcare, S / N: 1565888). Protein A is used as a chip (GE Healthcare, Cat. 29-1275-56). 10 mM Glycine-HCl pH 1.5 (GE Healthcare, Cat. BR-1003-54) was regeneration buffer. The running buffer, analyte dilution, and the sample dilution buffer were HBS-EP. The antibodies prepared in Example 2 and Example 3 were diluted with 1 × HBS-EP (GE Healthcare, Cat. BR-1006-69), and alpha-synuclein monomer (1 mg/ml) and fibril protein (3 mg / ml) were serially diluted in duplicate and analyzed at 6 concentrations (0, 0.39, 1.56, 6.25, 25, 100 nM) including 0 nM in total. For the capture, the monomer was for RU of 800 (theoretical), and a fibril was for RU of 100 (theoretical). The capture phase was performed at contact time of 60 seconds, a flow rate of 30 µl / min, and a stabilization period of 180 seconds. The association phase was performed at the association time of 120 seconds and the flow rate was 30 µl/min. The dissociation phase was performed at the dissociation time of 360 seconds and the flow rate of 30 µl/min. The regeneration phase was performed twice time at the regeneration time of 240 seconds (primary) and 60 seconds (secondary) and a flow rate of 30 µl/min. The fitting was carried out suing 1: 1 binding model, and the evaluation software was BIACore T200 Evaluation software (GE healthcare).

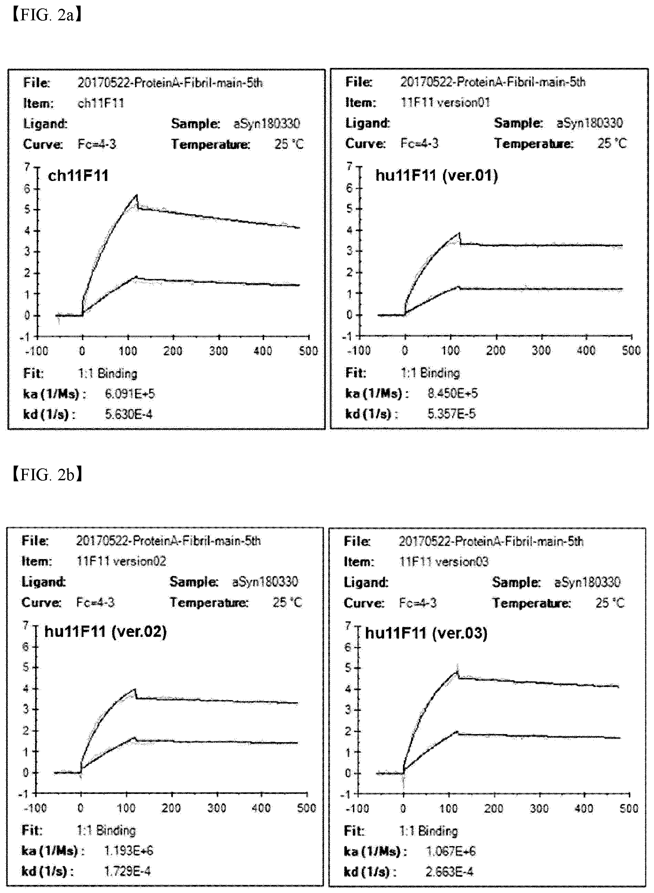

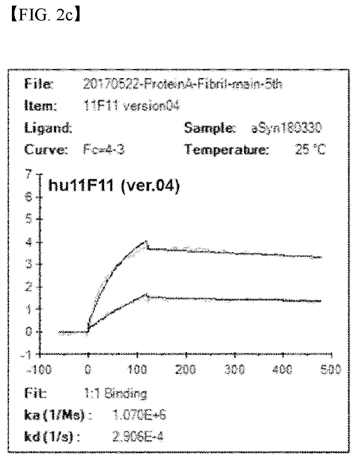

The analysis results are shown in Figs. 2a to 2c and the following Table.

**[Table 12]**

| Clone ID | K_{D}(nM) |
|---|---|
| Ch11F11 | 0.02472 |
| Hu11F 11 (ver.2) | 0.0596 |
| Hu11F11(ver.3) | 0.0316 |
| Hu11F11(ver.4) | 0.0204 |

As a result, the humanized antibodies in the expression, especially the humanized antibodies of chimeric antibody 11F11, such as hu11F11 (ver.2), hu11F11 (ver.3) and hu11F11 (ver.4), showed the same KD value as chimeric antibody 11F11. The binding of humanized antibodies had KD of 0.02 to 0.06 × 10⁻⁹ M, and low KD of 0.02 × 10⁻⁹ M of chimeric antibody, which was high affinity for floating bodies.

### Example 6. Production of IGF1R antibody (scFV)

### 6-1: Preparation of IGF1R antibody (scFV)

The monoclonal antibodies were prepared by using the phage display/panning technique. Specifically, the antigens used in the phage display panning and other analysis were used as the following proteins. The peptide consisting of 31 to 932 residues of the amino acid sequence of SEQ ID NO: 99, in which the signal sequence was excised from the extracellular domain of human IGF1R, was tagged with Histidine at C-terminus and used for this example (R&D Systems, USA, 391-GR). Monkey IGF1R (National Research Council Canada), mouse IGFIR (R&D systems, 6630-GR/CF), and rat IGF1R (National Research Council Canada) with His tag at C-terminus were used as an antigen for testing the interspecific cross-reactivity.

1 × 10¹⁰ of the ScFv (Single-chain variable fragment) library cells with diversity which were derived from human (prepared by SHIM Hyunbo at Ehwa Womon's University) were inoculated in 2X YT medium [17 g of Tripton (CONDA, 1612.00), 10 g of yeast extract (CONDA, 1702.00) and 5 g of NaCl (Sigma, S7653)] containing 34 µg/ml of chloramphenicol (Sigma, C0857), 2% glucose (Sigma, G5400) and 5 mM MgCl₂ (Sigma, C0857) at 30 °C for 3 hours to be OD600 of 0.5 to 0.7. Then, the cells were infected with a helper phage, and cultured in 2X YT medium containing 34 µg/ml of chloramphenicol, 5 mM MgCl₂, 70 µg/ml of kanamycin (Sigma, K1876) and 1 mM IPTG (ELPISBIO, IPTG025) at 30 °C for 16 hours to induce the phage packing. Subsequently, the culture solution was centrifuged at 4500 rpm at 4 °C for 15 minutes. The supernatant was added with 4% PEG 6000 (Fluka, 81253) and 3% NaCl (Sigma, S7653) and incubated for 1 hour on ice. The product was centrifuged at 8000 rpm for 20 minutes at 4 °C, and then, the pellet was suspended in PBS and centrifuged again at 4 °C and 12,000 rpm for 10 minutes to obtain a supernatant containing the phage library. The obtained supernatant was stored at 4 °C until the subsequent use.

### 6-2: Phage display panning

In order to screen the human IGFIR antibody, the panning was performed at three rounds according to the following. The phage library was synthetic human scFv library and the procedure of phage display panning and the result were shown in Table 13.

**[Table 13]**

| Step | | Panning | | |
|---|---|---|---|---|
| | | 1 round | 2 round | 3 round |
| Antigen | | IGF1R ECD (biotinylated) | IGF1R ECD (biotinylated) | MCF-7 cell |
| Coating method | | Indirect Immobilization | Indirect Immobilization | - |
| Input | | 7.0 x 10¹² | 6.0 x 10¹² | 5.0 x 10¹² |
| Output | IGF1R or MCF-7 | 4.9 x 10⁸ | 3.3 x 10⁵ | 1.2 x 10⁵ |
| washing | PBS-T** | 5 times | 10 times | 10 times |
| | PBS | 2 times | 2 times | 2 times |

Specifically, 1 ml of recombinant human IGF1R protein at a concentration of 5 ug/ml (R&D Systems, USA, 391-GR or Sino Biological Life Technologies, USA, 10164-H08H-50R) was added to an immunotube (maxisorp 444202) and coated on the surface of immuotubu at 4 °C for 16 hours. Then, the supernatant was removed and incubated with the addition of PBS containing 3% of BSA at 37 °C for 1 hour to block the non-specific binding by binding the BSA to the surface unbound by IGF1R. After removing the supernatant, the phage library prepared in Example 11-1 mixed with BSA 1.5% solution was put into the immunotube and reacted at 37 °C for 1 hour to allow the IGF1R specific phage to bind to antigen. Then, the product was washed with PBS-T solution (phosphate buffered saline-0.05% Tween 20) to remove the phage binding non-specifically, and the phage binding to IGF1R was collected with 100 mM triethylamine solution.

The collected phage was neutralized with 1M Tris buffer solution (pH 7.4) and transfected with E.coli K12 ER2738 at 37 °C for 1 hour, and the infected E. coli was spread out on LB agar medium containing tetracycline and carbenicillin, and cultured at 37 °C overnight. Next day, the cultured E. coli was suspended in a 5 ml of SB (superbroth) medium containing tetracycline and carbenicillin and was added by 50% glycerol at the same volume. One part was stored at -80 °C, and 50 ul of product was suspended in 40 ml of SB (superbroth) medium containing tetracycline and carbenicillin, added with 10¹² PFU of VCSM13 helper phage and cultured with stirring at 37 °C for 1 hour. Then, the culture solution was added by Kanamycin and cultured at 30 °C for about 16 hour, so as to culture only E.coli infected with the helper phage.

The next day, after centrifuging the culture solution, the supernatant was taken, and added to a buffer containing 4% PEG8000 and 3% sodium chloride (NaCl), reacted at 4 °C for about 1 hour, and the phage was precipitated and centrifuged. After removing the supernatant, the precipitated phage pool was re-suspended in PBS buffer containing 1% BSA, and was used for the next round of panning. As the panning round progressed, the number of washing using PBS-T was increased to amplify and concentrate the antigen-specific phage.

### 6-3: Single clone screening

The cell clones showing the binding affinity to ECD(extracellular domain) of human IGF1R and MCF-7 expressing IFG1R were selected.

Specifically, to select the monoclonal antibodies specifically biding to IGF1R from the phage pool obtained in Example 11-2, the following experiment was performed.

In order to separate the monoclones from the concentrated pool, the phage pool obtained on the LB-tetracycline / carbenicillin agar medium was smeared and cultured to secure a single colony. After inoculating these colonies in a 96-deep well plate and incubating overnight, 10 ul of the culture solution was re-inoculated into the 96-deep well plate and incubated at 37 °C for about 4 hours in the same manner to obtain an appropriate OD (0.5 to 0.7). After adding 20 MOI helper phage to the culture solution, the mixture was reacted at 37 °C for 1 hour. Thereafter, kanamycin was added to the culture medium, and cultured overnight at 30 °C. On the next day, the culture medium was centrifuged and the supernatant was taken to perform ELISA to select IGF1R-specific phage (Steinberger. Rader and Barbas III. 2000. Phage display vectors. In: Phage Display Laboratory Manual. 1 sted.Cold Spring Harbor Laboratory Press NY.USA. Pp.11.9-11.12).

100 ng of recombinant IGF1R was added to each well in an ELISA plate, and reacted at 4 °C for about 15 hours to coat the antigen on the plate. To prevent non-specific binding, PBS buffer containing 3% BSA was added at 200 ul per well, and then reacted at 37 °C for about 1 hour. The supernatant was discarded.

100 ul of the solution containing the prepared monoclonal phage was put in each well, reacted at 37 °C for 1 hour, and washed 3 times with 300 ul of PBS-T. To detect the phage bound to the IGF1R antigen, the anti-HA HRP was diluted 1: 5000 in PBS buffer containing 3% BSA, and reacted at 37 °C for 1 hour. After washing with 300 ul of PBS-T at 3 times, 100 ul of TMB (Tetramethylbenzidine, Sigma, T0440) was added to develop color, and 50 ul of 1N H₂SO₄ was added to quench the reaction. By measuring the absorbance at 450 nm, the clones with high absorbance compared to the control group of BSA were selected as antigen-specific antibody clones. 1564 clones were selected by screening twice.

### Example 7: Production of affinity variant of anti-IGF1R antibody

Antibodies were optimized by carrying out affinity variation for the selected clones by evaluating ligand binding capacity and BBB penetration ability. In the first trial, NNS hand-mix primers were prepared to randomize heavy chain CDR2 and light chain CDR3 based on 1564 scFv and amplified 1564 scFv gene containing randomization sequence using PCR technique. The amplified gene products were inserted into the pComb3x vector to make a library form suitable for phage display, and a number of scFv clones binding to IGF1R could be selected through the library panning and ELISA screening. For the selected clones, the amino acid sequences of the variable region were identified through gene sequencing.

In the second trial, two mini libraries were constructed for heavy and light chains that introduced germline back-mutation into CDR1, CDR2, and CDR3, respectively. The clones were finally obtained by selecting an affinity variant based on the productivity and antigen binding affinity of the clones.

### Example 8. Preparation of antibody variants having the deamidation residue

### 8-1:Deamidation residue identification

The deamidation reaction means, for example, that a symmetrical succinimide intermediate is formed by attacking the peptide bond in the side chain of asparagine, and this intermediate is converted to either aspartic acid or isoaspartic acid by hydrolysis. Particularly, when deamidation occurs in the CDR, the antibody is degraded and becomes the weak binding to the antigen, which may lead to reduced efficacy and sample heterogeneity. The sample heterogeneity causes complexity due to its identification in clinical approvals. Therefore, it was intended to identify the location where deamidation occurs, through in silico analysis and peptide mapping, and finally, to secure stability by preventing deamidation and to obtain superior property and efficacy simultaneously.

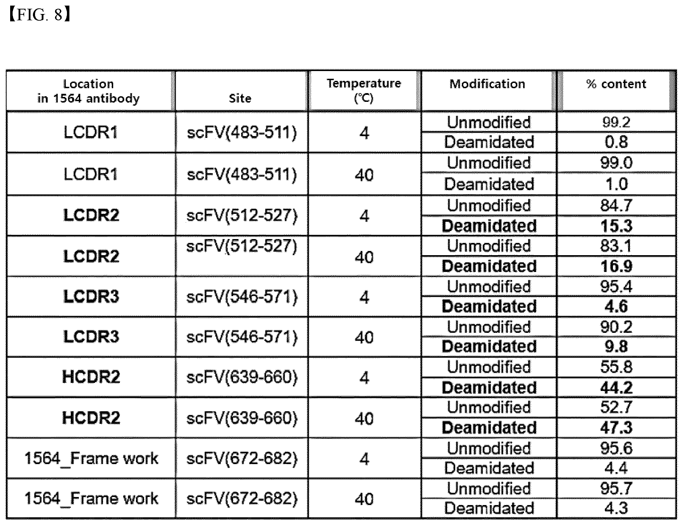

As shown in Fig. 8, the occurrence of actual deamidation was identified by in silico analysis and peptide mapping of the parental 1564 clone. In this regards, the samples were stored at 4 °C or 40 °C for one week before analysis, and it was confirmed that deamidation occurred in L-CDR2, L-CDR3, and H-CDR2. The affinity variants disclosed in Example 7 were also analyzed to confirm the location of deamidation.

### 8-2: Preparation of antibody variants

The process of removing the deamidation residue was performed by substituting with the following residue to prepare the mutant:
1) In the amino acid sequence, Asn was replaced with D or Q being similar to Asn. If the mutant had no change in the binding affinity, all the residues are replaced with Q.
2) N95a, the deamindation residue of LCDR3, was replaced with H, R, and K having positive charge. Clones subjected to this deamidation process are also referred to as (de)(StoP) deamidation clones.
3) Residues located immediately after the CDR where deamidation occurs were substituted. These residues are relatively small and low charged residues (e.g., glycine or serine). Therefore, by replacing the residues with other hydrophobic residues and relatively small size (e.g., valine or alanine), it was attempted to minimize the difference in binding affinity with the parental antibody (clones before residue substitution) (Table 21). The clones subjected to this deamidation process are also referred to as (de2)(StoP) deamidation clones. The table below shows how to replace the residue next to the residue where deamidation occurs.

**[Table 14]**

| Location at antibodies | **CDR (Seq.)** | Deamidation Site | Substitution |
|---|---|---|---|
| Light chain | 2 (A ***N*** S N R P S) | N51 | S52V |
| Light chain | 3 (G T W A G S L ***N*** G Y V) | N95a | G95bA |
| Heavy chain | 2 (S Y D ***N* G** N) | N54 | G55A |

Italic character: deamidation residue;
Bold and underlined character: residue to be replaced

### Example 9. Preparation of various forms of anti-IGF1R antibodies

### 9-1: Preparation of anti-IGF1R minibody

A minibody was prepared by connecting the whole scFv of the IGF1R specific monoclonal phage antibody obtained in Examples 6 to 8 to the C-terminus of Fc. To do so, the nucleotide sequence encoding the amino acid sequence of scFV disclosed in Table 12 was prepared, and the nucleotide sequence was cleaved with a restriction enzyme and cloned into a pcDNA-based expression vector containing a nucleotide sequence encoding Fc.

### 9-2: Preparation of anti-IGF1R bivalent antibody

The entire scFv of the IGF1R specific monoclonal phage antibody obtained in Examples 6 to 8 was prepared and two entire scFv were linked to each C-terminal of the therapeutic antibody in a IgG form to obtain bivalent antibody. To do so, the nucleotide sequence encoding the amino acid sequence of scFV disclosed in Table 12 was prepared, cleaved with a restriction enzyme, and cloned into a pcDNA-based expression vector containing a nucleotide sequence encoding a therapeutic antibody.

### 9-3: Preparation of anti-IGF1R IgG (Full-IgG) antibody

In order to convert the sequences of 1564 antibody and F06 antibody to full IgG1 (Full IgG) form among the IGF1R specific monoclonal phage antibodies obtained in Examples 6 and 7, the nucleotide sequences of heavy chain and light chain regions were synthesized (Genotec Inc.). The synthesized genes of heavy chain and light chain were cloned into expression vectors. One heavy chain linked with one molecule of anti-IGF 1R scFv, and the other heavy chain having no anti-IGF1R scFv and common light chain constituted the monovalent form of antibody.

### 9-4: Preparation of anti-IGF1R scFv monovalent antibody

Example 9-2 is a minibody form in which the scFv form of anti-IGF1R antibody is bound to each C-terminal of the two Fc of the heavy chain. In this example, one scFv is bound to C-terminus of only one Fc in a heavy chain. In the form of the antibody obtained in Examples 6 to 8, a vector in which 1564, F06, C04, VH5, VH16, VH35, VH9, VH2, VH7, and VH32 of the IGF1R specific monoclonal phage antibody was bound to the C-terminus of only one Fc, and a vector having no anti-IGF1R antibody bound to C-terminus were constructed. The knob-into-hole mutation was introduced into the Fc regions to produce a heteromeric form, when producing antibodies in cells.

### 9-5: Expression and purification of anti-IGF1R various antibodies

The vectors prepared in Examples 9-1 to 9-4 were introduced into cells as follows.

Specifically, CHO-S cells were adjusted to a concentration of 1.5 × 10⁶ cells / ml in CD-CHO (Gibco, 10743) medium, and then cultured at 8% CO ₂ at 37 °C for 1 day. On the day of DNA transfection, the cells grown to 2.5 to 3 × 10⁶ cells/ml were prepared at a concentration of 2.1 × 10⁶ cells / ml using CD-CHO medium containing 1% DMSO, and then were cultured under the condition of 8% CO₂, 37 °C for 3 hours. After centrifugation at 3000 rpm for 15 minutes, the supernatant was removed and re-suspended in RPMI 1640 medium with 2.5% FBS.

Subsequently, the combination of the vectors was diluted in Opti-MEM medium at 1 µg per ml of medium, and PEI (Polysciences, 23966, stock concentration: 1 mg / ml) was diluted 8 µg per ml of culture medium. After mixing the DNA and PEI mixtures and leaving the mixture at room temperature for 10 min, the mixture was poured in a flask containing cells and incubated for 4 hours at 5% CO₂, 37 °C, 100 rpm. Then, the mixture was cultured with addition of CD-CHO medium at the same volume as the culture volume and was incubated at 8% CO2, 37 °C, 110 rpm for 4 days.

The obtained culture solution was passed through an equilibration Mab selectsure (GE healthcare, 5 mL) equilibrated by passing with an equilibration buffer (50 mM Tris-HCl, pH 7.5, 100 mM NaCl) to allow the expressed antibody to bind to the column. Thereafter, after eluting with 50 mM Na-citrate (pH 3.4) and 100 mM NaCl solution, neutralization was performed using 1M Tris-HCl (pH 9.0) so that the final pH was 7.2. Thereafter, the buffer solution was exchanged with PBS (phosphate buffered saline, pH 7.4), and when the purity was high, it was stored at -20 °C after formulation and when the additional purification was required, it was stored at 4 °C until further purification

When the additional purification was required, it was purified using Hiload superdex 200 (GE Healthcare, Cat. No. 28-9893-36), and could be purified using a variety of different size exclusion chromatography. After equilibrating with an equilibration buffer (1x Phosphate buffered saline pH 7.4, Gibco, Cat. No. 10010-023), the primarily-purified sample was loaded on the column. The sample purified completely was stored in a frozen state at -20 °C after formulation.

### Example 10. Preparation of the bispecific antibody

The anti-IGF1R antibody in scFV form according to the present invention was prepared by linking the heavy chain variable region and the light chain variable region by using the liker (SEQ ID NO: 134), and was connected to C-terminus of heavy chain constant region of the complete form IGG of the anti-alpha-synuclein antibody by using a linker (SEQ ID NO: 133) to prepare the bispecific antibody. In addition, as a bispecific antibody format, a monovalent antibody was prepared by linking one molecule of the scFv form of the anti-IGF1R antibody per molecule of the IGG antibody of the anti-alpha-synuclein antibody, and the bivalent antibody was prepared by linking two molecules of the scFv form of the anti-IGF1R antibody per molecule of the IGG antibody of the anti-alpha-synuclein antibody respectively.

Exemplary sequences of the anti-alpha-synuclein antibody used for preparing the bispecific antibody in this example, and the combination of the recombinant heavy chain and the light chain of the bispecific antibody prepared according to the present invention are shown in the following table 10. The specific preparation method of bivalent bispecific antibody and the monovalent bispecific antibody are described below.

### 10-1: Bivalent bispecific antibody cloning

In order to construct a bivalent bispecific antibody expression vector, an antibody nucleotide sequence including a signal sequence was inserted into a multi cloning site (MCS) of the pcDNA3.4 vector (invitrogen). The bispecific antibody expression vector was a monocistronic vector, and heavy chain expression vectors and light chain expression vectors were prepared, respectively.

As the heavy chain sequence inserted to the heavy chain expression vector, the anti-IGF1R scFv was linked via a linker to C-terminus of the immunoglobulin where the heavy chain variable region encoding the anti-alpha-synuclein antibody and the human heavy chain constant region were linked. As the light chain sequence inserted to the light chain expression vector, the light chain variable region encoding the anti-alpha-synuclein antibody and the human light chain constant region were linked.

### 10-2: Monovalent bispecific antibody cloning

Monovalent bispecific antibody was a heterodimer comprised of a heavy chain (hole) of an anti-alpha-synuclein immunoglobulin in which the anti-IGF1R scFv was linked at C-terminus, and a heavy chain (knob) of an anti-alpha-synuclein immunoglobulinin which the scFv was not linked, and a light chain conjugated to the heterodimer.

In order to increase the conjugation efficiency of heavy chain heterodimer, Knob-in-hole technique was used. That is, the coding sequence of the heavy chain in hole type was replaced with T366S, L368A, and Y406V in the CH3 portion, and the coding sequence of the heavy chain in knob type was substituted with amino acid with T366W in the CH3 portion.

### 10-3: Transient expression

The prepared vector was performed with maxi-prep (Qiagen) to obtain a large amount of plasmid DNA. Then, they were introduced into cells as follows. In order to produce the monovalent BsAb, The expression vector DNA for the heavy chain and the expression vector DNA for the light chain were transfected at a ratio of 1: 1. To produce monovalent BsAb, the expression vector DNA for the heavy chin in hole type, the expression vector DNA for the heavy chin in knob type, and the expression vector DNA for the light chain were transfected at a ratio of 0.5: 0.5: 1.

At the day before transfection, the concentration of 3 × 10E6 to 4 × 10E6 viable cells / mL of ExpiCHO ^{™} (Gibco, Cat: A29127) cells was adjusted in ExpiCHO ^{™} expression medium (Gibco, Cat: A29100-01) medium, and then incubated at 8% CO₂, 37 °C and 120 rpm for 1 day. On the day of DNA transfection, the cells grown to 7 × 10E6 to 10 × 10E6 viable cells/mL and having survival rates of 95% or more were diluted to 6 × 10⁶ viable cells / mL with using fresh medium.

In order to transfect the parent cells, ExpiFectamine^{™} CHO and plasmid DNA complex was prepared by using the ExpiFectamine ^{™} CHO transfection kit (Gibco, Cat: A29129). Each of DNA and ExpiFectamine ^{™} CHO reagents was prepared at appropriate concentrations and inoculated on the old OptiPRO ^{™} SFM^{®} (Gibco, Cat: 12309019) medium which were dispensed and mixed to leave at room temperature for 5 minutes. The product was inoculated into parent cells, and began to culture after transfection. The day after transfection, the enhancer and feed included in the ExpiFectamine ^{™} CHO transfection kit were inoculated into transfected cells, and after 5 days, the feed was additionally inoculated and incubated for 10 days at 8% CO2, 37 °C, and 120 rpm to produce the transfected cells.

### 10-4: Medium harvest

In order to obtain the culture solution of the completed production, the culture medium was transferred to a centrifuge bottle for centrifugation and centrifuged at 4 °C and 6500 rpm for 30 minutes, followed by filtering with a filter having a size of 0.2 µm to obtain a culture medium with removing suspended solids. Then, the obtained culture medium was used for subsequent purification.

### Example 11. Analysis of IGF1R-specific binding affinity by using anti-IGF1R antibody

### 11-1: Analysis of IGF 1R-specific binding affinity by using anti-IGF1R antibody in a minibody form (ELISA)

The ELISA analysis was performed to test the binding affinity and the concentration-dependent binding of the minibody forms of the 996, 1226, 1564, and MKJP2 clones prepared in Example 9-1 to the recombinant IGF1R,

Specifically, human recombinant IGF1R, which is an antibody-binding target, is an extracellular domain (ECD), was purchased from R&D systems (6630-GR/CF). Human IGF1R was diluted with 1 ug/ml in PBS buffer, added at an amount of 100 ul per well in 96-well ELISA plate (Nunc-Immuno Plates, NUNC, Rochester, NY), coated by reacting at 4 °C for 16 hours, and then the supernatant was removed. PBS buffer containing 3% BSA (bovine serum albumin) was added to 200 ul per well and reacted for 2 hours to block non-specific binding.

The minibody antibodies of the 996, 1226, 1564, and MKJP2 clones prepared in Example 9-1 were diluted 3 times based on the highest concentration of 20 nM to make 12 points, and then transferred to each well 100 µl, and then treated at room temperature for 1 hour. After treatment, it was wash 4 times with PBS buffer containing 0.05% Tween20, and was reacted at room temperature for an hour by adding 100 ul of the anti-human HRP recognizing human Fc of the minibody diluted in blocking buffer at 1: 5000 per each well. After washing 4 times with 300 ul of PBS-T (Tween20 0.05%), the color development was performed using TMB (Tetramethylbenzidine, Sigma, T0440). The enzymatic reaction was quenched by 0.5 mol/L of sulfuric acid, and the absorbance was recorded and analyzed at 450 nm using a microplate reader. The experimental results are shown in Fig. 1a.

It was confirmed that the four minibody clones were bound to the human IGF1R recombinant protein in a concentration-dependent manner, and specifically, MKJP2 showed the highest binding ability, and subsequently, clones 996 and 1564 showed similar binding strength, and 1226 clone showed a slightly lower binding strength.

### 11-2: ELISA analysis of interspecific cross-reactivity of IGF1R antibodies

The interspecific cross-linking activity of the 1564 anti-IGF1R antibodies prepared according to the method of Example 9-2 and the anti-IGF1R antibodies obtained in Example 6-3 were analyzed by ELISA analysis. To this end, firstly, human, monkey, mouse and rat IGF1R antigens were diluted to 1 ug / ml, added to each well 100 ul, and reacted at 4 °C for 15 hours to be coated on the bottom of the plate. After removing the supernatant, 200 ul of PBS buffer containing 3% BSA was treated in each well to block non-specific binding. The anti-IGF1R antibodies were diluted by 5 times in PBSB (BSA 3% in PBS) at a maximum concentration of 400 nM, treated in each well, and reacted at 37 °C for 1 hour. Then, after washing with PBS buffer 5 times, the anti-human Fab HRP recognizing the Fab portion of the bound antibody was diluted 1: 20000 was treated at 100 ul of each well, and reacted at 37 °C for 1 hour. The product was washed 5 times with PBS buffer and the color development was performed with TMB (Tetramethylbenzidine, Sigma, T0440) according to the manufacturer's method. The enzymatic reaction was quenched by 0.5 mol/L sulfuric acid, and the absorbance was measured at 450 nm using a microplate reader (Molecular device). When many samples are used in the ELISA analysis, the plates were divided into two. The experimental results are shown in Table 15 below.

Specifically, ELISA results of bispecific antibodies to human IGF1R, ELISA results of 1564 IgG and bispecific antibodies to human IGF1R, ELISA results of bispecific antibodies to mouse IGF1R, and ELISA results of bispecific antibodies to rat IGF1R ELISA results, ELISA results of bispecific antibodies to monkey IGF1R are summarized in in Table 15 below.

The experimental results below show the advantages of evaluating the efficacy using animal models of various species, and thus, efficacy of therapeutic agents can be evaluated using the antibody according to the present invention in disease models of various species.

**[Table 15]**

| ELISA analysis results of antibody binding ability to IGF 1R of various species | | |
|---|---|---|
| Experiment | Antibody clone | Ec₅₀(nM) |
| ELISA for human IGF1R | ch11F11-1564 | 0.914 |
| | ch11F11-48G5 | 1.21 |
| | ch11F11-54H4 | 2.88 |
| | ch11F11-60H6 | 10 |
| | ch11F11-B11 | 7.13 |
| ELISA for human IGF1R | 1564 IgG | 0.0823 |
| | ch11F11-1564 | 0.379 |
| ELISA for mouse IGF1R | ch11F11 | N/A* |
| | ch11F11-1564 | 3.02 |
| | ch11F11 | N/A* |
| | ch11F11-48G5 | 6.2 |
| | ch11F11-54H4 | N/A |
| | ch11F11-60H6 | 18.6 |
| | ch11F11-B11 | 148 |
| ELISA for rat IGF1R | ch11F11 | N/A* |
| | ch11F11-1564 | 1.05 |
| | ch11F11-48G5 | 2.44 |
| | ch11F11-54H4 | 14.2 |
| | ch11F11-201** | N/A* |
| | ch11F11-1564 | 0.874 |
| | ch11F11-60H6 | 38 |
| | ch11F11-B11 | 35.1 |
| ELISA for monkey IGF1R | ch11F11 | N/A* |
| | ch11F11-1564 | 2.48 |
| | ch11F11-48G5 | 6.69 |
| | ch11F11-54H4 | 8.83 |
| | ch11F11-201** | N/A* |
| | ch11F11-1564 | 2.21 |
| | ch11F11-60H6 | N/A |
| | ch11F11-B11 | 180 |

| | | |
|---|---|---|
| *: not available *20 1: scFv form of Herceptin biosimilar | | |

### 11-3: The binding affinity analysis of the affinity variant to IGF1R (FACS)

The binding affinity of the affinity variants prepared in Example 7 was performed by ELISA for the ECD of IGF1R and the binding affinity for MCF-7 was analyzed by FACS.

As an analysis for the primary clones, Table 16 shows the results of the ELISA analysis for the ECD of IGF1R in the bispecific antibody form of the corresponding primary-selected clones, and Table 17 shows the result of analyzing the binding affinity to the MCF-7 cell line by FACS.

**[Table 16]**

| ELISA results of the binding of the bispecific antibody forms of primary-selected clones to ECD of IGF1R | |
|---|---|
| Antibody clone | EC₅₀(nM) |
| ch11F11-1564 | 0.442 |
| ch11F11-A06 | 1.19 |
| ch11F11-A07 | 1.2 |
| ch11F11-B02 | 0.919 |
| ch11F11-B09 | 1.08 |
| ch11F11-1564 | 0.49 |
| ch11F11-D03 | 0.666 |
| ch11F11-E06 | 0.668 |
| ch11F11-F06 | 0.467 |
| ch11F11-H04(G) | 0.67 |
| Hu3A9-1564 | 0.144 |
| Hu3A9-A02 | 0.13 |
| Hu3A9-A07 | 0.125 |
| Hu3A9-B10 | 0.156 |
| Hu3A9-B01 | 0.145 |
| Hu3A9-C04 | 0.107 |
| Hu3A9-E09 | 0.159 |

**[Table 17]**

| Results of FACS analysis of binding to MCF-7 cell line | |
|---|---|
| Samples | GEOmean |
| 2^{nd} Ab only | 2.92 |
| 1564 parental | 4.09 |
| F06 | 5.02 |
| A07 | 5.06 |
| B02 | 4.54 |
| B09 | 4.29 |
| D03 | 4.09 |
| E06 | 4.24 |
| F06 | 6.33 |
| C04 | 3.88 |

As a result, F06 clone was selected as the clone having the highest binding capacity in cell binding compared to the parental clone (1564 clone) (affinity matured), and C04 clone was selected as the clone with the lowest binding capacity in cell binding compared to the parental 1564 clone (affinity reduced).

As an analysis for secondary clones, Table 18 shows the ELISA results for the binding of bispecific antibody forms of clones made in the secondary production method to the ECD of IGF1R.

**[Table 18]**

| ELISA results of secondary-selected clones to the ECD of IGF1R | |
|---|---|
| Antibody clone | EC₅₀(nM) |
| Hu11F11(ver.2)-1564 | 0.259 |
| ch11F11-1564 monovalent | 0.347 |
| Hu11F11(ver.2)-C04 | 0.15 |
| Hu11F11(ver.2)-F06 | 0.147 |
| Hu11F11(ver.2)-1564 | 0.864 |
| ch11F11-F06 | 0.857 |
| Hu11F11(ver.2)-VH2 | 135 |
| Hu11F11(ver.2)-VHS | 0.366 |
| Hu11F11(ver.2)-1564 | 0.157 |
| Hu11F11(ver.2)-VH7 | 402 |
| Hu11F11(ver.2)-VH9 | 6.06 |
| Hu11F11(ver.2)-VH16 | 0.236 |
| Hu11F11(ver.2)-1564 | 0.149 |
| Hu11F11(ver.2)-VH32 | 121 |
| Hu11F11(ver.2)-VH35 | 0.167 |
| Hu11F11(ver.2)-VH27 | N/A* |

The clones to be analyzed with FACS analysis were selected as shown in Table 19, after excluding the clones having significantly lowered productivity and physical properties among the secondarily-produced clones.

**[Table 19]**

| Clones to be analyzed with FACS analysis | | |
|---|---|---|
| Category of binding affinity | Antibody clone | Explanation |
| Binding affinity similar to parental 1564 clone | C04 | FACS and in vivo analysis |
| | F06 | FACS and in vivo analysis |
| | VH5 | FACS and in vivo analysis |
| | VH16 | FACS and in vivo analysis |
| | VH35 | FACS and in vivo analysis |
| Binding affinity decreased by 50 times | VH9 | FACS and in vivo analysis |
| | C12 | Undesired physical properties |
| Binding affinity decreased by 50 times or more | VH2 | FACS and in vivo analysis |
| | VH6 | Undesired physical properties |
| | VH7 | FACS and in vivo analysis |
| | VH27 | Undesired physical properties |
| | VH32 | FACS and in vivo analysis |

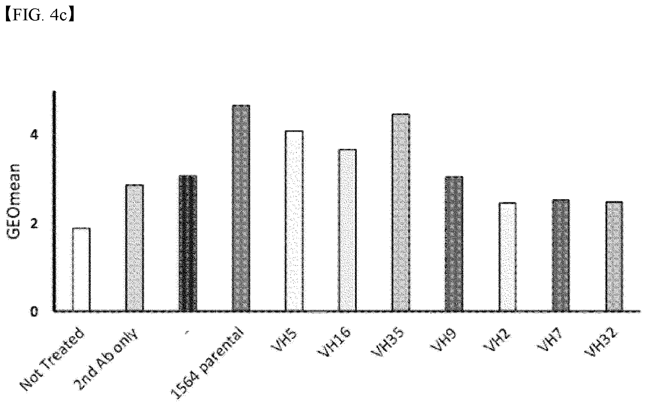

Fig. 4c is the result of analyzing the binding of the clones to the MCF-7 cell line by using FACS, and all of the analyzed clones had a lower binding affinity to MCF-7 compared to the parental clone 1564. The results show that the clones showing the decreased binding capacity in ELISA also showed decreased binding capacity in FACS.

The selected antibody clones are F06, C04, VH2, VH5, VH7, VH9, VH16 and VH32, and amino acid sequences for heavy chain variable regions and light chain variable regions for these antibodies are shown in Tables 4 and 5 above.

Among the antibody clones, deamidation hot spots present in the VH5, VH16, and F06 variants were removed according to Table 14 of Example 8-2 to prepare mutants, and the variants were prepared as bispecific antibodies according to Example 10. VH5 and VH16, were used for preparing bivalent bispecific antibodies with hu11F11 (ver.2), and F06 was used for preparing monovalent bispecific antibodies with hu11F11 (ver.2). Using the prepared bispecific antibody, three variants of VH5, VH16 and F06 (i.e., hu11f11-F06, hu11f11-VH5, hu11F11-VH16 as bispecific antibodies) and deamidated mutants (i.e., hu11f11-F06(de2)(StoP), hu11f11-VH5(de2)(StoP), hu11F11-VH16(de2)(StoP) as bispecific antibodies) were analyzed for binding to MCF-7 according to the above FACS analysis method. The components of the bispecific antibodies are described in Table 10, and the substitution of the deamidation site of these antibodies is described in Table 14 of Example 8-2.

The FACS analysis results are shown in Table 20 below. It was confirmed that the binding affinity of all three deamidated mutants (hu11f11-F06(de2)(StoP), hu11f11-VH5(de2)(StoP), hu11F11-VH16(de2)(StoP)) did not decreased compared to parental antibodies (VH5, VH16, F06).

**[Table 20]**

| Variant | WT | (de2)(StoP) | |
|---|---|---|---|
| | MFI | MFI | Binding affinity % to binding affinity of WT |
| F06 (de2)(StoP) | 9.61 | 8.61 | 89.59 |
| VH5(de2)(StoP) | 6.44 | 8.03 | 124.69 |
| VH16 (de2)(StoP) | 6.13 | 6.42 | 104.73 |

| | | | |
|---|---|---|---|
| * MFI = mean fluorescence intensity Negative control (2ndary Ab only)'s MFI: 2.29 | | | |

Using the bispecific antibodies prepared above, the binding ability of three variants of VH5, VH16 and F06 and their deamidated mutants to human IGF1R protein were analyzed by ELISA according to the method of Example 15-2. The results are shown in the following table. It was confirmed that the binding affinity of all three mutants did not decrease compared to the parental antibody.

**[Table 21]**

| Variant | WT | (de2)(StoP) | |
|---|---|---|---|
| | EC50 (nM) | EC50 (nM) | % WT |
| F06 (de2)(StoP) | 0.112 | 0.0575 | 195 |
| VH5 (de2)(StoP) | 0.125 | 0.0561 | 222.81 |
| VH16 (de2)(StoP) | 0.143 | 0.122 | 117.21 |

### 11-4: BIAcore analysis for Human IGF1R

The binding capacity of the antibody according to the present invention to human IGF1R was analyzed.

For the IgG form of the 1564 clone, the degree of binding to human IGF1R was analyzed by SPR analysis. The anti-his antibody against the His tag bound to the human IGF1R ECD as an antigen was diluted to 20 µg/ml in acetate pH4.0 buffer, and then immobilized in the reference/analytic channel of the CM4 chip to 10,000 RU as a target RU according to the amine coupling method. During capture, PBS was used as a running buffer, and the flow rate was maintained at 30 µL/min. During association/dissociation, the flow rate was 40 µL/min, and PBS was used as the running buffer. The association/dissociation was 5 minutes and 20 minutes, respectively. The analysis was performed in the order of baseline 1, activation (EDC NHS), human IGF1R loading, quenching (1 M Ethanolamine), baseline 2, association, and dissociation. Evaluation was performed using a bivalent model, and analyzed using Biacore T200 Evaluation software (version 1.0, S / N: 04Y15X11-0149).

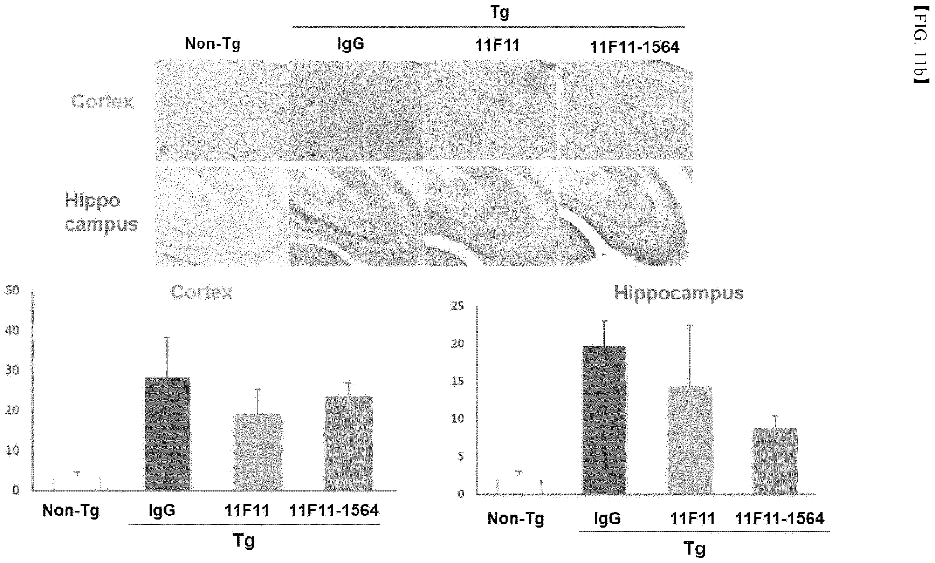

As a result of the analysis, the KD of the 1564 IgG antibody was confirmed to be 2.5305 × 10⁻⁹ nM, and the F06 IgG antibody was confirmed to be 4.7802 × 10⁻⁷ nM, all of which showed high binding ability to human IGF1R. The results of the analysis are shown in FIG. 11b. In particular, when the 1564 clone was produced in the form of IgG, the 1564 clone showed dissociation constant of 2.5305 × 10⁻⁹ nM to the human IGF1R, and the 1564 clone confirmed that there was no significant change in binding affinity depending on the forms of antibodies.

### Example 12. Binding ability analysis for anti-IGF1R antibody to cell line expressing human IGF1R and brain endothelial cells

### 12-1: FACS analysis for MCF-7

To confirm whether the minibody forms of clones 996, 1226, and 1564 prepared in Example 9-1 bind to endogenous IGF1R on the cell surface, the binding affinity analysis weas performed for cell lines expressing human IGF1R and brain endothelial cells by FACS. The degree of binding to MCF-7, which is known as a breast cancer cell line to overexpress IGF1R, was tested by FACS.

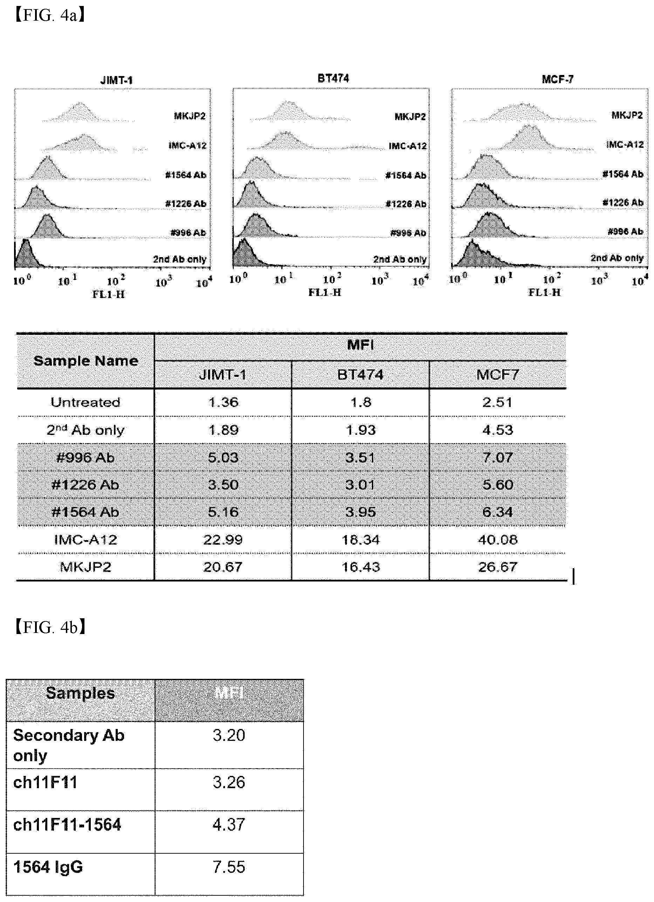

Specifically, each of the three minibody was diluted to 20 ug/ml, treated to 0.43 × 10E6 of the MCF-7 cell lines per sample, and reacted at 4 °C for 1 hour. After washing twice with PBS buffer, the anti-human FITC was diluted at 1: 500, treated and reacted at 4 °C for 1 hour. After washing twice with PBS buffer, the binding degrees of the anti-IGF1R minibodies were measured using a FACS Calibur instrument. MCF-7 cells treated with only secondary antibodies were used as a control. The experimental results are shown in Fig. 4a.

A02, A06, A07, B01, B02, B09, B10, C04, D03, E06, F06, H04 (Gly), H04 (Val), VH2, VH5, VH7, VH9, VH16, VH32 and VH35 prepared in Example 7 and Example 9-2 were analyzed for their binding affinity to MCF-7 in the same manner as above. Clone 1564 were prepared by the method of Example 14-2 and compared as parental clones, and MCF-7 cells treated with only secondary antibodies were used as controls. The analysis results are shown in Fig. 4c.

According to the results of the above experiment, it was expressed as Mean Fluorescence Intensity (MFI) of the sample, and the scFV in three minibodies, the affinity variants in the bispecific antibodies and the parental clone (1564 clone) bound specifically to the endogenous IGF1R expressed on the cell surface. The result shows that the clones obtained in the above examples can be used for the intended purpose by binding to IGF1R in a form actually present in the body.

### 12-2: FACS analysis for JIMT-1 and BT474

The minibodies of clones 996, 1226, and 1564 prepared in Example 12-1 in substantially the same manner, except that JIMT-1 and BT474 of breast cancer cell lines were used instead of the MCF-7 cell lines used in Example 14-1. The morphology was confirmed to bind to the endogenous IGF1R on the cell surface. The experimental results are shown in Fig. 4a.

According to the above experimental results, it was expressed as Mean Fluorescence Intensity (MFI) of the corresponding sample, and it was confirmed that scFvs in the tested three minibodies specifically bound to endogenous IGF1R on the surface of various cell lines expressing IGF1R.

### 12-3: FACS analysis of mouse brain endothelial cells

It was analyzed whether the bispecific antibody form of the 1564 clone prepared by the method of Example 9-2 and the IgG form of the 1564 clone prepared by the Example 14-3 method bound to bEND.3 of the brain endothelial cell. In this regards, the group treated only the secondary antibody and the group treated with only therapeutic antibody in IgG form (CH11F11) were used as negative controls. FACS analysis was performed in the same manner as in Examples 12-1 and 12-2. The analysis results are shown in Fig. 4b.

All tested clones showed the binding to bEND.3 except the negative controls. The results confirmed that various forms of clone 1564 specifically bound to IGF1R expressed on the surface of brain endothelial cells.

### Example 13. Intracellular internalization analysis of anti-IGF1R antibody

### 13-1: MCF-7 internalization assay - 1564, 996, 1226, MKJP2 (minibody)

The example was carried out to test whether the minibody forms of the 996, 1226, 1564, and MKJP2 clones prepared in Example 9-1 were intracellularly internalized in a cell line expressing IGF1R, and the antibody introduced into the cell was passed through the RMT Pathway without being degradation. In order that the anti-IGF1R antibody is used as a shuttle to improve BBB-penetrating capacity, the antibody should be internalized into brain endothelial cells constituting BBB.

The intracellular internalization of the antibodies according to the present invention was tested by using the MCF-7 cell line expressing IGF1R. Specifically, after plating 30,000 MCF-7 cell lines in an 8-well slide chamber, the cells were cultured for 1 day. The cultured cell lines were treated in each well at 4 °C for 2 hours with 5 µg/ml of minibody antibodies of the 996, 1226, 1564 and MKJP2 clones prepared in Example 9-1, washed three times with cold DMEM culture, and also treated with Alexa488-conjugated anti -human Fc antibody at 4 °C for 1 hour.

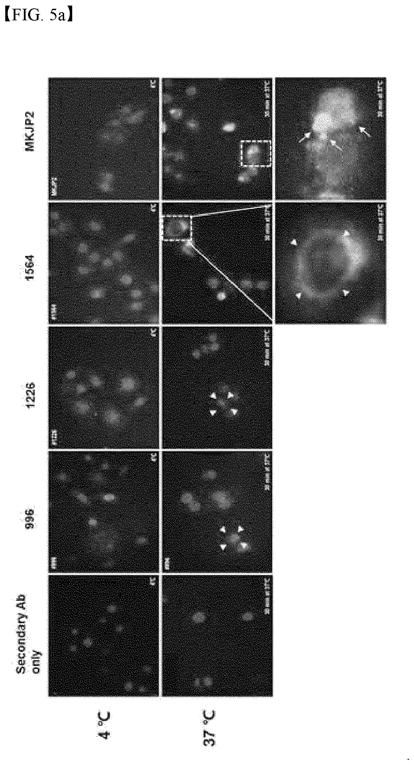

To test the internalization of the antibody complex, the plate was transferred to a CO₂ incubator and incubated at 37 °C for 30 minutes. The culture was fixed by adding 100% methanol and the reaction was terminated simultaneously. After fixation, it was washed 3 times with PBS. On the fluorescence microscope, the internalization degree of the antibody was imaged in the green filter region (Alexa488). In the imaging process, the nuclei inside cells were stained using DAPI to confirm the location of each cell. The experimental results are shown in Fig. 5a.

From the experimental results, all four antibodies tested in the experiment using the MCF-7 cell line were shown to be internalized well. In particular, it was found that the internalization of MKJP2 and 1564 occurred more than other clones.

### 13-2: MCF-7 internalization assay - C04, F06, VH5, VH16, VH35, VH9, VH2, VH7, VH32

1564 variants having the change in binding capacity to IGF1R were tested or the IGF1R binding on the cell surface by FACS analysis using MCF-7 cell line expressing IGF1R. 2 × 10E5 MCF7 cells were treated with the bispecific antibody made by the scFv anti-IGF 1R antibody at a concentration of10 ug/mL for 30 minutes. After washing with PBS buffer containing 1% BSA, the secondary antibody bound with FITC to detect human antibodies was treated for 1 hour. After washing with PBS buffer, FACS analysis confirmed the extracellular binding and internalization of various variants with the changed binding affinity.

As shown in Table 22, the bispecific antibody including 1564 IGF 1R antibody was found to have an increased internalization and an increased intensity at 37 °C than the refrigerated condition. These results suggest that the 1564 variants bind well to cells and internalize into the cells in a binding-dependent manner.

**[Table 22]**

| Sample | GeoMean |
|---|---|
| | Internalization at 37°C |
| Not Treated | 1.88 |
| 2nd Ab only | 2.86 |
| hu3A9 WT | 3.4 |
| hu3A9x1564 WT | 7.72 |
| hu11F11 WT | 3.18 |
| hu11F11x1564 WT | 7.34 |
| hu3A9x1564_C04 | 7.23 |
| hu3A9x1564_F06 | 19.8 |
| hu11F11x1564_VH5 | 6.1 |
| hu11F11x1564_VH16 | 5.83 |
| hu11F11x1564_VH35 | 7.28 |
| hu11F11x1564_VH9 | 5.01 |
| hu11F11x1564_VH2 | 3.19 |
| hu11F11x1564_VH7 | 3.84 |
| hu11F11x1564_VH32 | 3.24 |

### 13-3: Internalization analysis to human brain endothelial cells

It was tested whether the bivalent form and the monovalent form of the clone 1564 prepared in Examples 9-2 and 9-4 were internalized into primary human microvascular brain endothelial cells (HMBEC). The therapeutic antibody IgG (11F11) was used as a negative control.

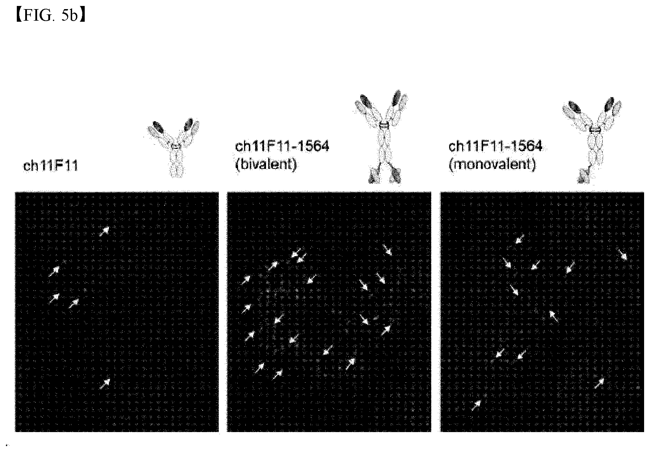

HMBEC (Cell Systems, cat #: ACBRI376) was plated in a 12-well plate at 90% confluency, and followed by test antibody After fixing with 4% paraformaldehyde and rinsing with PBS on next day, the blocking and permeabilizing were performed by using a solution containing 3% BSA and TritonX for 50 minutes. After rinsing with PBS, an antibody against human Fc (Goat anti-human antibody) was incubated for 2 hours and 30 minutes, rinsed with PBS, and treated with a secondary antibody against the corresponding primary antibody for 1 hour. After rinsing with PBS, the cells were stained by Hoechst for 10 minutes at a concentration of 1: 1000 for nuclear staining. The result was analyzed under the condition of LSM 780 NLO EC Plan-Neofluar 100X / 1.3 Oil with a confocal microscope. The experimental results are shown in Fig. 5b.

The bivalent form and the monovalent form of the 1564 clone showed an increased internalization compared to the negative control therapeutic antibody (11F11). This result shows that the anti-IGF1R antibody described above can effectively internalize the therapeutic antibody into brain endothelial cells constituting BBB, as various forms of bispecific antibodies containing a therapeutic antibody linked to it, thereby increasing BBB-penetrating ability of the therapeutic antibody.

### 13-4: Analysis of cellular fate in human brain endothelial cells

If the antibody is internalized and co-localized with a lysosome-related marker in the cell, the antibody cannot pass through the BBB due to the degradation in the brain endothelial cell. In contrast, if the antibody is co-localized with an early endosome associated with exocytosis or a marker known to be associated with BBB passage, the antibody is expected to cross the BBB by receptor-mediated transcytosis that is internalized into brain endothelial cells and then exits into the brain.

After treating HMBEC in the same manner with the 1564 bivalent form among the antibodies tested in Example 13-2, it was analyzed which cellular component in these cells co-localize with these antibodies. However, each of the following antibodies was treated simultaneously with Goat anti-human antibodies that detect the treated antibodies after blocking and permeabilization.
- Anti-Cathepsin D: lysosomal marker
- Anti-Caveolin-1: caveolin-mediated transcytosis marker (which is thought to be the main mechanism of BBB passage
- Anti-EEA1: early endosome marker

The remaining parts of the methods were the same as in Example 13-2, but the secondary antibodies to the markers were treated respectively.

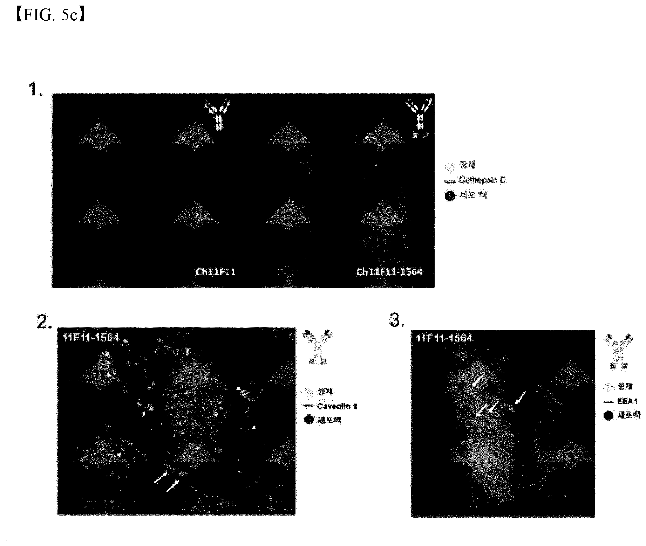

The analysis results are shown in Fig. 5c. The 1564 clone in the bispecific antibody form did not co-localize with Cathepsin D, but co-localized with caveolin-1 and EEA1 inside the cell membrane and cells. These results indicate that after the 1564 clone was internalized, it was possible to pass BBB through RMT pathway without going through the intracellular degradation mechanism.

### Example 14. Analysis of the effect of anti-IGF1R antibody on IGF1R signaling

### 14-1: Proliferation assay of MCF-7 cell line by using IGF1R

Whether the anti-IGF1R antibody according to the present invention interferes with the binding between IGF1R (IGF1 receptor) and its ligand was confirmed using cell proliferation efficacy by IGF 1.

The minibody antibodies of the 996, 1226, 1564 and MKJP2 clones prepared in Example 9-1 were diluted 5 times from 400 nM, respectively, to prepare diluted samples, and then 25 µl of the diluted samples were treated with 25 µl of 20 ng/ml IGF1, respectively. The MCF-7 cell lines expressing IGF1R was cultured, and passaged by removing the medium on the day of the experiment, and 20,000 cell lines of each well (corresponding to 50 µl) were added to a 96 well plate in which IGF1 and test antibody were dispensed.

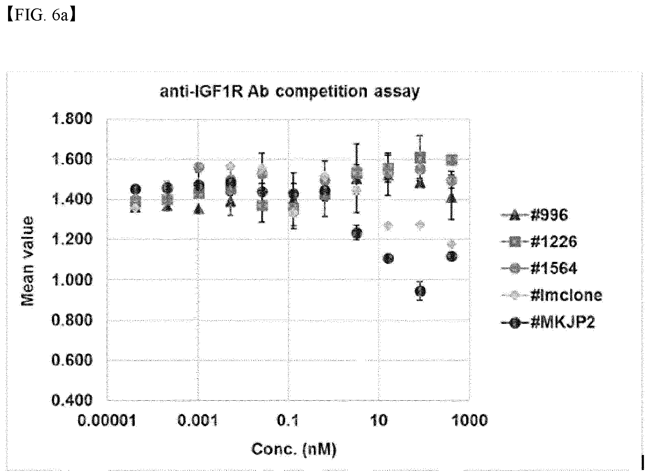

After incubating at an appropriate temperature and humidity for 3 days, 10 µl of CCK-8 reagent was treated in order to measure the degree of cell growth, and incubated in a CO₂ incubator for 4-5 hours. Then, it was taken out and the absorbance was measured at a wavelength of 450 nm with the spectrophotomer. The experimental results are shown in Fig. 6a.

According to the experimental results, it was confirmed that the antibody according to the present invention did not inhibit the cell proliferation of MCF-7 caused by the signaling of IGF1 to IGF1R. The anti-IGF 1R antibody (Imclone) as a control group inhibited the cell proliferation of MCF-7 by IGF 1 signaling to IGF1R in the treating-concentration dependent manner. Therefore, the antibody of the present invention is an antibody having the ability binding to IGF 1R expressed in endothelial cells constituting BBB and penetrating BBB, but does not inhibit signaling by IGF 1 in the body. Thus, it was confirmed that the antibody according to the present invention could be used as a BBB shuttle.

### 14-2: Analysis for IGF1R inhibition of signaling component in MCF-7 cell line

When IGF1 binding to the cells expressing IGF 1R delivered the signaling into cells, the anti-IGF 1R antibody according to the present invention was tested to determine whether IGF1 was involved in the receptor and downstream signaling component of the signaling, That is, anti-IGF1R antibody was treated to the MCF-7 cell lines expressing IGF1R, and then total IGF1R, phosphorylated IGF1R, total Akt as downstream factors of IGF1R, and phosphorylated Akt amount in the cells were analyzed.

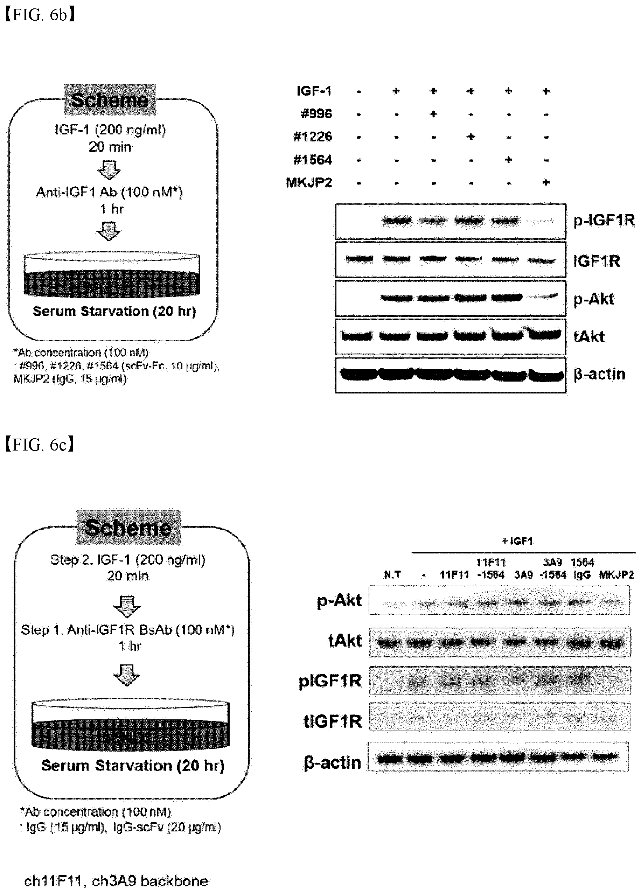

After culturing MCF-7 cells, the culture medium was changed to a serum-free culture medium at 20 hours before treatment with the anti-IGF1R antibody. The minibody antibodies of 996, 1226, 1564 and MKJP2 clones prepared in Example 4-1 were treated with 100 nM in the MCF-7 cell lines, respectively and treated with 200 ng/ml of IGF 1 after 1 hour. After 20 minutes, the cells were washed with PBS and then lysed with M-PER added by protease and phosphatase inhibitor cocktail. After measuring the protein concentration using the BCA assay kit, 12.5 µg of protein was loaded onto an SDS-PAGE gel for electrophoresis, and then transferred to a PVDF membrane. The blocking was performed at room temperature with gentle shaking for 1 hour with PBST (0.1% Tween 20) containing 5% BSA, and then the primary antibody against IGF1R or Akt was treated with slow shaking at 4 °C overnight. Beta-actin antibody was used as a loading control. After washing, the secondary antibody was treated with shaking slowly at room temperature for 1 hour, and then washed. ECL solution was added, and signals were observed using Image Quant Las 4000. The experimental results are shown in Fig. 6b.

According to the experimental results, it was confirmed that the antibody according to the present invention did not affect the total IGF1R, phosphorylated IGF1R, total Akt as a downstream factor of IGF1R, and the amount of phosphorylated Akt in the cells.

### 14-3: Analysis for IGF1R inhibition of signaling component in mouse brain endothelial cells

When IGF1 binding to the cells expressing IGF1R delivered the signaling into cells, the anti-IGF1R antibody according to the present invention was tested to determine whether IGF1 was involved in the receptor and downstream signaling component of the signaling, That is, 11F11-1564 and 3A9-1564 CH11F11 and ch3A9, alpha-synuclein monospecific antibodies described in Korean Patent Publication No. 2018-0081465) produced by the method of Example 9-2 and 1564 clone in IgG form produced by the method of Example 9-3 were treated to the bEND3 cell lines expressing IGF1R, and then total IGF1R, phosphorylated IGF1R, total Akt as downstream factors of IGF 1R, and phosphorylated Akt amount in the cells were analyzed.

While incubating the bEND3 cells, the culture medium was changed to a serum-free culture medium at 20 hours before treatment with the anti-IGF1R antibody. The bispecific antibodies of the 1564 and MKJP2 clones of Example 14-2 were treated respectively with 100 nM in the bEND cell line and treated with 200 ng/ml of IGF 1 after 1 hour. After 20 minutes, the cells were washed with PBS and then lysed with M-PER added by protease and phosphatase inhibitor cocktail. After measuring the protein concentration using the BCA assay kit, 12.5 µg of protein was loaded onto an SDS-PAGE gel for electrophoresis, and then transferred to a PVDF membrane. The blocking was performed at room temperature with gentle shaking for 1 hour with PBST (0.1% Tween 20) containing 5% BSA, and then the primary antibody against IGF1R or Akt was treated with slow shaking at 4 °C overnight. Beta-actin antibody was used as a loading control. After washing, the secondary antibody was treated with shaking slowly at room temperature for 1 hour, and then washed. ECL solution was added, and signals were observed using Image Quant Las 4000. The experimental results are shown in Fig. 6c.

According to the experimental results, it was confirmed that the antibody according to the present invention did not affect the total IGF1R, phosphorylated IGF1R, total Akt as a downstream factor of IGF1R, and the amount of phosphorylated Akt in the cells.

### Example 15. Analysis for in vivo BBB-penetrating ability of anti-IGF1R antibody (co-localization assay)

### 15-1. Minibody co-localization with brain vessel

The following experiment was conducted to confirm whether the anti-IGF1R antibodies of the present invention were distributed along the brain vasculature in vivo.

Specifically, PBS buffer or 10 mg/kg of IgG control, and the minibody antibodies of clones 996, 1226, and 1564 prepared in Example 14-1 were administered to the tail vein of a 6-8 week old BALB/c male mouse, respectively. After 4 hours, the mouse brain was intracardially perfused with a sufficient amount of 0.9% NaCl solution and 4% paraformaldehyde. The fixed brain was extracted and sectioned at 20 µm, and co-staining was performed with anti-mouse CD31 as a vascular marker, and anti-human Fc antibodies, to confirm co-localization of the brain vessels and the tested IGF1R. A secondary antibody conjugated with Alexa 488 for CD31, and the secondary antibody conjugated with Alexa 594 for human Fc were used for imaging CD31 and human Fc under a fluorescence microscope. The experimental results are shown in Fig. 7a.

According to the experimental results, it was confirmed that the non-blocking antibodies for the ligand binding according to the present invention had an excellent BBB-penetrating ability. As a result of the staining the brain tissues with vascular markers (anti-CD31, green) and human antibodies (anti-human Fc, red) according to the method of analyzing the antibody co-localization degree with cerebral blood vessels by immunostaining (Neuron (2016) Yu-Zuchero et al.), the non-blocking antibodies for the ligand binding according to the present invention showed a higher degree of co-localization than the IgG control group.

### 15-2. Analysis for in vivo BBB-penetrating ability of bispecific antibody

The anti-IGF1R antibody of the present invention was attempted to confirm *in vivo* BBB-penetrating ability in normal rats. PBS buffer or 10 mg/kg of IgG control, and therapeutic antibody for Parkinson's disease (11F11) or the bivalent bispecific antibody (11F11-1564) containing 1564 clone linked to the therapeutic antibody were administered to the tail vein of SD rats, respectively. At 24 hours, the amounts of antibodies in CSF and brain were analyzed by the mass spectrometry. The mass spectrometry was performed as the same method as Example 20-1.

The bispecific antibody to which the 1564 clone was bound showed higher CSF and brain penetration ability than the therapeutic antibody to which the anti-IGF1R antibody was not bound, and the efficacy was confirmed at both 10 and 30 mg/kg doses. The bispecific antibody showed the brain-penetrating ability up to about 4.5 times higher than the monospecific antibody at 30 mg/kg dose.

Clone 1564 were prepared in bivalent form and monovalent form according to Examples 9-2 and 9-4, and then administered at 30 mg/kg or 60 mg/kg in the same manner as described above, and the amounts of antibodies in CSF and brain were analyzed after 24 hours. The two types of bispecific antibodies bound to 1564 clone showed higher CSF and brain penetration ability than monospecific antibodies. In particular, the bivalent form showed a higher BBB penetration ability than the monovalent form, which was increased brain-penetrating ability up to 5-fold.

The results of FIG. 7b and FIG. 7c show that 1564 clone improves the BBB-penetrating ability of the therapeutic antibody in the body even when bound to the therapeutic antibody in various forms.

The affinity variants of 1564 clone prepared according to Example 2 were expected to increase PK in a serum compared to the parental clone. Therefore, it was expected that the BBB-penetrating ability would be improved by remaining in the serum for a long time and continuously maintaining the BBB influx. After the affinity variants produced in bivalent form according to Example 9-2 or monovalent form according to Example 9-4 was administered intravenously to SD rats at 30 mg/kg, the blood was collected from the eye vein gun at 0, 24 and 48 hours. The tested antibodies were divided into two experiments according to the backbone of the therapeutic antibody. The bispecific antibodies of the corresponding variants used in the experiment are shown in the following Table.

**[Table 23]**

| Bispecific antibodies used for in vivo BBB penetration analysis | |
|---|---|
| Chimeric backbone clones | Humanized Backbone clones |
| Ch11F11-1564 bivalent | Hu11F11(ver.2)-1564 bivalent |
| Ch11F11-1564 monovalent | Hu11F11(ver.2)-VH5 bivalent |
| Ch11F11-C04 monovalent | Hu11F11(ver.2)-VH16 bivalent |
| Ch11F11-F06 bivalent | Hu11F11(ver.2)-VH35 bivalent |
| Ch11F11-F06 monovalent | Hu11F11(ver.2)-VH9 bivalent |
| ** | Hu11F11(ver.2)-VH2 bivalent |
| ** | Hu11F11(ver.2)-VH7 bivalent |
| ** | Hu11F11(ver.2)-VH32 bivalent |

The blood levels of antibodies were analyzed by ELISA. After the goat anti-human Fc antibody was coated on a 96-well plate, an appropriate amount of the diluted sample was treated and then detected with an antibody conjugated with an anti-human Fab HRP. The analysis results are shown in Fig. 7d and Fig. 7e.

As a result, in the first test group, the monovalent form of 1564, the monovalent form of F06, and the monovalent from of C04 showed longer serum PK than bivalent of the parental 1564 clone. In the second test group, the bivalent forms of VH2, VH5, VH7, VH9, VH16, and VH32 except for the VH35 showed an increased serum PK compared to the parental 1564 bivalent.

In order to analyze the BBB-penetrating ability of the groups, CSF was extracted from the rats at 48 hours and analyzed by the same ELISA method. The analysis results are shown in Fig. 7f.

In the first test group, 1564 monovalent, F06 monovalent, and C04 monovalent forms showing an increased serum PK showed increased CSF antibody compared to parental 1564 bivalent. In the second test group, the bivalents of VH2, VH5, VH7, VH9, VH16, and VH32, which also showed an increased serum PK, showed an increased CSF antibody compared to parental 1564 bivalent. VH35 showed shorter serum PK and low CSF antibody level compared to parental 1564 bivalent.

The results of Fig. 7d, Fig. 7e and Fig. 7f showed that serum PK was an important factor in the BBB-penetrating ability of the antibody due to the continuous BBB influx of the antibody, and that the BBB-penetrating abilities of bispecific antibodies having BBB shuttle and serum PK increased. In particular, in the case of F06 monovalent form with the highest CSF antibody level, it showed about 5-fold higher CSF-penetrating ability than the parental 1564 bivalent. In Examples 18-2 and 18-3, since 1564 bivalent antibody showed about 3-fold higher CSF-penetrating ability than the monospecific antibody in CSF, it was expected that F06 monovalent form would show a up to about 15-fold higher BBB-penetrating ability than the monospecific antibody.

### Example 16. Epitope mapping of anti-IGF1R antibodies

### 16-1. ELISA analysis of anti-IGF1R antibody, boiled IGFIR protein and native IGF1R protein

This example attempted to confirm whether the anti-IGF1R antibody recognizes a linear epitope or conformational epitope. ELISA was performed with the bivalent bispecific antibodies of 1564, 48G5, 54H4, 60H6, and B11 and ECD protein of native human IGF1R or a heated protein (boiled IGF1R). The ELISA method was performed as the same as that shown in Example 11. The analysis results are shown in the following table.

**[Table 24]**

| Clone ID | EC50(nM) for native IGF1R | EC50(nM) for boiled IFG1R |
|---|---|---|
| ch11F11-1564 | 0.914 | N/A* |
| ch11F11-48G5 | 1.21 | N/A |
| ch11F11-54H4 | 2.88 | N/A |
| ch11F11-60H6 | 10 | N/A |
| ch11F11-B11 | 7.13 | 410 |

| | | |
|---|---|---|
| *N/A: Not available | | |

The clones showed similar binding to ECD protein of native human IGF 1R as in Example 15, but did not bind to ECD protein of boiled human IGF1 of which the tertiary structure was destroyed by applying heat. This means that the anti-IGF1R antibody of the present invention binds to a conformational epitope, but not a linear epitope.

### 16-2. Epitope mapping of anti-IGF1R antibody

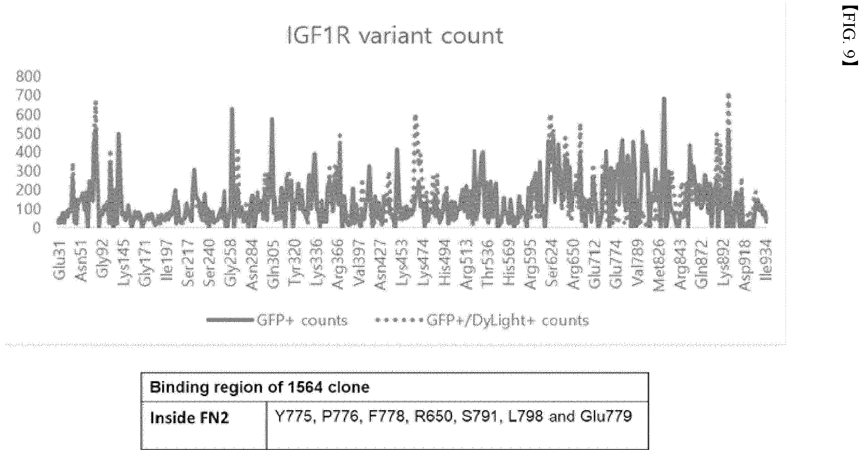

To analyze the conformational epitope of 1564 clone, alanine scanning was performed as follows. The OGFAR3 cell, an ovarian cancer cell line confirmed to have low IGF1R expression, was made to express the IGF1R library in which an eGFP tag was fused at N-terminus and the C-terminal kinase domain was removed. The IGF1R library contains the mutations in which the residues on the IGF1R surface are substituted with alanine. The prepared library was transfected into OVCAR3 cells. The cells identified with IGF1R expression were treated with 1564 antibody, and then labeled fluorescently by being treated with a secondary antibody labeled with DyLight650. The labeled cells were classified according to the presence or absence of IGF1R expression, the expression of IGF1R, and the presence or absence of 1564 binding, and the RNA deep sequencing was performed by using the Illumina HiSeq technique to analyze the frequency of each alanine mutation in the corresponding cell group. The corresponding frequency was normalized as a result for cells expressing wild-type IGF1R, and then the relative frequency was calculated to select the mutations whose number decreased in the 1564-labeled cell group. Based on this observation, it was found that the epitope of 1564 clone was located in the FN2 domain, and the residues belonging to it were Y775, P776, F778, R650, S791, and L798. The results and the sequences recognized by the 1564 clone are shown in FIG. 9. Since these residues are not known to involve in the binding of IGF1 according to the prior literature, the results faithfully describe the properties of 1564 clone in Example 16-1.

### Example 17. Comparison of antigen binding affinities of monospecific antibody and bispecific antibody

### 17-1: Binding affinities of monospecific antibody and bispecific antibody to alpha-synuclein antigen

When the scFv form of IGF1R antibody was linked to the alpha-synuclein antibody in IgG type, the effect on the binding affinity of the alpha-synuclein antibody was analyzed.

The alpha-synuclein aggregates were coated on a 96-well plate at a concentration of 1 ug/ml for 18 hours, and after washing, was treated with each antibody by diluting by 5 times from 400 nM. The bound antibodies were bound to anti-human Fc-HRP and then performed by color development with TMB solution, to measure the degree of binding of the antibody.

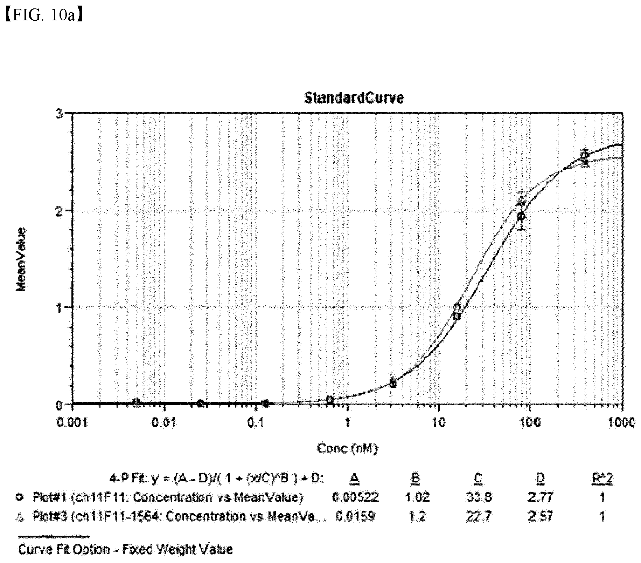

As shown in Fig. 10a, it was confirmed that the binding affinity to alpha-synuclein aggregates were the same in the monospecific antibody and the bispecific antibody.

### 17-2: Binding affinities of monospecific antibody and bispecific antibody to IGF1R antigen

To compare the binding degrees of the single alpha-synuclein antibody and the bispecific antibody to the IGF1R antigen, the experiment was performed in the same manner as in Example 22.

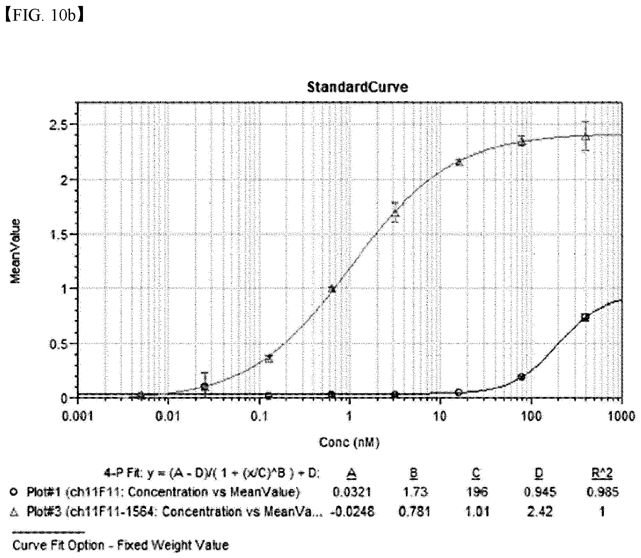

As shown in FIG. 10b, it was confirmed that the bispecific antibody having the scFv form of IGF1R antibody bound well in a concentration-dependent manner, but the monospecific antibody having no IGF1R antibody region did not bind.

### 17-3: Analysis of binding ability of a humanized alpha-synuclein antibody

The difference in binding affinity between the bispecific chimeric antibody and the bispecific humanized antibody was analyzed by performing the experiment in the same manner as in Example 17-1.

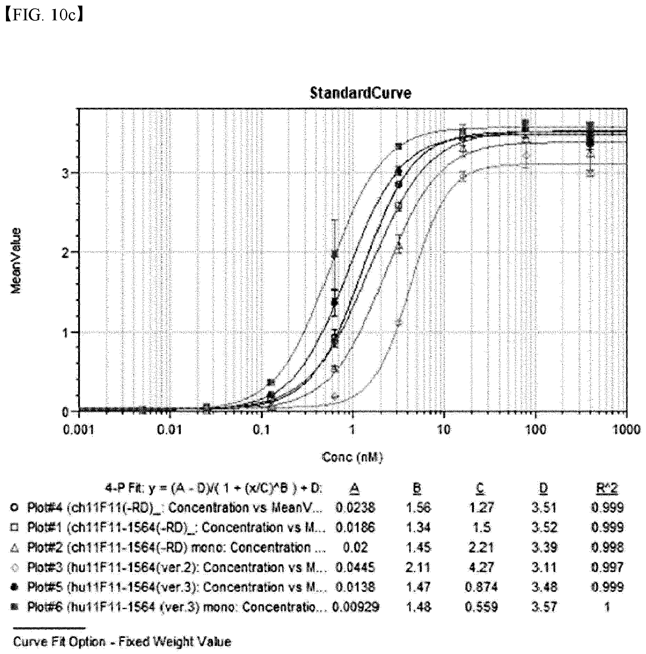

As shown in FIG. 10c, the bispecific humanized antibodies had binding affinities to alpha-synuclein aggregates at a level similar to those of bispecific chimeric antibody, and it was confirmed that monovalent bispecific antibody with one scFv of IGF1R also exhibited the binding affinities at a level similar to chimeric antibodies.

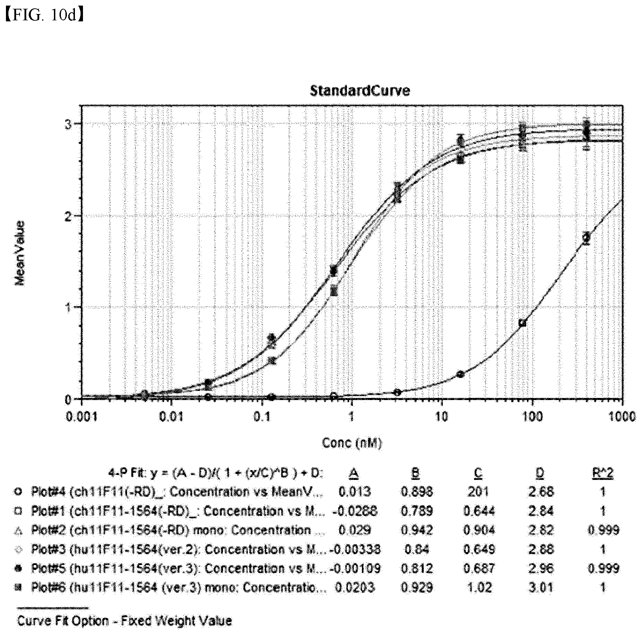

As a result of analyzing the binding affinities to IGF1R between the bispecific chimeric antibody and the bispecific humanized antibody by performing the experiment in the same manner as in Example17-2, all bispecific antibodies represented the same binding affinity, but the monospecific antibody having no IGF1R scFv did not bind, as shown in FIG. 10d.

These results suggest that it has the same activity and no change in the binding affinity to alpha-synuclein aggregates and IGF1R, when it is humanized to replace the mouse antibody region acting as an immunogen in the human body.

### 17-4: Comparison of phagocytosis of the monospecific antibody and the bispecific antibody

Phagocytosis refers to the action of removing extracellular substances by involving in various receptors of macrophages. Various protein aggregates induce an immune response or an inflammatory reaction, which adversely affects the human body. Particularly, it is known that it is promoted through the interaction between the Fc region of the antibody and FcrR on the cell surface, when the antibody is administered to remove the alpha-synuclein aggregates. For this reason, the activity against phagocytosis of a monospecific antibody and a bispecific antibody liked with IGF1R scFv was compared.

BV-2 microglial cells derived from mouse were used to compare phagocytosis between the monospecific antibody and the bispecific antibody. BV-2 cells were cultured in RPMI1640 medium, prepared at a concentration of 2 × 10⁶ cells/ml, and dispensed at 100 uL in U-bottom 96 well plates. 10 ug/ml of alpha-synuclein aggregates and 25 ug/ml of antibodies were diluted with RPMI1640 medium, mixed, and left at room temperature for 20 minutes. The mixture of alpha-synuclein aggregates and antibodies were treated with BV-2 cells and left for 15 minutes. The alpha-synuclein aggregates in the supernatant were removed by centrifugation at 1200 rpm, and washed three times with PBS buffer (pH2.5) to remove aggregates or antibodies bound to the cell surface. The cells were fixed with 4% paraformaldehyde and washed with PBS buffer. To confirm the phagocytosis of aggregates and antibodies into the cells, 0.5% triton X-100 was added to loosen the cell membrane, washed with PBS buffer, and treated with pan-alpha-synuclein antibody for 1 hour. The bound pan-alpha-synuclein antibody was treated with an anti-rabbit-alexa-488 antibody for 1 hour, and then FACS analysis confirmed the aggregates entering into the cell by macrophage.

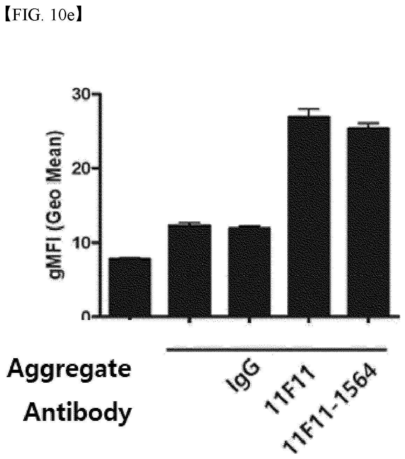

As shown in FIG. 10e, it was confirmed that the normal human IgG did not affect macrophage and the phagocytosis of alpha-synuclein aggregates was increased, when treating with the alpha-synuclein antibody. When the monospecific antibody and the bispecific antibody were compared, it was confirmed that the phagocytosis occurred at a similar level, and that the scFv form of IGF1R antibody bound to C-terminus of the IgG did not affect the action of the alpha-synuclein antibody.

### Example 18. Evaluation of efficacy of bispecific antibody

According to Example 10, the bivalent bispecific antibody comprised of chimeric 11F11 antibody and scFv of 1564 clone was prepared, and the bispecific antibody and the single alpha-synuclein antibody were tested for in vivo efficacy in a transgenic mouse (mThy-1 human α-synuclein, UC San Diego) overexpressing human alpha-synuclein. 2.5 mg/kg of the monospecific antibody or human IgG, or the same mole of the bivalent bispecific antibodies were administered intraperitoneally weekly for 3 months. Five mice per a group were used, and non-transgenic littermate was used as a control. Subsequently, perfusion was performed as follows.

After the last administration was completed, the animals were anesthetized with chloral hydrate under humanitarian regulations and then perfused with 0.9% physiological saline, for the analysis of pathology in the brain. Subsequently, one half (sagittal section) of perfused brain was stored in 4% paraformaldehyde (pH7.4, 4 °C) in phosphate buffer until the analysis time, and the other half was immediately frozen (-70 °C).

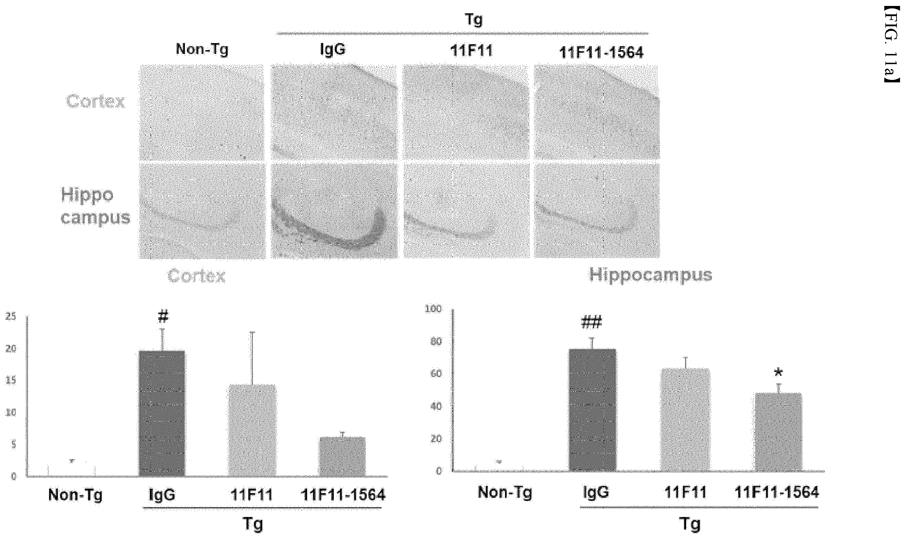

The pathological analysis was conducted as follows. The half brain fixed to paraformaldehyde was cut into continuous sections at 40 µm thickness by free-floating using a vibrometer. To confirm the expression level of alpha-synuclein in the brain of each administration group, the sections containing cortex, hippocampus and striatum were incubated with alpha-synuclein antibodies (p129 α-syn antibody of aggregate marker, abcam, ab59264, or whole alpha-synuclein antibodies) at 4 °C overnight. Alternatively, in order to analyze the activity degree of astrocytes, the sections were analyzed for GFAP (glial fibrillary acidic protein) (AB5804, millipore) or in order to analyze the neuro-inflammation degree, the sections were incubated with an antibody to IL-1β (ab9722, abcam), respectively. Alternatively, an antibody against NeuN (Chemicon, # MAB377) was treated to analyze the degree of neuronal cell death in hippocampus. After incubation with the primary antibody, the biotin-linked goat anti-rabbit IgG (1: 100, Vector Laboratories) and Avidin D-horseradish peroxidase (1: 200, ABC Elite, Vector Laboratories) were treated and detected with diaminobenzidine (DAB). Each immune-stained section was observed with a bright field microscope to measure optical density. The results are disclosed in FIGS. 11a to 11e.

### 18-1. Analysis of alpha-synuclein reduction ability by a chimeric antibody and a bispecific antibody

FIG. 11a shows the result of staining and measuring cortex and hippocampus among the mouse brain tissue by using p-129 α-Syn antibody after administering the antibodies to mouse for testing whether the chimeric 11F11 antibody and the bivalent bispecific antibody comprised of 1564 clone and the chimeric 11F11 antibody can remove alpha-synuclein aggregates in a mouse animal model (TG) overexpressing human alpha-synuclein. p-129 α-syn is phosphorylated form at 129^{th} residue and a maker of aggregates, and is represented as dark brown spots or aggregates in stained tissue.

According to FIG. 11a, the IgG-treated group showed a higher staining degree of p-129 α-syn than the non-tg control group (#: one way ANOVA, p \u003c0.01). On the contrary, in the group treated with the monospecific antibodies or the bispecific antibodies, the staining degree of p-129 α-syn or aggregates was significantly reduced. In particular, in hippocampus, the degree of reduction in the bispecific antibody treatment group was higher than that of the chimeric 11F11 antibody (^{∗}: one way ANOVA, p \u003c0.05). FIG. 11b shows the experiment result in the same manner as in FIG. 11a, except for the staining with a whole alpha-synuclein antibody as a marker. The detection of all alpha-synuclein indicates that the antibody of the present invention has the ability to clear the alpha-synuclein itself and inhibit the cell-to-cell transmission. In other aspects, it can also be interpreted as inhibiting the formation of aggregates from monomers or removing all monomers. The increased human alpha-synuclein in TG mouse is reduced compared to the IgG administration group by administration of the monospecific antibodies and the bispecific antibodies. In particular, in hippocampus, the bispecific antibodies were more effective than the monospecific antibodies.

The results indicate that the chimeric 11F11 antibody and the bispecific antibody effectively reduce alpha-synuclein and its aggregate levels in Parkinson's disease animal models even at a low dose of 2.5 mg/kg. In particular, the bispecific antibody is superior to the monospecific antibody, which suggests that the bispecific antibody can reach the brain more than the monospecific antibody and treat the disease effectively based on the improved BBB-penetrating ability.

### 18-2. Analysis of astroglosis and inflammatory cytokine level reduction ability of the chimeric antibody and the specific antibody

Glyosis is a non-specific reaction that occurs in glial cells in response to damage to the central nervous system and is triggered by BBB damage, or the substances such as TGF-beta and interleukin. Representatively, it includes astrogliosis and GFAP protein is used as a marker. Thus, the effect of reducing the astrocytosis and inflammatory cytokine release triggering the astrocytosis were analyzed by administering the chimeric 11F11 antibody and the bispecific antibody comprised of 1564 clone and the chimeric antibody. The results of the analysis are disclosed in FIG. 21c and FIG. 21d.

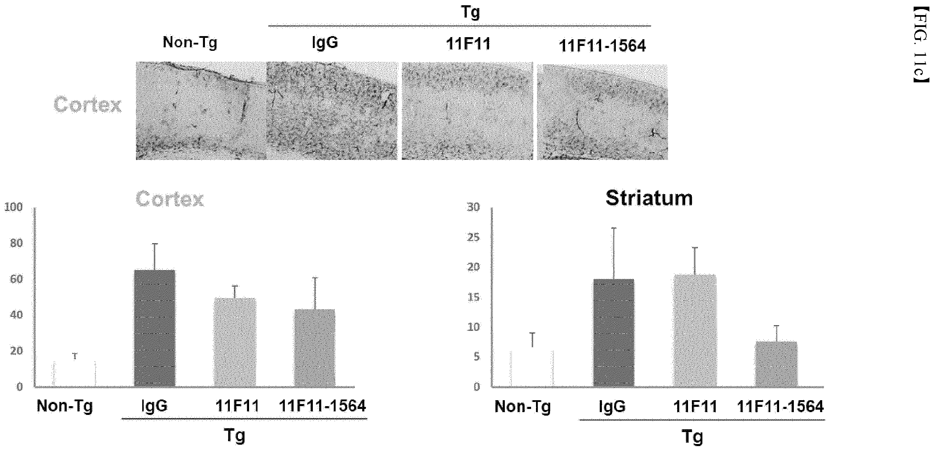

FIG. 11c shows a result of staining and measuring the mouse brain tissue using GFAP (astrogliosis) as a marker after administering the antibodies, to test whether the chimeric 11F 11 antibody and the bispecific antibody comprised of 1564 clone and the chimeric antibody prepared in an example of the present invention can reduce astrogliosis *in vivo.* The monospecific antibody and the bispecific antibody inhibited astrogliosis compared to the IgG control group. In particular, it was confirmed that the efficacy of the bispecific antibody was superior to that of the monospecific antibody in striatum.

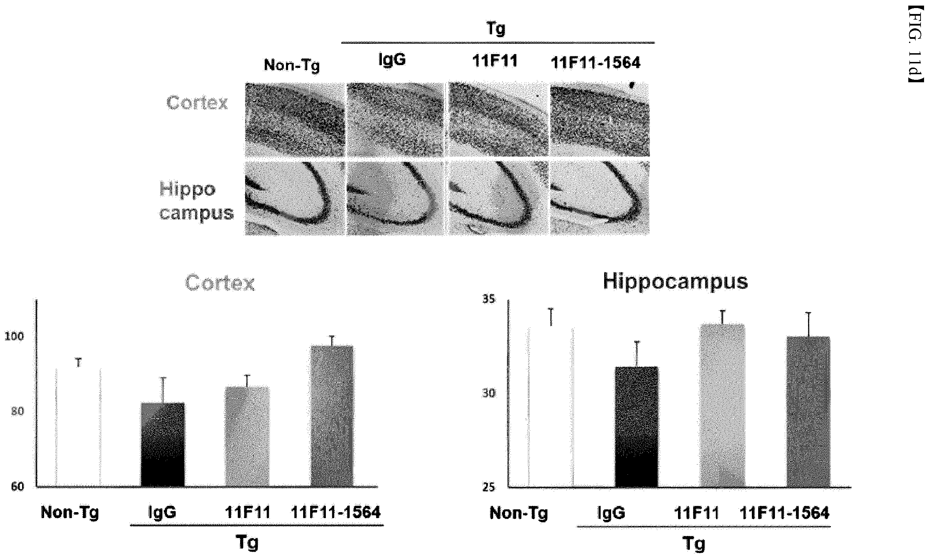

FIG. 11d shows a result of staining and measuring the mouse brain tissue using IL-1 beta antibody as a marker after administering the antibodies, to test whether the chimeric 11F11 antibody and the bispecific antibody comprised of 1564 clone and the chimeric antibody prepared in an example of the present invention can reduce inflammatory cytokines in vivo. IL-1 beta causes an inflammation, leading to the death and inflammatory response of various neurons. In the hippocampus of rats administered by the antibodies according to the present invention, IL-1 beta was reduced in the groups administered by monospecific antibodies and the bispecific antibodies- compared to the IgG control group, and in particular, the reduction ability of the bispecific antibody was significantly superior to that of the monospecific antibody (##: One- way ANOVA, p \u003c0.005; *: one way ANOVA, p \u003c0.05).

As shown in the figures, the antibody according to the present invention has been shown to reduce the astrogliosis and decrease the release of inflammatory cytokine of IL-1beta, which triggers the astrogliosis, compared to the control.

### 18-3. Analysis of neurodegeneration reduction ability of the chimeric antibody and the bispecific antibody

It has been confirmed in the prior literature that the death of brain cells occurs due to the neurotoxicity and the inflammatory response of alpha-synuclein. Whether the monospecific antibodies and the bispecific antibodies of the present invention can inhibit brain cell death caused by alpha-synuclein *in vivo* was analyzed.

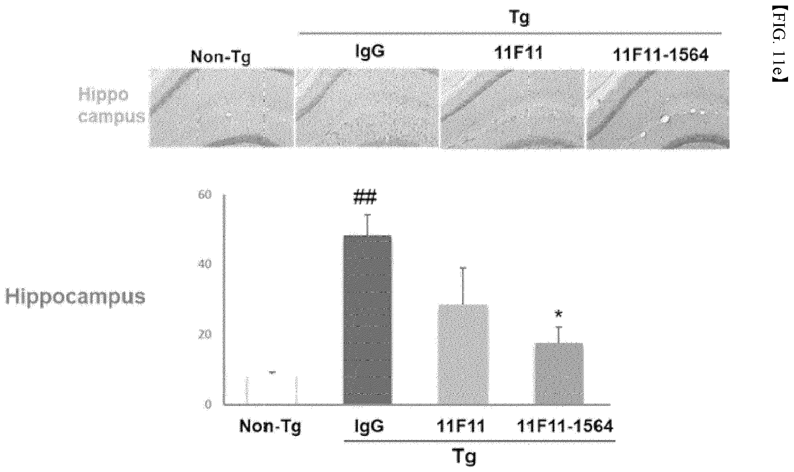

As a result of staining with NeuN which was a marker of neurons in cortex and hippocampus, it was found that both the monospecific antibody and the bispecific antibody reduced the degree of brain cell death compared to the IgG control group. Particularly, in cortex, it was confirmed that the bispecific antibody had superior inhibition ability of brain cell death compared to the monospecific antibody. The results are shown in Fig. 11e.

### Example 19. Increased half-life by Fc engineering and improved BBB-penetrating ability due to the increased half-life

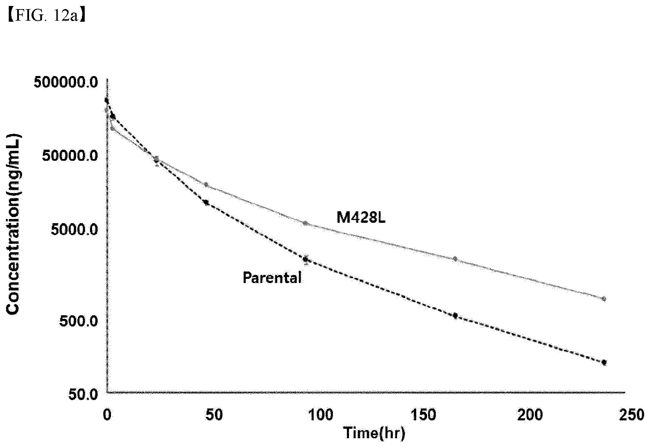

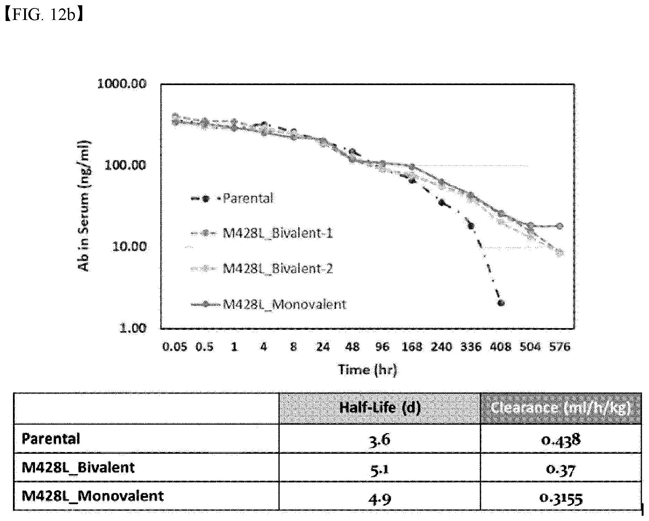

FcRn is an important receptor on the cell membrane that increases the half-life by drawing and circulating the antibody into cells, so as to inhibit the degradation of antibody when the antibody circulates in blood vessels. The BBB-penetrating ability is also important for the transcytosis activity of antibody, but it is well known that the transcytosis activity of antibody is important in the BBB-penetrating ability, but the antibodies pass through BBB depending on the concentration of antibodies in blood vessels. For this reason, in order to increase the half-life of the bispecific antibody, the bispecific antibodies were prepared by increasing the binding affinity to FcRn by changing methionine (Met) to leucine (Leu) at 428th amino acid in the Fc region. As a result of comparing the half-life by the administration of WT bispecific antibody and M428L bispecific antibody at a concentration of 10 mg/kg to tg mouse expressing Human FcRn, the increased half-life effect was confirmed to be about 50%, as shown in FIG. 11. To confirm the half-life increase again, the PK profiles were analyzed by administering WT bispecific antibody, M428L bivalent bispecific antibody, and M428L monovalent bispecific antibody to monkeys. In the case of the WT bispecific antibody, as shown in FIG. 22a, the blood concentration rapidly decreased after 168 hours, while the M428L bispecific antibodies with high binding affinity to FcRn maintained an improved blood concentration compared to WT. It was confirmed that the half-life increased by about 1.5 days in the M428L bispecific antibody compared to the WT bispecific antibody. In particular, in terms of clearance, the M428L monovalent bispecific antibody was the best clearance and the WT bispecific antibody showed the fastest clearance (Fig. 12b).

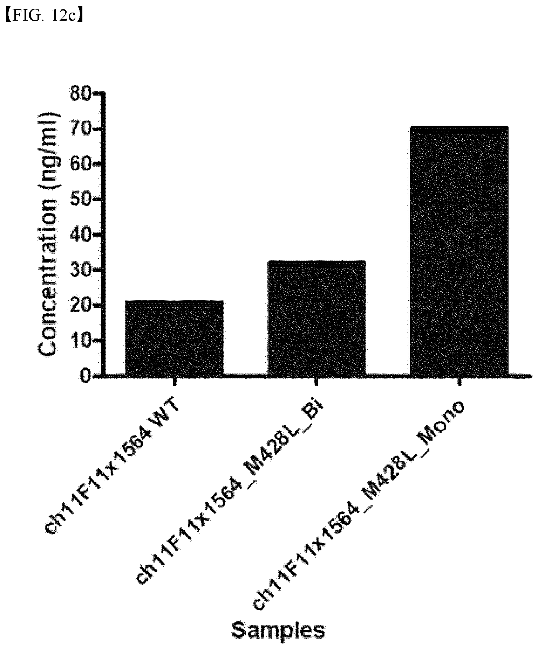

To verify the improved BBB passage due to the increased half-life effect, CSF was extracted at 24 hours after the antibody administration, and the amount of antibody in CSF was analyzed. After coating 100 ng/ml of IGF1R in a refrigerated state for 18 hours, CSF was added to detect the antibody bound to IGF1R. As can be seen in FIG. 12c, it was confirmed that the amount of the M428L bispecific antibody to pass BBB, which had a large amount of antibodies in the blood, was large and the M428L monovalent bispecific antibody showed improved BBB-penetrating ability than the M428L bivalent bispecific antibody, as well as the excellent BBB penetrating ability.

### Example 20. Efficacy evaluation of deamidated affinity variant-based bispecific antibodies

Monovalent bispecific antibodies including hu11F11(ver.2) (humanized antibody of 11F11) and F06 scFv (affinity variant of 1564), especially bispecific antibodies including mutant having modified in some residues of CDR for remove deamidation (including hu11F11(ver.2)-F06(de2)(StoP) monovalent), were produced according to Example 10. The prepared bispecific antibody and the alpha-synuclein monospecific antibody were tested and compared for in vivo efficacy in a transgenic mouse overexpressing human alpha-synuclein (mThy-1 human α-synuclein, UC San Diego).

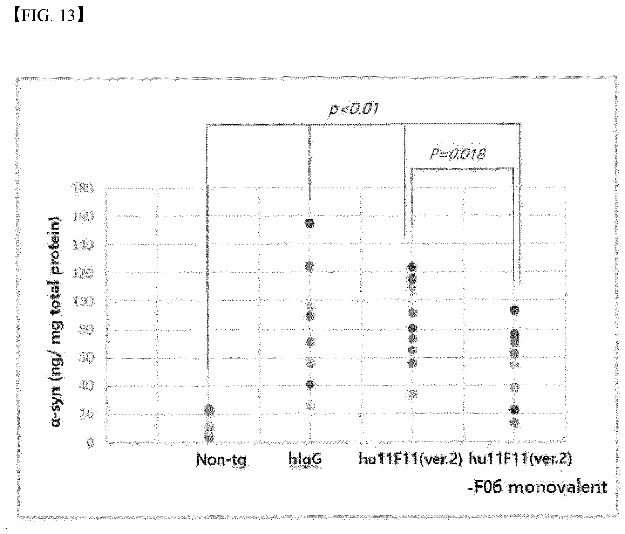

Specifically, in 4-month old transgenic mouse, 20 mg/kg of hu11F11 (ver.2) and 23.4 mg/kg of bispecific antibody equivalent to the same mole number were administered intraperitoneally at 0, 72, 144, or 192 hours for 8 days. In twenty-four hours after the last dose, the animals were anesthetized with chloral hydrate and cardiac perfused with 0.9% saline. Brains were isolated and snap frozen at -70 °C until analysis time. The brain tissue was ground and centrifuged to remove debris, and the supernatant was obtained. It was analyzed for α-syn with ELISA analysis to measure quantitively the amount of α-syn in the brain lysate (Invitrogen #KHB0061). The result is shown in FIG. 13.

As shown in FIG. 13 , the humanized antibody 11F11(ver.2) and the deamidated F06 variant-based bispecific antibody prepared in an example of the present invention decreased α-syn in the brain compared to the IgG control. In particular, the bispecific antibody showed a superior ability to reduce α-syn in the brain compared to the monospecific antibody of α-syn.

### Example 21. IGF1R-specific antigen binding affinity analysis (ELISA) for the bispecific antibody including (de2)(StoP)deamidated anti-IGF1R antibody

This example was performed to test whether the bispecific antibody including the deamidated anti-IGF1R antibody according to Example 8 has normal antigen binding affinity, and to analyze simultaneously the antigen-binding affinity of the deamidated anti-IGF1R antibody in various anti-IGF1R antibodies and the bispecific antibody formats in different ways. For this purpose, the monovalent bispecific antibody and the bivalent bispecific antibody including each of non-deamidated antibodies (wild type), (de)(StoP) deamidated antibodies, and (de2)(StoP) deamidated antibodies prepared based on the anti-IGF1R clones F06, VH5 and VH16 were produced. Their binding affinity and concentration-dependent binding to recombinant IGF1R were quantitatively analyzed and compared through sandwich ELISA. The hu11F11 (ver.2) clone was used as the anti-a-syn antibody.

Human recombinant IGF1R of an antigen for antibody binding was purchased from Sino biological as an extracellular domain (ECD) (10164-H08H). In a 96-well ELISA plate (Nunc-Immuno Plates, NUNC, Rochester, NY), human IGF1R was diluted to 1 ug/ml in PBS buffer and put in 100 ul per well, followed by reaction at 4 °C for 16 hour, and after coating, the supernatant was removed. 200 ul of PBS buffer containing 1% BSA (bovine serum albumin) was added per well and reacted at 37° C for 2 hours to block non-specific binding.

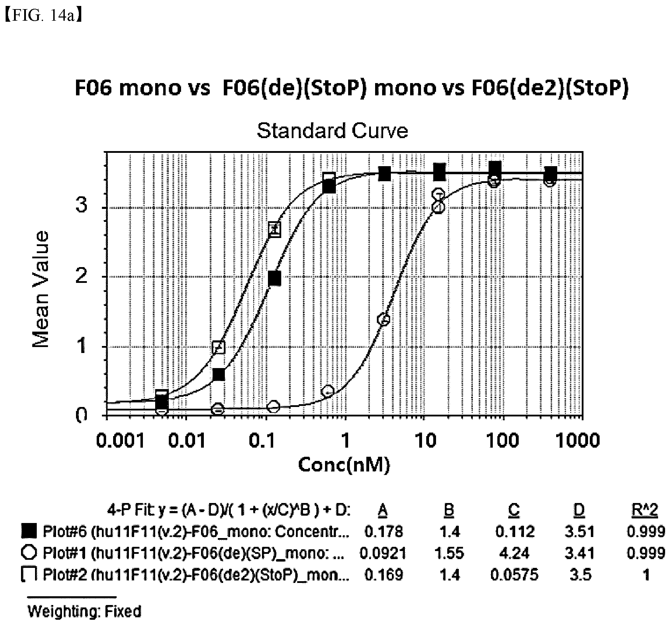

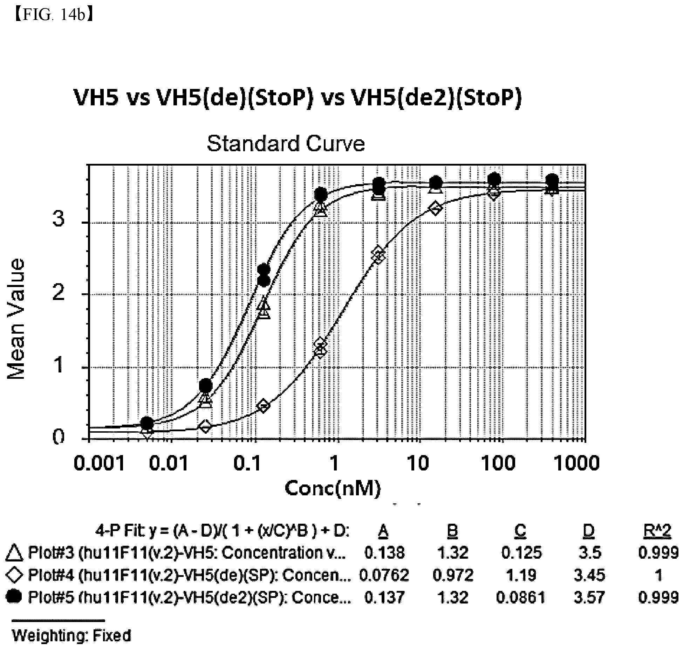

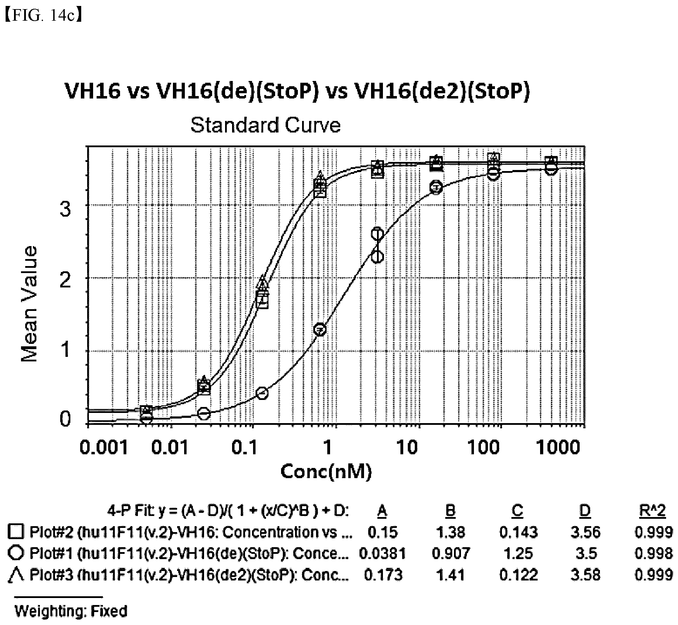

The prepared bispecific antibodies and each control antibody (wild type and (de)(StoP) deamindated antibody) were diluted with five times to a maximum concentration of 400 nM to produce eight (8) points. The diluted solutions were added each well at 100 µl, and reacted at 37 °C for 2 hours to binding the antibodies to the coated antigen. After completion of the reaction, washing was performed 4 times using 300 ul of PBS buffer containing 0.05% Tween20, and anti-human Fc-HRP recognizing human Fc present in the bispecific antibody was diluted 1:2000 in blocking buffer to each well. 100 ul of each was reacted for 1 hour at 37 °C. After washing 4 times using 300 ul of PBS-T (Tween20 0.05%) again, TMB (Tetramethylbenzidine, Sigma, T0440) was used to develop color. The enzymatic reaction was stopped with 0.5 mol/L sulfuric acid, and the absorbance was recorded and analyzed at 450 nm using a microplate reader (molecular device). The experimental results are shown in FIGs. 14a to 14c and Table 25. Fig. 14a is an experimental result for F06 monovalent antibody, F06(Stop) monovalent antibody and F06(de2)(Stop) antibody, Fig. 14b is an experimental result for VH5 antibody, VH5(de)(Stop) antibody and VH5(de2)(Stop) antibody, and Fig. 14c is an experimental result for VH16 antibody, VH16(de)(Stop) antibody and VH16(de2)(Stop) antibody.

According to Figs. 14a, 14b, and 14c, regardless of the anti-IGF1R clone type and monovalent / bivalent format, (de2)(StoP) deamidated antibody maintained the antigen binding ability to be level of the non-deamidated wild type, and had an excellent antigen-binding ability compared to (de)(StoP) deamidated antibody. Table 25 below shows the experimental results of the non-deamidated antibody (wild type), (de)(StoP) deamidated antibody and (de2)(StoP) deamidated antibody based on F06, VH5 and VH16.

**[Table 25]**

| Clones | (de)(StOP) | | (de2)(StoP) | |
|---|---|---|---|---|
| | EC50 (nM) | % WT | EC50 (nM) | % WT |
| F06 mono | 4.24 | 2.64 | 0.0575 | 195.00 |
| VH5 bi | 1.19 | 10.50 | 0.0561 | 222.81 |
| VH16 bi | 1.25 | 11.44 | 0.122 | 117.21 |

Table 25 show the comparison of the numerical values obtained from the sandwich ELISA results of (de)(StoP) deamidated antibody and (de2)(StoP) deamidated antibody. According to the result of Table 25, it was confirmed that regardless of the anti-IGF1R clone type and monovalent / bivalent format, (de2)(StoP) deamidated antibody had an excellent antigen-binding ability compared to (de)(StoP) deamidated antibody.

### Example 22. Cell surface IGF1R-specific antigen binding affinity analysis (FACS) for the bispecific antibody including (de2)(StoP)deamidated anti-IGF1R antibody

The cell surface IGF 1R-specific antigen binding affinity analysis was performed by FACS using (de2)(StoP) deamindated bispecific antibodies and their wild type antibodies and (de)(StoP) deamindated bispecific antibodies as controls used in Example 21. MCF7 cells overexpressing IGF1R were used in the analysis.

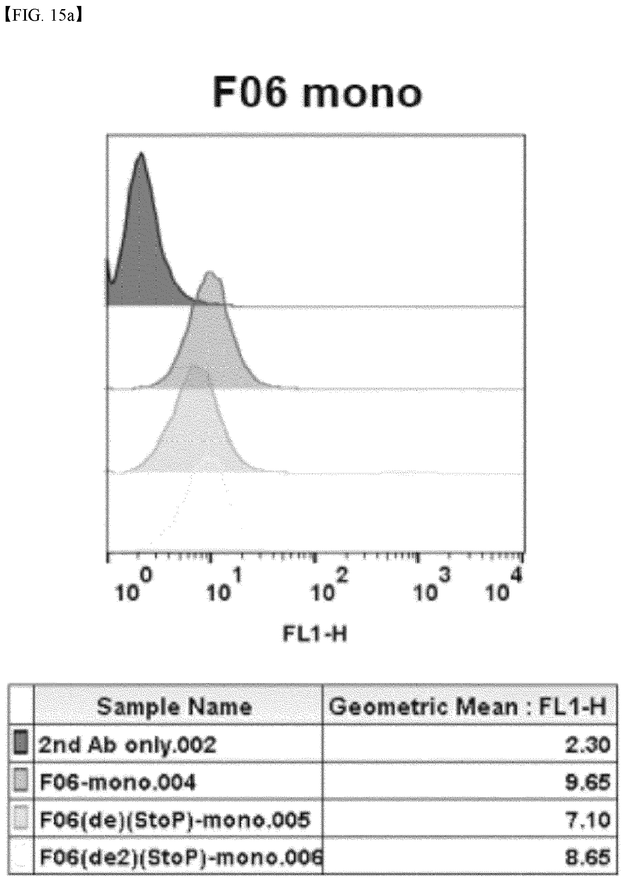

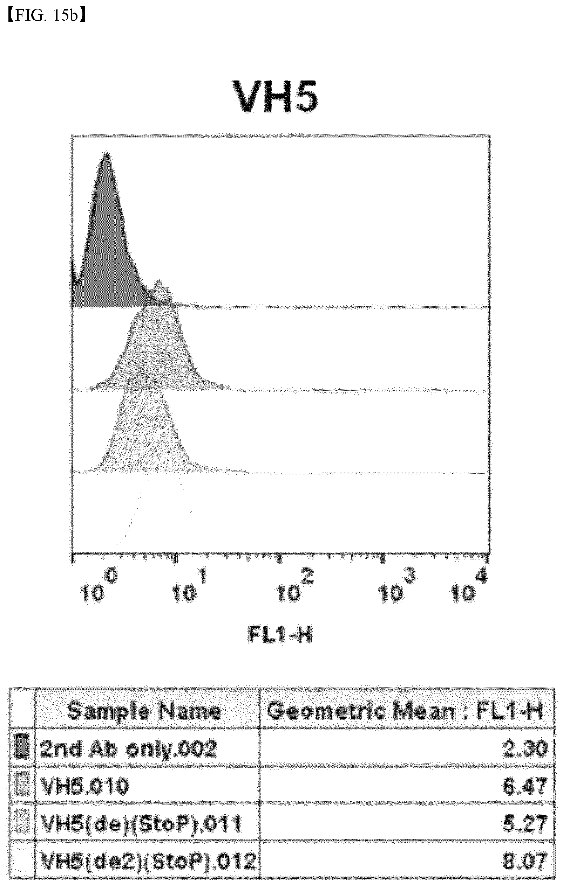

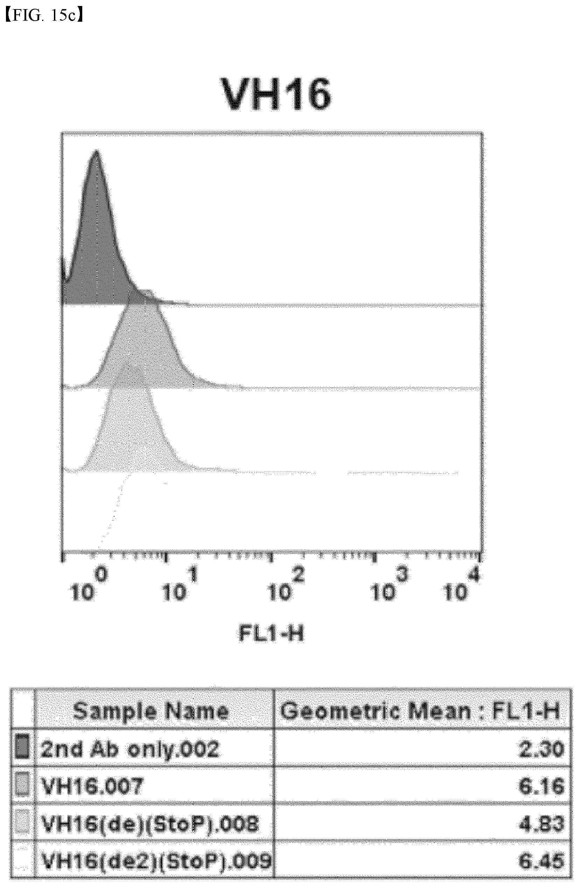

Specifically, each bispecific antibody was diluted to 10 ug/ml, added to MCF-7 cell lines at 0.5 × 10⁶ MCF-7 cell lines, and reacted at 4°C for 2 hours. After washing twice using PBS buffer, anti-human FITC was diluted 1:1000 and treated, and reacted at 4°C for 1 hour. After washing twice again with PBS buffer, the binding degree of anti-IGF1R BsAb was measured using a FACSCalibur instrument. As a control, MCF-7 cells treated with only the secondary antibody were used, and the experimental results are shown in FIGs. 15a to 15c.

As the experimental results, similar to the results confirmed in Example 21, the antigen binding affinity of (de2) (StoP) deamidated antibody was equivalent to that of non-deamidated wild type antibody, and showed superior antigen-binding ability compared to (de)(StoP) deamidated antibody, regardless of anti-IGF 1R clone type and monovalent / bivalent formats.

### Example 23. In vivo BBB penetration capacity analysis for the bispecific antibody based on (de2)(StoP) deamidated anti-IGF1R antibody

The in vivo BBB penetration ability of the bispecific antibody including deamidated anti-IGF1R antibody according to Example 8 was confirmed in SD (Sprague-Dawley) rats. Experimental groups and doses are summarized in the table below.

**[Table 26]**

| Clone ID | Dose |
|---|---|
| hu11F11(ver.2) | 30 mg/kg |
| hu11F11(ver2)-1564 monovalent | 35.46 mg/kg |
| hu11F11(ver2)-F06 monovalent(de)(StoP) | 35.46 mg/kg |
| hu11F11(ver2)-F06 monovalent(de2)(StoP) | 35.46 mg/kg |

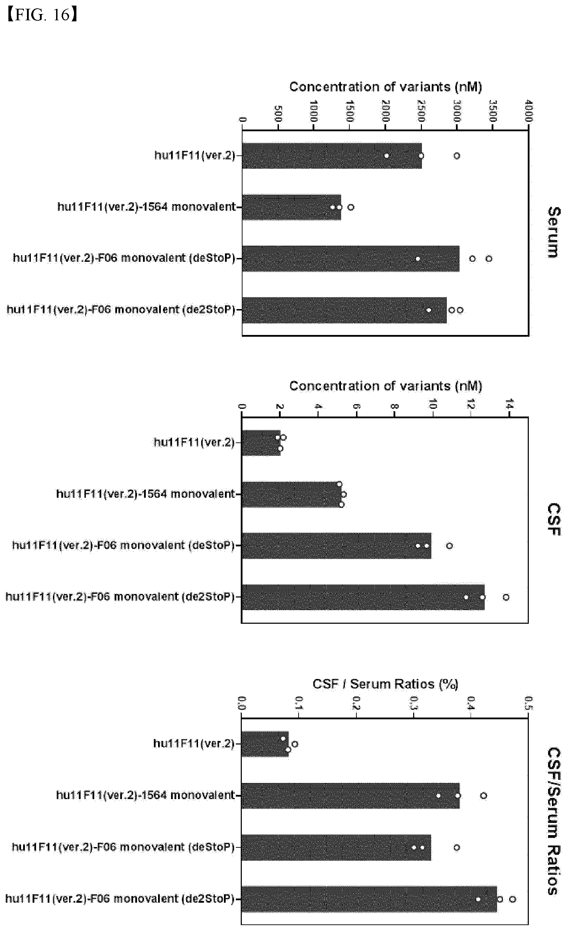

As described in Table 26, the amount of antibodies in serum and cerebrospinal fluid (CSF) in 24 hours after a single administration of α-syn monospecific antibody or α-syn/IGF1R bispecific antibody into the caudal vein of rats, were analyzed with the mass spectrometry analysis. The specific mass spectrometry method was performed substantially in the same manner as in Example 15, and the analysis result is shown in FIG. 16.

In FIG. 16, the serum concentration representing the blood concentration of antibody at 24 hours after administration, and was similarly observed in (de)(StoP) deamidated antibody and (de2)(StoP) deamidated antibody. On the other hand, the concentration in the cerebrospinal fluid representing the brain delivery of the antibody was highest in the (de2)(StoP) deamidated antibody. This shows that the bispecific antibody of the present invention exhibits excellent BBB penetrating ability by the deamidated anti-IGF1R antibody.

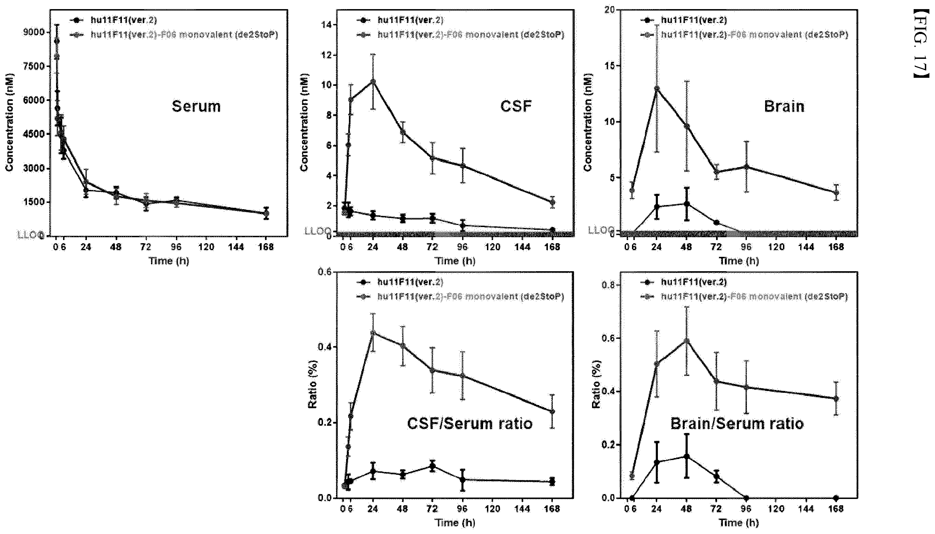

In order to observe the efficacy of the bispecific antibody of the present invention for a longer period of time, after the anti-alpha-synuclein monospecific antibody of hu11F11 (ver.2) and hu11F11(ver2)-F06 monovalent(de2)(StoP), which was (de2)(StoP) deamidated bispecific were administrated once into the caudal vein of rats, the amounts of antibodies in serum, cerebrospinal fluid and brain were analyzed up to 168 hours after administration with with the mass spectrometry analysis. The specific mass spectrometry method was performed substantially in the same manner as in Example 15, and the analysis result is shown in FIG. 17.

According to FIG. 17, the serum concentration of the antibody was similarly observed for the anti-alpha-synuclein monospecific antibody and the (de2)(StoP) deamidated bispecific antibody. However, in case of the concentration in the cerebrospinal fluid representing the brain delivery of the antibody, compared to the monospecific antibody, the area under the curve (AUC) of the (de2)(StoP) deamidated bispecific antibody increased about 5.8-fold and the concentration in the cerebrospinal fluid at 24 hours was about 10-fold or more in the (de2)(StoP) deamidated bispecific antibody. In addition, the brain concentration also increased about 7.9-fold in the area under the curve (AUC) in the (de2)(StoP) deamidated bispecific antibody, compared to the monospecific antibody. From this result, it was confirmed that the bispecific antibody of the present invention exhibited excellent BBB penetrating ability by including the (de2)(StoP) deamidated IGR1R antibody.

### [The invention also pertains to the following]

### [Item 1]

An anti-α-Syn/anti-IGF1R bispecific antibody, comprising an anti-α-Syn antibody or an antigen binding fragment thereof; and anti-IGF1R antibody or an antigen binding fragment thereof, wherein the anti-IGF1R antibody comprises a heavy chain variable region and a light chain variable region,
the heavy chain variable region comprises a heavy chain CDR1 (H-CDR1) comprising an amino acid sequence elected from the amino acid sequences of SEQ ID NO: 1 or SEQ ID NO: 10, a heavy chain CDR2 (H-CDR2) comprising an amino acid sequence selected from the amino acid sequences of SEQ ID NOs: 2 to 7 and SEQ ID NOs: 11 to 18, and a heavy chain CDR3 (H-CDR3) comprising an amino acid sequence selected from the amino acid sequences of SEQ ID NOs: 8 to 9 and SEQ ID NO: 19, and
the light chain variable region comprises a light chain CDR1 (L-CDR1) comprising an amino acid sequence selected from the amino acid sequences of SEQ ID NO: 20, a light chain CDR2 (L-CDR2) comprising an amino acid sequence selected from the amino acid sequences of SEQ ID NOs: 21 to 23, and a light chain CDR3 (L-CDR3) comprising an amino acid sequence selected from the amino acid sequences of SEQ ID NOs: 24 to 28 and SEQ ID NOs: 29 to 31.

### [Item 2]

The bispecific antibody according to Item 1, wherein the anti-α-Syn antibody or antigen binding fragment thereof specifically binds to C-terminal region of human, monkey, rat and mouse alpha-synuclein.

### [Item 3]

The bispecific antibody according to Item 1, wherein the anti-α-Syn antibody or antigen binding fragment thereof specifically binds to a peptide comprising at least consecutive eleven amino acids of 110 to 120 residues or a peptide comprising at least consecutive twelve amino acids of 111 to 122 residues from N-terminus in an amino acid sequence of SEQ ID NO: 173.

### [Item 4]

The bispecific antibody according to Item 1, wherein the anti-IGF1R antibody or antigen binding fragment thereof specifically recognizes and binds to at least an amino acid selected from the group consisting of Y775, P776, F778, R650, S791, L798, Glu779, L641, H808, E809, L813, V397, D435, W434, Y460 and C488 in a protein comprising an amino acid sequence of SEQ ID NO: 174.

### [Item 5]

The bispecific antibody according to Item 4, wherein the anti-IGF1R antibody or antigen binding fragment thereof recognizes and binds to at least a binding site selected from the group consisting of binding site 1 to binding site 3 in a protein comprising an amino acid sequence of SEQ ID NO: 174, and wherein the binding site 1 comprises at least one selected from the group consisting of Y775, P776, F778, R650, S791, L798 and Glu779, the binding site 2 comprises at least one selected from the group consisting of L641, H808, E809 and L813, and the binding site 3 comprises at least one selected from the group consisting of V397, D435, W434, Y460 and C488.

### [Item 6]

The bispecific antibody according to Item 1, wherein the anti-IGF1R antibody or antigen binding fragment thereof comprises a heavy chain variable region and a light chain variable region,
the heavy chain variable region comprises a heavy chain CDR1 (H-CDR1) comprising an amino acid sequence elected from the amino acid sequences of SEQ ID NO: 1, a heavy chain CDR2 (H-CDR2) comprising an amino acid sequence selected from the amino acid sequences of SEQ ID NO: 3, and SEQ ID NOs: 5 to 7, and a heavy chain CDR3 (H-CDR3) comprising an amino acid sequence selected from the amino acid sequences of SEQ ID NOs: 8 to 9, and
the light chain variable region comprises a light chain CDR1 (L-CDR1) comprising an amino acid sequence selected from the amino acid sequences of SEQ ID NO: 20, a light chain CDR2 (L-CDR2) comprising an amino acid sequence selected from the amino acid sequences of SEQ ID NOs: 22 and 23, and a light chain CDR3 (L-CDR3) comprising an amino acid sequence selected from the amino acid sequences of SEQ ID NOs: 26 to 28.

### [Item 7]

The bispecific antibody according to Item 1, wherein the heavy chain variable region of the anti-IGF1R antibody or antigen binding fragment thereof comprises,
a heavy chain variable region framework1 (H-FR1) comprising an amino acid sequence of SEQ ID NO: 32,
a heavy chain variable region framework 2(H-FR2) comprising an amino acid sequence of SEQ ID NO: 33 or SEQ ID NO: 34,
a heavy chain variable region framework 3 (H-FR3) comprising an amino acid sequence of SEQ ID NO: 35, and
a heavy chain variable region framework 4 (H-FR4) comprising an amino acid sequence of SEQ ID NO: 36, and
the light chain variable region comprises a light chain variable region framework1 (L-FR1) comprising
an amino acid sequence selected from SEQ ID NO: 37,
a light chain variable region framework 2 (L-FR2) comprising an amino acid sequence of SEQ ID NO: 38,
a light chain variable region framework 3 (L-FR3) comprising an amino acid sequence of SEQ ID NO: 39 or SEQ ID NO: 40, and
a light chain variable region framework 4 (L-FR4) comprising an amino acid sequence of SEQ ID NO: 41 or SEQ ID NO: 42.

### [Item 8]

The bispecific antibody according to Item 1, wherein the heavy chain variable region of the anti-IGF1R antibody comprises an amino acid sequence selected from SEQ ID NOs: 43 to 87, and the light chain variable region comprises an amino acid sequence selected from SEQ ID NOs: 88 to 132.

### [Item 9]

The bispecific antibody according to Item 1, wherein the antigen binding fragment of anti-IGFR antibody is selected from the group consisting of scFv, (scFv)2, scFv-Fc, Fab, Fab' and F(ab')2.

### [Item 10]

The bispecific antibody according to Item 1, wherein the anti-α-Syn antibody is a complete antibody, and anti-IGFR antibody is Fv-fragment.

### [Item 11]

The bispecific antibody according to Item 10, wherein the complete antibody of anti-α-Syn antibody is in a form of IgG1, IgG2, IgG3 or IgG4.

### [Item 12]

The bispecific antibody according to Item 10, wherein the bispecific antibody is a monovalent form of bispecific antibody including a molecule of anti-IGFR antibody bonded to a heavy chain CH3.

### [Item 13]

The bispecific antibody according to Item 1, wherein the anti-α-Syn antibody or antigen binding fragment thereof comprises,
a heavy chain variable region including a heavy chain CDR1 (H-CDR1) comprising an amino acid sequence of SEQ ID NO: 135, a heavy chain CDR2 (H-CDR2) comprising an amino acid sequence selected from SEQ ID NO: 136 or 137, and a heavy chain CDR3 (H-CDR3) comprising an amino acid sequence of SEQ ID NO: 138; and
a light chain variable region including a light chain CDR1 (H-CDR1) comprising an amino acid sequence of SEQ ID NO: 139, a light chain CDR2 (H-CDR2) comprising an amino acid sequence of SEQ ID NO: 140, and a light chain CDR3 (H-CDR3) comprising an amino acid sequence of SEQ ID NO: 141.

### [Item 14]

A pharmaceutical composition for prevention or treatment for α-synucleinopathy, comprising a bispecific antibody according to any one of Items 1 to 13.

### [Item 15]

The pharmaceutical composition according to Item 14, wherein the α-synucleinopathy is Parkinson's disease (PD), Parkinson's disease dementia (PDD), dementia with Lewy bodies, (DLB), Lewy body variant of Alzheimer's disease (LBV)), Combined Alzheimer's and Parkinson disease, or multiple system atrophy( MSA).

## Claims

1. An anti-IGF 1R antibody or an antigen binding fragment thereof, wherein the anti-IGF 1R antibody comprises a heavy chain variable region and a light chain variable region,
wherein the heavy chain variable region comprises a heavy chain CDR1 (H-CDR1) comprising an amino acid sequence selected from the amino acid sequences of SEQ ID NO: 1 and SEQ ID NO: 10, a heavy chain CDR2 (H-CDR2) comprising an amino acid sequence selected from the amino acid sequences of SEQ ID NOs: 2 to 7 and SEQ ID NOs: 11 to 18, and a heavy chain CDR3 (H-CDR3) comprising an amino acid sequence selected from the amino acid sequences of SEQ ID NOs: 8 to 9 and SEQ ID NO: 19, and
wherein the light chain variable region comprises a light chain CDR1 (L-CDR1) comprising an amino acid sequence of SEQ ID NO: 20, a light chain CDR2 (L-CDR2) comprising an amino acid sequence selected from the amino acid sequences of SEQ ID NOs: 21 to 23, and a light chain CDR3 (L-CDR3) comprising an amino acid sequence selected from the amino acid sequences of SEQ ID NOs: 24 to 28 and SEQ ID NOs: 29 to 31.

2. The anti-IGF1R antibody or an antigen binding fragment thereof according to Claim 1, wherein the anti-IGF1R antibody or antigen binding fragment thereof specifically recognizes and binds to at least an amino acid selected from the group consisting of Y775, P776, F778, R650, S791, L798, Glu779, L641, H808, E809, L813, V397, D435, W434, Y460 and C488 in a protein comprising an amino acid sequence of SEQ ID NO: 174.

3. The anti-IGF1R antibody or an antigen binding fragment thereof according to Claim 2, wherein the anti-IGF1R antibody or antigen binding fragment thereof recognizes and binds to at least a binding site selected from the group consisting of binding site 1 to binding site 3 in a protein comprising an amino acid sequence of SEQ ID NO: 174, and wherein the binding site 1 comprises at least one selected from the group consisting of Y775, P776, F778, R650, S791, L798 and Glu779, the binding site 2 comprises at least one selected from the group consisting of L641, H808, E809 and L813, and the binding site 3 comprises at least one selected from the group consisting of V397, D435, W434, Y460 and C488.

4. The anti-IGF1R antibody or an antigen binding fragment thereof according to Claim 1, wherein the anti-IGF1R antibody or antigen binding fragment thereof comprises a heavy chain variable region and a light chain variable region,
the heavy chain variable region comprises a heavy chain CDR1 (H-CDR1) comprising an amino acid sequence elected from the amino acid sequences of SEQ ID NO: 1, a heavy chain CDR2 (H-CDR2) comprising an amino acid sequence selected from the amino acid sequences of SEQ ID NO: 3, and SEQ ID NOs: 5 to 7, and a heavy chain CDR3 (H-CDR3) comprising an amino acid sequence selected from the amino acid sequences of SEQ ID NOs: 8 to 9, and
the light chain variable region comprises a light chain CDR1 (L-CDR1) comprising an amino acid sequence selected from the amino acid sequences of SEQ ID NO: 20, a light chain CDR2 (L-CDR2) comprising an amino acid sequence selected from the amino acid sequences of SEQ ID NOs: 22 and 23, and a light chain CDR3 (L-CDR3) comprising an amino acid sequence selected from the amino acid sequences of SEQ ID NOs: 26 to 28.

5. The anti-IGF1R antibody or an antigen binding fragment thereof according to Claim 1, wherein the heavy chain variable region of the anti-IGF1R antibody or antigen binding fragment thereof comprises,
a heavy chain variable region framework1 (H-FR1) comprising an amino acid sequence of SEQ ID NO: 32,
a heavy chain variable region framework 2(H-FR2) comprising an amino acid sequence of SEQ ID NO: 33 or SEQ ID NO: 34,
a heavy chain variable region framework 3 (H-FR3) comprising an amino acid sequence of SEQ ID NO: 35, and
a heavy chain variable region framework 4 (H-FR4) comprising an amino acid sequence of SEQ ID NO: 36; and
wherein the light chain variable region of the anti-IGF1R antibody or antigen binding fragment thereof comprises:
a light chain variable region framework1 (L-FR1) comprising an amino acid sequence selected from SEQ ID NO: 37,
a light chain variable region framework 2 (L-FR2) comprising an amino acid sequence of SEQ ID NO: 38,
a light chain variable region framework 3 (L-FR3) comprising an amino acid sequence of SEQ ID NO: 39 or SEQ ID NO: 40, and
a light chain variable region framework 4 (L-FR4) comprising an amino acid sequence of SEQ ID NO: 41 or SEQ ID NO: 42.

6. The anti-IGF1R antibody or an antigen binding fragment thereof according to Claim 1, wherein the heavy chain variable region of the anti-IGF 1R antibody comprises an amino acid sequence selected from SEQ ID NOs: 43 to 87, and the light chain variable region comprises an amino acid sequence selected from SEQ ID NOs: 88 to 132.

7. The anti-IGF1R antibody or an antigen binding fragment thereof according to Claim 1, wherein the antigen binding fragment of anti-IGFR antibody is selected from the group consisting of scFv, (scFv)₂, scFv-Fc, Fab, Fab' and F(ab')₂.

8. A pharmaceutical composition comprising the anti-IGF1R antibody or an antigen binding fragment thereof according to any one of claims 1-7.

9. The anti-IGF1R antibody or an antigen binding fragment thereof according to any one of claims 1-7 or the pharmaceutical composition according to claim 8 for use in a method of preventing or treating a disease by therapy.

10. The anti-IGF1R antibody or an antigen binding fragment thereof according to any one of claims 1-7 or the pharmaceutical composition according to claim 8 for use in a method of preventing or treating a brain disease, wherein the method comprises delivering a physiologically active substance through the blood-brain-barrier into the inner brain by using the anti-IGF1R antibody or the antigen binding fragment thereof.
